(19) Europäisches Patentamt
European Patent Office
Office européen des brevets

(11) EP 4 339 188 A1

(12) EUROPEAN PATENT APPLICATION
published in accordance with Art. 153(4) EPC

(43) Date of publication:
**20.03.2024 Bulletin 2024/12**

(21) Application number: **22806865.6**

(22) Date of filing: **13.05.2022**

(51) International Patent Classification (IPC):
***C07D 215/46*** (2006.01) ***C07D 233/76*** (2006.01)
***C07D 241/12*** (2006.01) ***C07D 231/12*** (2006.01)
***C07D 269/02*** (2006.01) ***C07D 295/02*** (2006.01)
***A61K 31/4706*** (2006.01) ***A61K 31/4166*** (2006.01)
***A61K 31/421*** (2006.01) ***A61K 31/4155*** (2006.01)
***A61K 31/5375*** (2006.01) ***A61K 31/496*** (2006.01)
***A61P 31/14*** (2006.01) ***A61P 31/18*** (2006.01)
***A61P 31/20*** (2006.01) ***A61P 37/02*** (2006.01)

(52) Cooperative Patent Classification (CPC):
**A61K 31/4155; A61K 31/4166; A61K 31/421; A61K 31/4706; A61K 31/4709; A61K 31/496; A61K 31/517; A61K 31/5375; A61K 31/541; A61P 3/10; A61P 9/00; A61P 11/00; A61P 29/00; A61P 31/14; A61P 31/18;** (Cont.)

(86) International application number:
**PCT/CN2022/092860**

(87) International publication number:
**WO 2022/237903 (17.11.2022 Gazette 2022/46)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **14.05.2021 CN 202110530276**

(71) Applicant: **Shanghai Pharmaceuticals Holding Co., Ltd.**
**Shanghai 201203 (CN)**

(72) Inventors:
- **WANG, Yuji**
  **Shanghai 201203 (CN)**
- **DU, Lisha**
  **Shanghai 201203 (CN)**
- **XIA, Guangxin**
  **Shanghai 201203 (CN)**
- **ZHAO, Fei**
  **Shanghai 201203 (CN)**
- **LI, Gongsheng**
  **Shanghai 201203 (CN)**
- **HAO, Lijun**
  **Shanghai 201203 (CN)**
- **WU, Yichen**
  **Shanghai 201203 (CN)**
- **KE, Ying**
  **Shanghai 201203 (CN)**

(74) Representative: **Haseltine Lake Kempner LLP**
**One Portwall Square**
**Portwall Lane**
**Bristol BS1 6BH (GB)**

# (54) QUINAZOLINE COMPOUND, AND PREPARATION METHOD THEREFOR AND APPLICATION THEREOF

(57) Provided are a quinazoline compound and an application thereof. Specifically provided is a quinazoline compound as shown in formula I. The compound is novel in structure and has a good inhibitory effect on cell factors TNF-$\alpha$, IL-6, and IL-1$\beta$.



Processed by Luminess, 75001 PARIS (FR)

(Cont. next page)

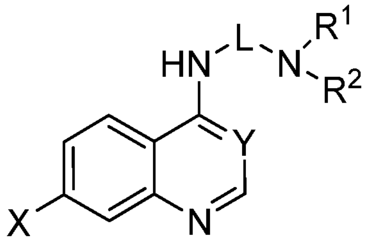

I

(52) Cooperative Patent Classification (CPC): (Cont.)
**A61P 31/20; A61P 35/00; A61P 37/02; A61P 37/08; C07D 215/46; C07D 231/12; C07D 233/76; C07D 241/12; C07D 269/02; C07D 295/02; C07D 401/12; C07D 403/12; C07D 413/12; C07D 417/12; C07D 491/107;**
Y02A 50/30

## Description

The present application claims the priority of Chinese Patent Application No. 2021105302760 with an application date of May 14, 2021. The contents of the above Chinese patent application are incorporated herein by reference in its entirety.

TECHNICAL FIELD

The present disclosure relates to a quinazoline compound, a preparation method therefor, and a use thereof.

BACKGROUND

Inflammatory diseases have become a significant threat to global public health. Inflammation is closely related to the pathological process of various diseases, including many infectious and autoimmune diseases, malignant tumors, cardiovascular diseases, diabetes, and other chronic non-communicable diseases of significance. Autoimmune diseases refer to a class of diseases induced by the immune system attacking the body's own organs, tissues, or cells, causing damage. These primarily include psoriasis, rheumatoid arthritis (RA), systemic lupus erythematosus (SLE), and multiple sclerosis (MS), etc.

Inflammatory cytokines TNF-$\alpha$, IL-6, and IL-1$\beta$ are abundantly expressed in the serum and local lesion tissues of patients with autoimmune diseases. Inhibiting the formation of these three factors can effectively reduce the inflammatory response and reduce the damage to bodily tissues caused by inflammation. Hydroxychloroquine (HCQ) was introduced in 1944, initially gaining attention as an anti-malarial drug. It was later discovered that HCQ also has anti-inflammatory properties, and thus it has been used as an anti-inflammatory agent to treat certain inflammatory diseases. It is widely used in the treatment of systemic lupus erythematosus, rheumatoid arthritis, Sjogren's syndrome, porphyria cutanea tarda, etc.

Modifying HCQ to develop newer drugs with better activity has been a hot research topic in the field of medicinal chemistry.

CONTENT OF THE PRESENT INVENTION

The technical problem to be solved by the present disclosure is to provide a structurally novel quinazoline compound with anti-inflammatory effects. The compounds of the present disclosure exhibit good inhibitory effects on cytokines TNF-$\alpha$, IL-6, and IL-1$\beta$.

The present disclosure provides a quinazoline compound of formula I, a pharmaceutically acceptable salt thereof, a solvate thereof, or a solvate of the pharmaceutically acceptable salt thereof:

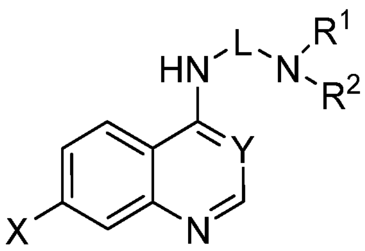

I

;

wherein X is halogen;

Y is CH or N;

L is $-(CH_2)_m-$ or $-(CH_2)_m-$ substituted by 1, 2, or 3 $C_{1-4}$ alkyl groups; m is 4, 5, or 6;

$R^1$ is H or

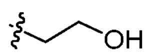

;

$R^2$ is $C_{1-4}$ alkyl or $C_{1-4}$ alkyl substituted by 1, 2, or 3 $R^{2-1}$,

$R^{2-1}$ is 3- to 7-membered heterocycloalkyl, 5- to 6-membered heteroaryl, $C_{6-12}$ aryl, 3-to 7-membered heterocycloalkyl

substituted by 1, 2, or 3 $R^{2-1-1}$, 5- to 6-membered heteroaryl substituted by 1, 2, or 3 $R^{2-1-2}$, or $C_{6-12}$ aryl substituted by 1, 2, or 3 $R^{2-1-3}$; in the 5- to 6-membered heteroaryl and the 5- to 6-membered heteroaryl substituted by 1, 2, or 3 $R^{2-1-2}$, the heteroatom in the 5- to 6-membered heteroaryl is 1, 2, or 3 heteroatoms selected from one or more than one of oxygen, sulfur, and nitrogen; in the 3- to 7-membered heterocycloalkyl and the 3- to 7-membered heterocycloalkyl substituted by 1, 2, or 3 $R^{2-1-1}$, the heteroatom in the 3-to 7-membered heterocycloalkyl is 1, 2, or 3 heteroatoms selected from one or more than one of oxygen, sulfur, and nitrogen;

$R^{2-1-1}$ is independently $C_{1-4}$ alkyl;

$R^{2-1-2}$ is independently $C_{1-4}$ alkyl or $C_{1-4}$ alkoxy;

$R^{2-1-3}$ is independently halogen, cyano, $C_{1-4}$ alkoxy, $C_{1-4}$ alkyl substituted by 1, 2, or 3 halogens, $C_{1-4}$ alkoxy substituted by 1, 2, or 3 halogens, $C_{6-12}$ aryl, 5- to 6-membered heteroaryl,

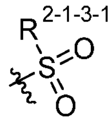 , or 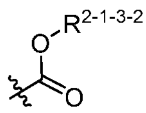 ;

the heteroatom in the 5- to 6-membered heteroaryl is 1, 2, or 3 heteroatoms selected from one or more than one of oxygen, sulfur, and nitrogen;

$R^{2-1-3-1}$ is independently $C_{1-4}$ alkyl;

$R^{2-1-3-2}$ is independently $C_{1-4}$ alkyl;

alternatively, $R^1$ and $R^2$, together with the nitrogen atom between them, form a ring A, and the ring A is 4- to 12-membered heterocycloalkyl or 4- to 12-membered heterocycloalkyl substituted by 1, 2, or 3 $R^{2-2}$; in the case where ring A is 4- to 12-membered heterocycloalkyl or 4- to 12-membered heterocycloalkyl substituted by 1, 2, or 3 $R^{2-2}$, the heteroatom in the 4-to 12-membered heterocycloalkyl is 1, 2, or 3 heteroatoms selected from one or more than one of oxygen, sulfur, and nitrogen;

$R^{2-2}$ is independently oxo, $C_{1-4}$ alkyl, or halogen.

In a certain embodiment, the quinazoline compound of formula I satisfies 1, 2, or 3 of the following conditions:

i: $R^1$ is

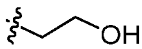 ;

ii: $R^2$ is $C_{1-4}$ alkyl substituted by 1, 2, or 3 $R^{2-1}$, and $R^{2-1}$ is 3- to 7-membered heterocycloalkyl, 5- to 6-membered heteroaryl, 3- to 7-membered heterocycloalkyl substituted by 1, 2, or 3 $R^{2-1-1}$, or 5- to 6-membered heteroaryl substituted by 1, 2, or 3 $R^{2-1-2}$;

iii: $R^1$ and $R^2$, together with the nitrogen atom between them, form a ring A, and the ring A is a spiro or bridged ring; alternatively, $R^1$ and $R^2$, together with the nitrogen atom between them, form a ring A, and the ring A is a monocyclic ring, wherein the ring A is 4- to 12-membered heterocycloalkyl substituted by 2 or 3 $R^{2-2}$; $R^{2-2}$ is independently oxo, $C_{1-4}$ alkyl, or halogen, and when substituents in the 4- to 12-membered heterocycloalkyl contain oxo, the number of oxo groups is 2 or 3.

In a certain embodiment, X is halogen;

Y is CH or N;

L is $-(CH_2)_m-$ or $-(CH_2)_m-$ substituted by 1, 2, or 3 $C_{1-4}$ alkyl groups; m is 4 or 5;

$R^1$ is H or

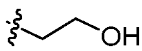 ;

$R^2$ is $C_{1-4}$ alkyl substituted by 1, 2, or 3 $R^{2-1}$,

$R^{2-1}$ is 3- to 7-membered heterocycloalkyl, 5- to 6-membered heteroaryl, $C_{6-12}$ aryl, 3-to 7-membered heterocycloalkyl substituted by 1, 2, or 3 $R^{2-1-1}$, 5- to 6-membered heteroaryl substituted by 1, 2, or 3 $R^{2-1-2}$, or $C_{6-12}$ aryl substituted by 1, 2, or 3 $R^{2-1-3}$; in the 5- to 6-membered heteroaryl and the 5- to 6-membered heteroaryl substituted by 1, 2, or 3 $R^{2-1-2}$, the heteroatom in the 5- to 6-membered heteroaryl is 1, 2, or 3 heteroatoms selected from one or more

than one of oxygen, sulfur, and nitrogen; in the 3- to 7-membered heterocycloalkyl and the 3- to 7-membered heterocycloalkyl substituted by 1, 2, or 3 $R^{2-1-1}$, the heteroatom in the 3-to 7-membered heterocycloalkyl is 1, 2, or 3 heteroatoms selected from one or more than one of oxygen, sulfur, and nitrogen;

$R^{2-1-1}$ is independently $C_{1-4}$ alkyl;

$R^{2-1-2}$ is independently $C_{1-4}$ alkyl or $C_{1-4}$ alkoxy;

$R^{2-1-3}$ is independently halogen, cyano, $C_{1-4}$ alkoxy, $C_{1-4}$ alkyl substituted by 1, 2, or 3 halogens, $C_{1-4}$ alkoxy substituted by 1, 2, or 3 halogens, 5- to 6-membered heteroaryl,

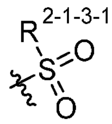,

or 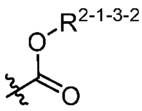;

the heteroatom in the 5- to 6-membered heteroaryl is 1, 2, or 3 heteroatoms selected from one or more than one of oxygen, sulfur, and nitrogen;

$R^{2-1-3-1}$ is independently $C_{1-4}$ alkyl;

$R^{2-1-3-2}$ is independently $C_{1-4}$ alkyl;

alternatively, $R^1$ and $R^2$, together with the nitrogen atom between them, form a ring A, wherein the ring A is 5- to 12-membered hetercycloalkyl $A_1$, and the heterocycloalkyl $A_1$ is a bridged ring; or the ring A is 5- to 10-membered heterocycoalkyl $A_2$ substituted by 2 or 3 $R^{2-2-1}$, and the heterocycloalkyl $A_2$ is a monocyclic ring; or the ring A is 8- to 12-membered heterocycloalkyl $A_3$ substituted by 1, 2, or 3 $R^{2-2-2}$, and the heterocycloalkyl $A_3$ is a spiro ring; in the heterocycloalkyl, the heteroatom is 1, 2, or 3 heteroatoms selected from one or more than one of oxygen, sulfur, and nitrogen;

$R^{2-2-1}$ is independently oxo, $C_{1-4}$ alkyl, or halogen;

$R^{2-2-2}$ is independently oxo, $C_{1-4}$ alkyl, or halogen;

and the quinazoline compound satisfies 1, 2, or 3 of following conditions:

i: $R^1$ is

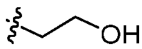;

ii: $R^2$ is $C_{1-4}$ alkyl substituted by 1, 2, or 3 $R^{2-1}$, and $R^{2-1}$ is 3- to 7-membered heterocycloalkyl, 5- to 6-membered heteroaryl, 3- to 7-membered heterocycloalkyl substituted by 1, 2, or 3 $R^{2-1-1}$, or 5- to 6-membered heteroaryl substituted by 1, 2, or 3 $R^{2-1-2}$;

iii: $R^1$ and $R^2$, together with the nitrogen atom between them, form a ring A, wherein the ring A is 5- to 12-membered heterocycloalkyl $A_1$, and the heterocycloalkyl $A_1$ is a bridged ring; or the ring A is 8- to 12-memebered heterocycloalkyl $A_3$ substituted by 1, 2, or 3 $R^{2-2-2}$, and the heterocycloalkyl $A_3$ is a spiro ring; alternatively, $R^1$ and $R^2$, together with the nitrogen atom between them, form a ring A, wherein the ring A is 5- to 10-membered heterocycloalkyl $A_2$ substituted by 2 or 3 $R^{2-2-1}$, and the heterocycloalkyl $A_2$ is a monocyclic ring; $R^{2-2-1}$ is independently oxo, $C_{1-4}$ alkyl, or halogen, and when substituents in the 5- to 10-membered heterocycloalkyl contain oxo, the number of oxo groups is 2 or 3.

In a certain embodiment, in the quinazoline compound of formula I, the pharmaceutically acceptable salt thereof, the solvate thereof, or the solvate of the pharmaceutically acceptable salt thereof, some groups are defined as follows and other groups are as defined in any other embodiment (hereinafter referred to as "in a certain embodiment"): L is $-(CH_2)_m-$ or $-(CH_2)_m-$ substituted by 1 methyl group; m is 4 or 5; for example, L is

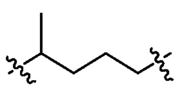, 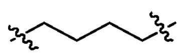, or 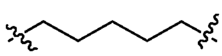.

In a certain embodiment, L is

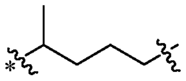, 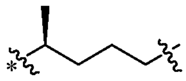, 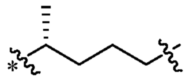,

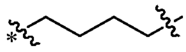, or 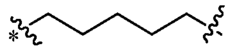,

wherein the side marked with * indicates connection to the N in the NH structure of the parent nucleus.

In a certain embodiment, X is F or Cl.

In a certain embodiment, $R^2$ is $C_{1-4}$ alkyl substituted by 1, 2, or 3 $R^{2-1}$.

In a certain embodiment, $R^{2-1-3}$ is independently halogen, cyano, $C_{1-4}$ alkoxy, $C_{1-4}$ alkyl substituted by 1, 2, or 3 halogens, $C_{1-4}$ alkoxy substituted by 1, 2, or 3 halogens, 5- to 6-membered heteroaryl,

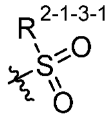, or 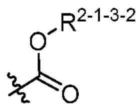;

the heteroatom in the 5- to 6-membered heteroaryl is 1, 2, or 3 heteroatoms selected from one or more than one of oxygen, sulfur, and nitrogen.

In a certain embodiment, $R^1$ and $R^2$, together with the nitrogen atom between them, form a ring A, wherein the ring A is 5- to 12-membered hetercycloalkyl $A_1$, and the heterocycloalkyl $A_1$ is a bridged ring; or the ring A is 5- to 10-membered heterocycoalkyl $A_2$ substituted by 2 or 3 $R^{2-2-1}$, and the heterocycloalkyl $A_2$ is a monocyclic ring; or the ring A is 8- to 12-membered heterocycloalkyl $A_3$ substituted by 1, 2, or 3 $R^{2-2-2}$, and the heterocycloalkyl $A_3$ is a spiro ring.

$R^{2-2-1}$ is independently oxo, $C_{1-4}$ alkyl, or halogen;
$R^{2-2-2}$ is independently oxo, $C_{1-4}$ alkyl, or halogen.

In a certain embodiment, $R^1$ and $R^2$, together with the nitrogen atom between them, form a ring A, wherein the ring A is 5- to 12-membered hetercycloalkyl $A_1$, and the heterocycloalkyl $A_1$ is a bridged ring; or the ring A is 5- to 10-membered heterocycoalkyl $A_2$ substituted by 2 or 3 $R^{2-2-1}$, and the heterocycloalkyl $A_2$ is a monocyclic ring; or the ring A is 8- to 12-membered heterocycloalkyl $A_3$ substituted by 1, 2, or 3 $R^{2-2-2}$, and the heterocycloalkyl $A_3$ is a spiro ring;

$R^{2-2-1}$ is independently oxo, $C_{1-4}$ alkyl, or halogen;
$R^{2-2-2}$ is independently oxo, $C_{1-4}$ alkyl, or halogen;
in $A_2$, when substituents in the 5- to 10- membered heterocycloalkyl contain oxo, the number of oxo groups is 2 or 3.

In a certain embodiment, $R^1$ and $R^2$, together with the nitrogen atom between them, form a ring A, and the ring A can be

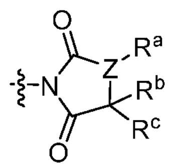 or 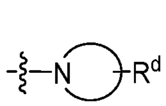;

wherein Z is CH or N;
$R^a$, $R^b$, and $R^c$ are each independently H or $C_{1-4}$ alkyl; alternatively, $R^b$ and $R^c$, together with the atom to which they are attached, form a $C_{3-6}$ hydrocarbon ring or a 3- to 6-membered heterocycle; the heteroatom in the 3- to 6-membered heterocycle is 1 or 2 heteroatoms selected from one or more than one of oxygen, sulfur, and nitrogen;
the structural moiety

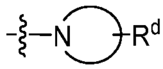

represents 3- to 6-membered nitrogen-containing heterocycloalkyl substituted by 2 or 3 $R^d$; aside from nitrogen, the heteroatom in the heterocyloalkyl is 1, 2, or 3 heteroatoms selected from one or more than one of oxygen, sulfur, and nitrogen;

$R^d$ is H, oxo, or halogen; or adjacent two $R^d$, together with the atom to which they are attached, form a $C_{3-6}$ hydrocarbon ring, a 3- to 6-membered heterocycle, a $C_{3-6}$ hydrocarbon ring substituted by 1, 2, or 3 $R^{d-1}$, or a 3- to 6-membered heterocycle substituted by 1, 2, or 3 $R^{d-1}$; or two $R^d$ linking to the same atom, together with the atom to which they are attached, form a $C_{3-6}$ hydrocarbon ring, a 3- to 6-membered heterocycle, a $C_{3-6}$ hydrocarbon ring substituted by 1, 2, or 3 $R^{d-1}$, or a 3- to 6-membered heterocycle substituted by 1, 2, or 3 $R^{d-1}$; the heteroatom in the heterocycle is 1, 2, or 3 heteroatoms selected from one or more than one of oxygen, sulfur, and nitrogen;

$R^{d-1}$ is independently $C_{1-4}$ alkyl.

In a certain embodiment, $R^a$ is H, and $R^b$ and $R^c$ are each independently $C_{1-4}$ alkyl.

In a certain embodiment, $R^2$ is methyl substituted by 1 or 2 $R^{2-1}$ or ethyl substituted by 1 or 2 $R^{2-1}$.

In a certain embodiment, ring A is

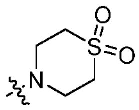, 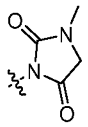, 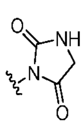, 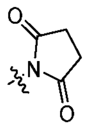, 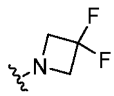,

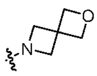, 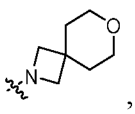, 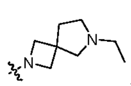, 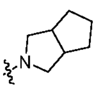, 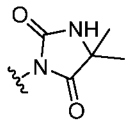, 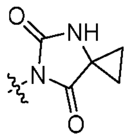, 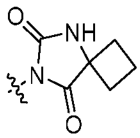, 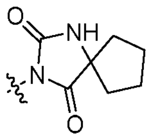,

or

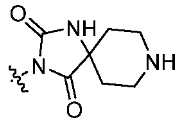.

In a certain embodiment, $R^2$ is

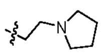, 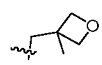, 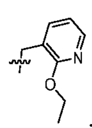, 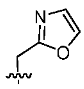, 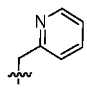, 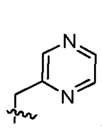,

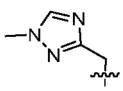, 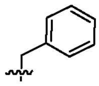, 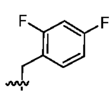, 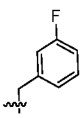, 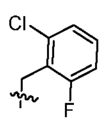, 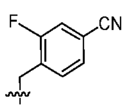, 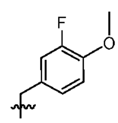, 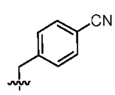, 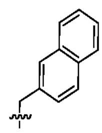,

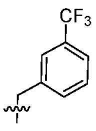, 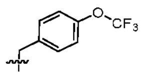, 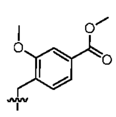, 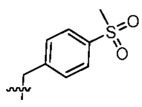, 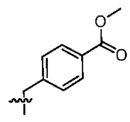, or 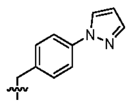.

In a certain embodiment, in $R^2$, in the $C_{1-4}$ alkyl and the $C_{1-4}$ alkyl substituted by 1, 2, or 3 $R^{2-1}$, the $C_{1-4}$ alkyl can independently be methyl, ethyl, n-propyl, isopropyl, n-butyl, *tert*-butyl, isobutyl, or sec-butyl, such as methyl or ethyl.

In a certain embodiment, in $R^{2-1}$, in the 3- to 7-membered heterocycloalkyl and the 3-to 7-membered heterocycloalkyl substituted by 1, 2, or 3 $R^{2-1-1}$, the heteroatom in the 3- to 7-membered heterocycloalkyl can independently be 1 heteroatom being N or O.

In a certain embodiment, in $R^{2-1}$, in the 3- to 7-membered heterocycloalkyl and the 3-to 7-membered heterocycloalkyl substituted by 1, 2 or 3 $R^{2-1-1}$, the 3- to 7-membered heterocycloalkyl can independently be 4- to 5-membered heterocycloalkyl, such as tetrahydropyrrolyl or oxetanyl, also such as

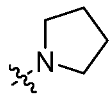 or 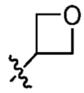 .

In a certain embodiment, in $R^{2-1}$, in the 5- to 6-membered heteroaryl and the 5- to 6-membered heteroaryl substituted by 1, 2, or 3 $R^{2-1-2}$, the type of heteroatoms in the 5- to 6-membered heteroaryl can be selected from one or both of N and O.

In a certain embodiment, in $R^{2-1}$, in the 5- to 6-membered heteroaryl and the 5- to 6-membered heteroaryl substituted by 1, 2, or 3 $R^{2-1-2}$, the 5- to 6-membered heteroaryl can independently be oxazolyl, triazolyl, pyridyl, or pyrazinyl, such as

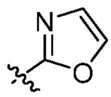 , 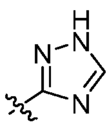 ,

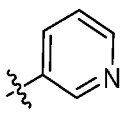 , 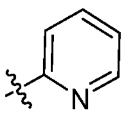 , or 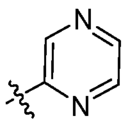 .

In a certain embodiment, in $R^{2-1}$, in the $C_{6-12}$ aryl and the $C_{6-12}$ aryl substituted by 1, 2, or 3 $R^{2-1-3}$, the $C_{6-12}$ aryl can independently be phenyl or naphthyl, such as

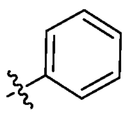

or

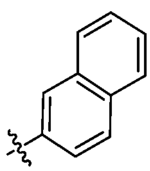 .

In a certain embodiment, in $R^{2-1-1}$, the $C_{1-4}$ alkyl is independently methyl, ethyl, *n*-propyl, isopropyl, *n*-butyl, *tert*-butyl, isobutyl, or *sec*-butyl, such as methyl.

In a certain embodiment, in $R^{2-1-2}$, the $C_{1-4}$ alkyl is independently methyl, ethyl, *n*-propyl, isopropyl, *n*-butyl, *tert*-butyl, isobutyl, or *sec*-butyl, such as methyl.

In a certain embodiment, in $R^{2-1-2}$, the $C_{1-4}$ alkoxy is independently methoxy, ethoxy, or propoxy, such as ethoxy.

In a certain embodiment, in $R^{2-1-3}$, the halogen is independently F, Cl, Br, or I, such as F or Cl.

In a certain embodiment, in $R^{2-1-3}$, the $C_{1-4}$ alkoxy is independently methoxy, ethoxy, or propoxy, such as methoxy.

In a certain embodiment, in $R^{2-1-3}$, the $C_{1-4}$ alkyl substituted by 1, 2, or 3 halogens is independently trifluoromethyl, trifluoroethyl, or trifluoropropyl, such as trifluoromethyl.

In a certain embodiment, in $R^{2-1-3}$, the $C_{1-4}$ alkoxy substituted by 1, 2, or 3 halogens is independently trifluoromethoxy, trifluoroethoxy, or trifluoropropoxy, such as trifluoromethoxy.

In a certain embodiment, in $R^{2-1-3}$, the $C_{6-12}$ aryl is phenyl or naphthyl.

In a certain embodiment, in $R^{2-1-3}$, the 5- to 6-membered heteroaryl can be pyrazolyl, pyrazinyl, pyridazinyl, pyridyl, pyrimidinyl, or pyrrolyl, such as pyrazolyl, also such as

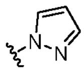.

In a certain embodiment, in $R^{2-1-3-1}$ and $R^{2-1-3-2}$, the $C_{1-4}$ alkyl can independently be methyl, ethyl, *n*-propyl, isopropyl, *n*-butyl, *tert*-butyl, isobutyl, or *sec*-butyl, such as methyl.

In a certain embodiment, in $R^{2-2}$, the $C_{1-4}$ alkyl can independently be methyl, ethyl, *n*-propyl, isopropyl, *n*-butyl, *tert*-butyl, isobutyl, or *sec*-butyl, such as methyl or ethyl.

In a certain embodiment, in $R^{2-2}$, the halogen can independently be F, Cl, Br, or I, such as F.

In a certain embodiment, in $R^a$, $R^b$, and $R^c$, the $C_{1-4}$ alkyl can independently be methyl, ethyl, *n*-propyl, isopropyl, *n*-butyl, *tert*-butyl, isobutyl, or *sec*-butyl, such as methyl.

In a certain embodiment, $R^b$ and $R^c$, together with the atom to which they are attached, form a $C_{3-6}$ hydrocarbon ring, and the $C_{3-6}$ hydrocarbon ring can be cyclopropane, cyclobutane, cyclopentane, or cyclohexane, such as cyclopropane, cyclobutane, or cyclopentane.

In a certain embodiment, $R^b$ and $R^c$, together with the atom to which they are attached, form a 3- to 6-membered heterocycle, and the heteroatom in the 3- to 6-membered heterocycle can be 1 heteroatom being N.

In a certain embodiment, $R^b$ and $R^c$, together with the atom to which they are attached, form a 3- to 6-membered heterocycle, the 3- to 6-membered heterocycle can be 6-membered heterocycle, such as piperidine, also such as

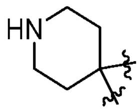.

In a certain embodiment, in the definition of

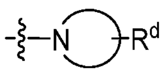,

in the 3- to 6-membered nitrogen-containing heterocycloalkyl, the heteroatom can independently be 1 or 2 heteroatoms being N, N, or S.

In a certain embodiment, in the definition of

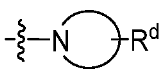,

the 3- to 6-membered nitrogen-containing heterocycloalkyl can independently be thiomorpholinyl, tetrahydropyrrolyl, or azetidinyl, such as

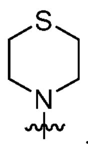, 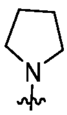, or 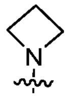.

In a certain embodiment, in the definition of

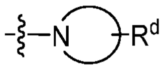
,

the $C_{3-6}$ hydrocarbon ring can independently be cyclopropane, cyclobutane, cyclopentane, or cyclohexane, such as cyclopentane.

In a certain embodiment, in the definition of

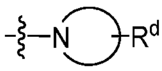
,

in the 3- to 6-membered heterocycle, the heteroatom can independently be 1 heteroatom being N or O.

In a certain embodiment, in the definition of

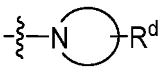
,

the 3- to 6-membered heterocycle can independently be oxetane, tetrahydropyrrole, or tetrahydropyran, for example, when two $R^d$ linking the same atom, together with the atom to which they are attached, form a 3- to 6-membered heterocycle or a 3- to 6-membered heterocycle substituted by 1, 2, or 3 $R^{d-1}$, the 3- to 6-membered heterocycle can be

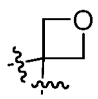 , 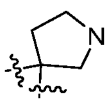 , or 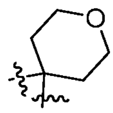 .

In a certain embodiment, in $R^{d-1}$, the $C_{1-4}$ alkyl can be methyl, ethyl, *n*-propyl, isopropyl, *n*-butyl, *tert*-butyl, isobutyl, or *sec*-butyl, such as methyl.

In a certain embodiment, X is halogen; Y is CH or N; $R^1$ is

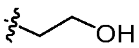
;

$R^2$ is $C_{1-4}$ alkyl substituted by 1, 2, or 3 $R^{2-1}$; $R^{2-1}$ is $C_{6-12}$ aryl substituted by 1, 2, or 3 $R^{2-1-3}$; L is preferably

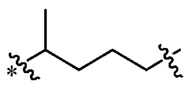
.

In a certain embodiment, X is halogen; Y is N; $R^1$ is

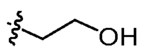 , $R^2$ is 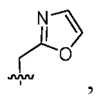 ,

Lis - $(CH_2)_m$-, and m is 4 or 5.

In a certain embodiment, X is Cl; Y is CH; L is $-(CH_2)_m-$ substituted by 1 methyl group, and $R^1$ and $R^2$, together with the nitrogen atom between them, form a ring A, and the ring A is

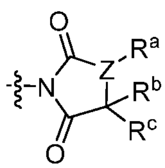
,

wherein $R^a$ is $C_{1-4}$ alkyl, and $R^b$ and $R^c$ are both H.

In a certain embodiment, X is Cl; Y is N; L is $-(CH_2)_m-$ substituted by 1 methyl group, and m is 4 or 5; $R^1$ and $R^2$, together with the nitrogen atom between them, form a ring A, and the ring A is

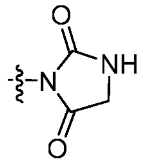
or
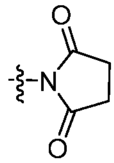
.

In a certain embodiment, X is Cl; Y is CH; L is $-(CH_2)_m-$ substituted by 1 methyl group, and m is 4 or 5; $R^1$ and $R^2$, together with the nitrogen atom between them, form a ring A, wherein the ring A is

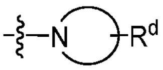

, and $R^d$ is oxo or halogen; or adjacent two $R^d$, together with the atom to which they are attached, form a $C_{3-6}$ hydrocarbon ring; or two $R^d$ linking to the same atom, together with the atom to which they are attached, form a 3- to 6-membered heterocycle or a 3- to 6-membered heterocycle substituted by 1, 2, or 3 $R^{d-1}$; the heteroatom in the heterocycle is 1, 2, or 3 heteroatoms selected from one or more than one of oxygen, sulfur, and nitrogen.

In a certain embodiment, X is Cl; Y is CH; L is $-(CH_2)_m-$ substituted by 1 methyl group, and m is 4 or 5; $R^1$ is H; $R^2$ is $C_{1-4}$ alkyl substituted by 1, 2, or 3 $R^{2-1}$, and $R^{2-1}$ is 3- to 7-membered heterocycloalkyl substituted by 1, 2, or 3 $R^{2-1-1}$.

In a certain embodiment, $R^1$ is

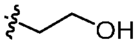
.

In a certain embodiment, $R^2$ is $C_{1-4}$ alkyl substituted by 1, 2, or 3 $R^{2-1}$, and $R^{2-1}$ is 3-to 7-membered heterocycloalkyl, 5- to 6-membered heteroaryl, 3- to 7-membered heterocycloalkyl substituted by 1, 2, or 3 $R^{2-1-1}$, or 5- to 6-membered heteroaryl substituted by 1, 2, or 3 $R^{2-1-2}$.

In a certain embodiment, $R^1$ and $R^2$, together with the nitrogen atom between them, form a ring A, wherein the ring A is 5- to 12-membered hetercycloalkyl $A_1$, and the heterocycloalkyl $A_1$ is a bridged ring; or the ring A is 8- to 12-membered heterocycoalkyl $A_3$ substituted by 1, 2, or 3 $R^{2-2-2}$, and the heterocycloalkyl $A_3$ is a spiro ring; in the heterocycloalkyl, the heteroatom is 1, 2, or 3 heteratoms selected from one or more than one of oxygen, sulfur, and nitrogen.

In a certain embodiment, when $R^1$ and $R^2$, together with the nitrogen atom between them, form a ring A, the ring A is 5- to 10-membered heterocycloalkyl $A_2$ substituted by 2 or 3 $R^{2-2-1}$, and the heterocycloalkyl $A_2$ is a monocyclic ring; $R^{2-2-1}$ is independently oxo, $C_{1-4}$ alkyl, or halogen, and when substituents in the 5- to 10-membered heterocycloalkyl contain oxo, the number of oxo groups is 2 or 3; in the heterocycloalkyl, the heteroatom is 1, 2, or 3 heteratoms selected from one or more than one of oxygen, sulfur, and nitrogen.

In a certain embodiment, the quinazoline compound of formula I can be a compound of formula 1-1 or 1-1':

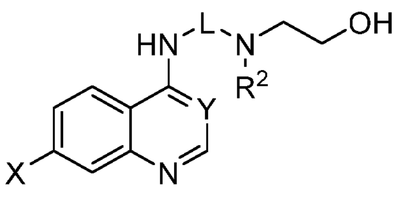
I-1

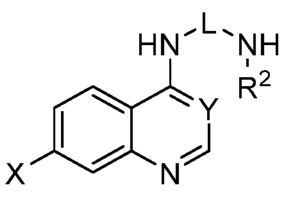
I-1'

wherein each substituent is defined as above.

In a certain embodiment, the quinazoline compound of formula I can be a compound of formula I-2:

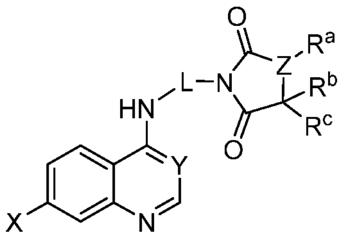

I-2

;

wherein each substituent is defined as above.

In a certain embodiment, the quinazoline compound of formula I can be a compound of formula I-2:

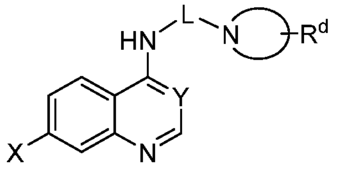

I-3

;

wherein each substituent is defined as above.

In a certain embodiment, the quinazoline compound of formula I is any one of the following compounds:

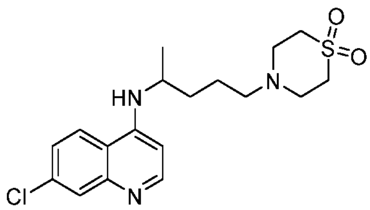
,
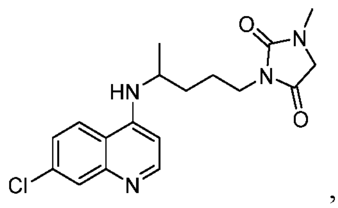
,
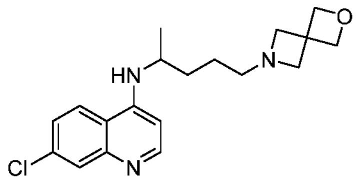
,

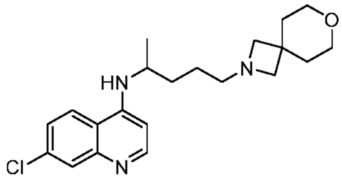
,
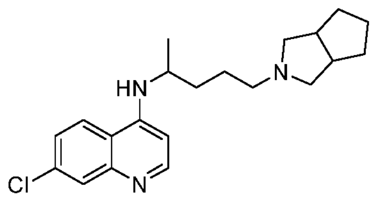
,
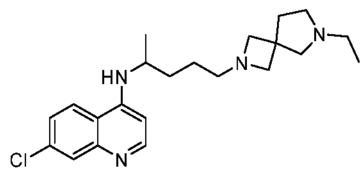
,

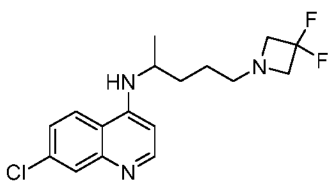
,
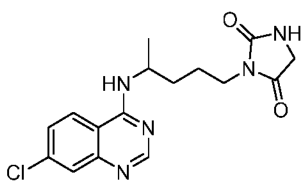
,
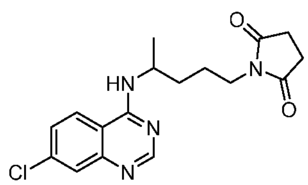
,

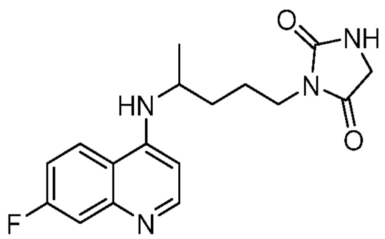
,
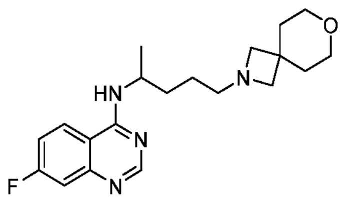
,
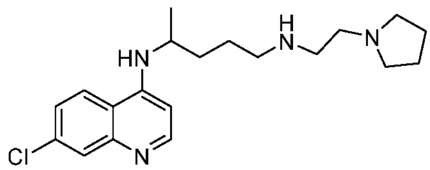
,

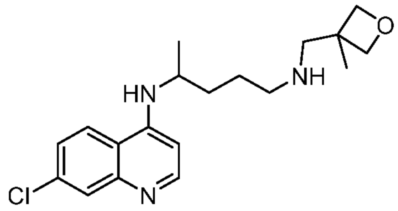,
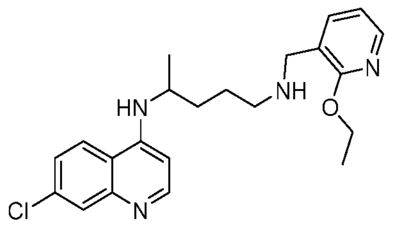,
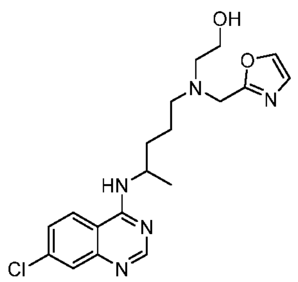,

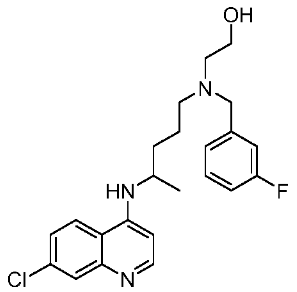,
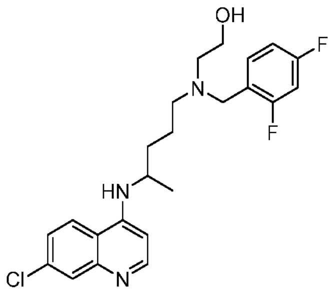,
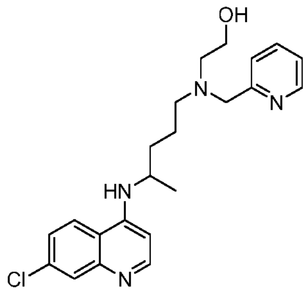,

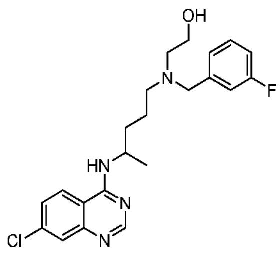,
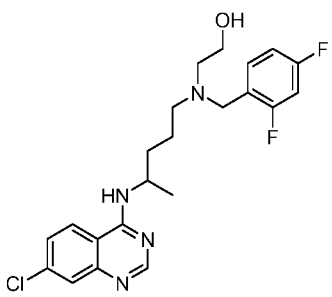,
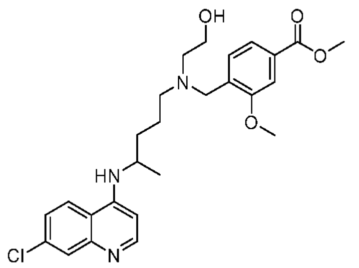,

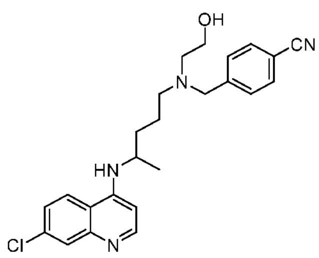,
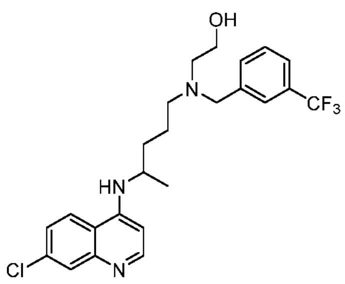,
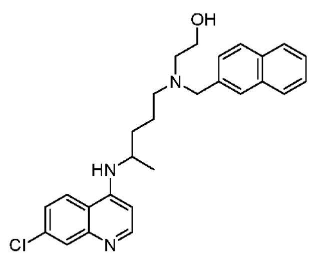,

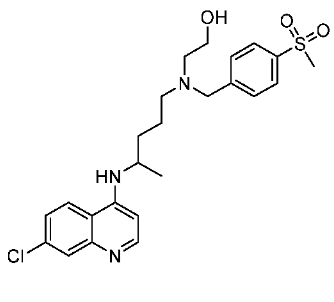,
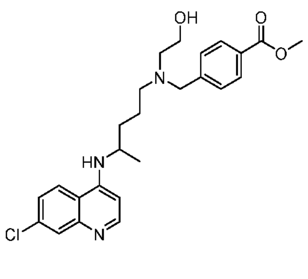,
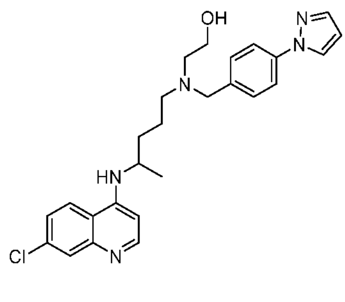,

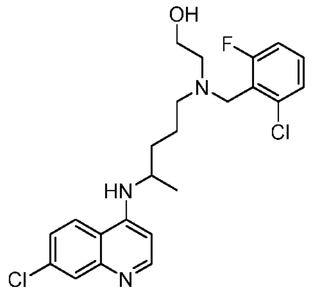 , 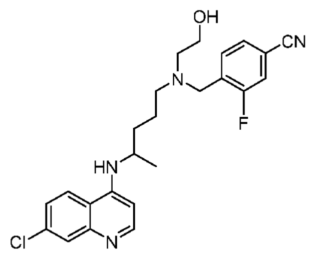 , 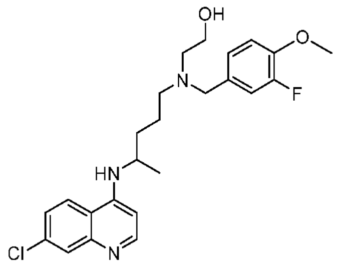 ,

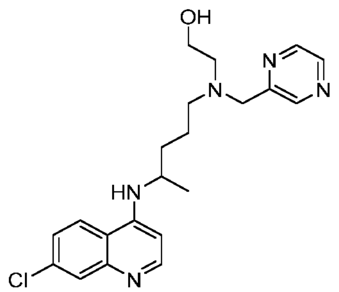 , 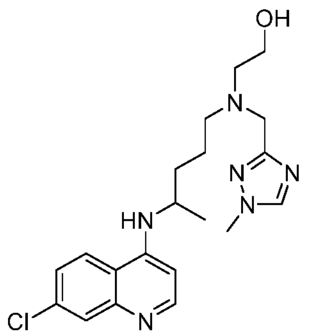 , 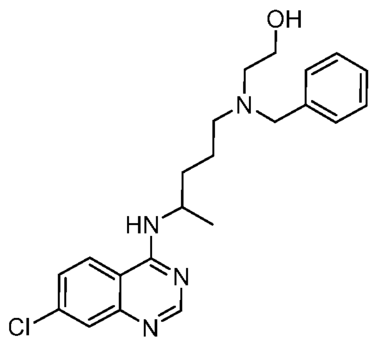 ,

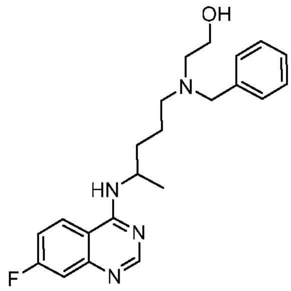 , 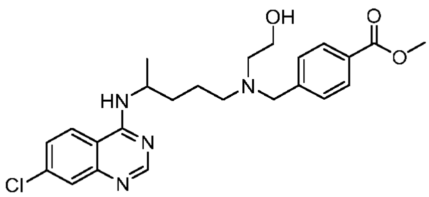 , 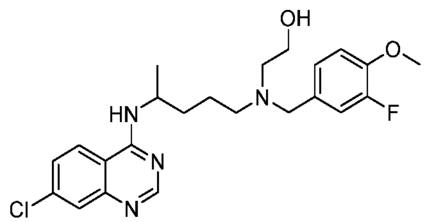 ,

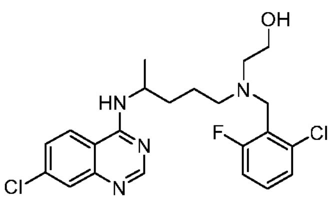 , 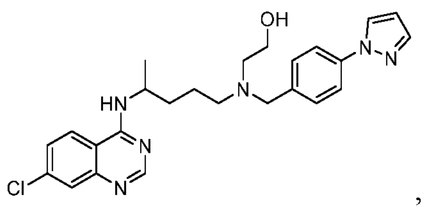 , 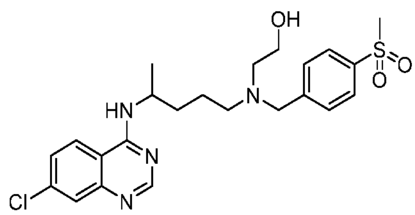 ,

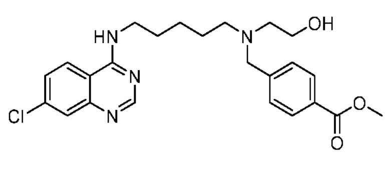 , 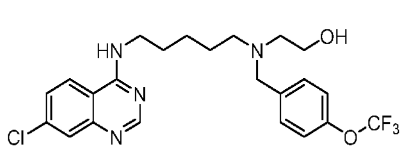 , 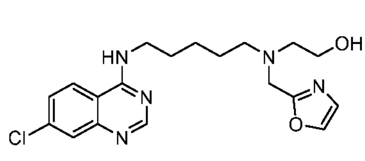 ,

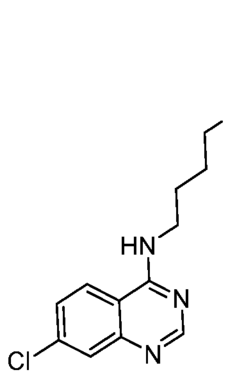 , 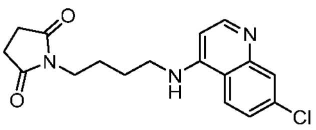 , 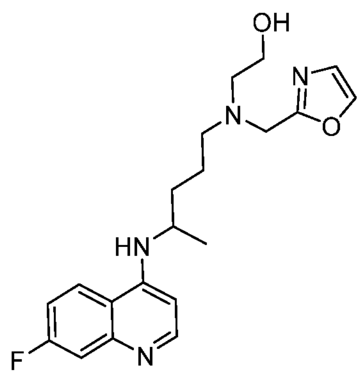 ,

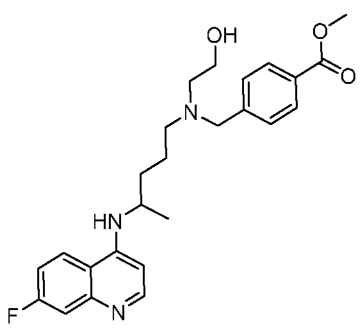 , 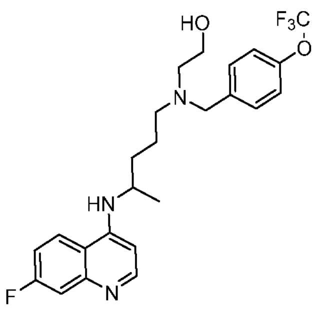 , 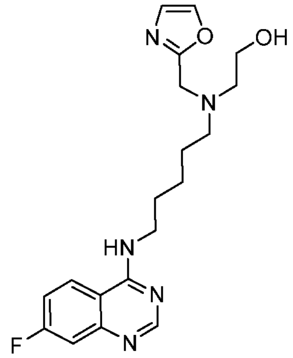 ,

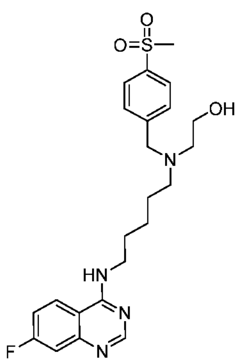 , 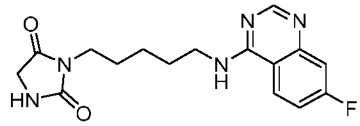 , 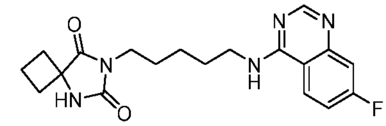 ,

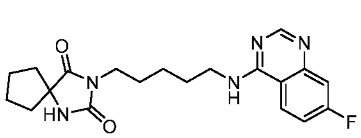 , 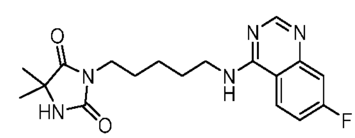 , 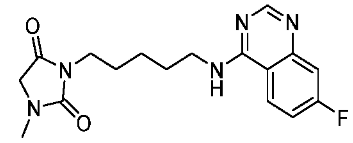 ,

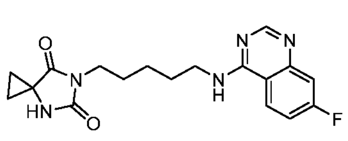 , 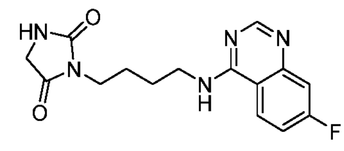 , 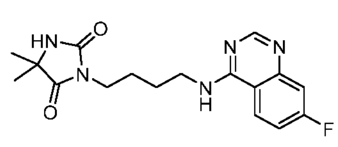 ,

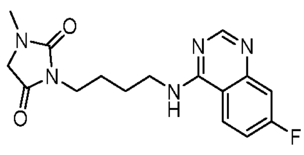 , 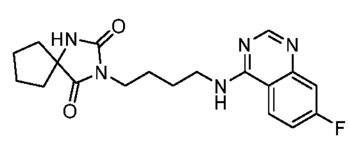 , 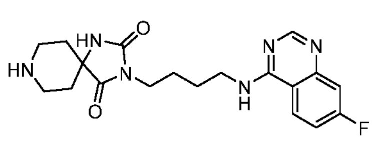 ,

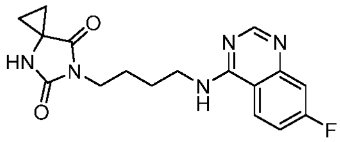, 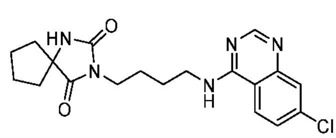, 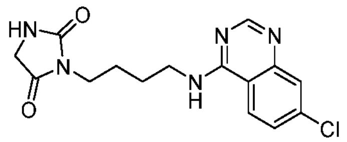,

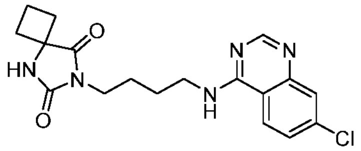, 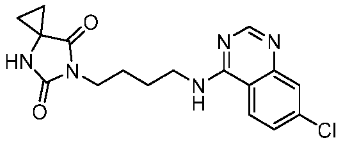, 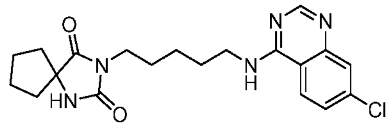,

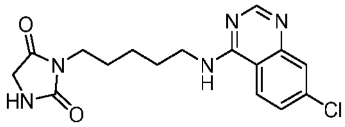, 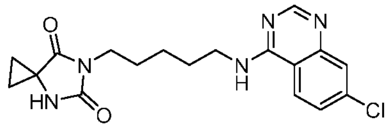, 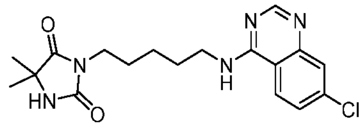,

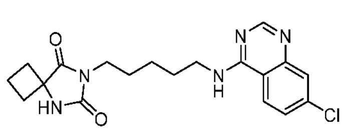, 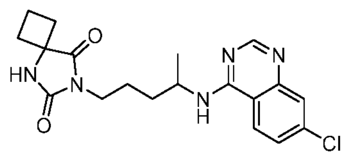, 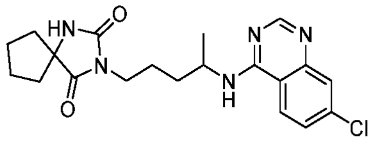,

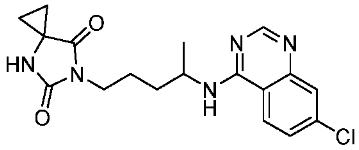, 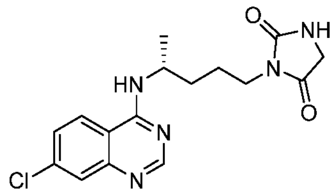, 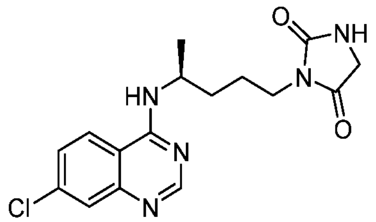,

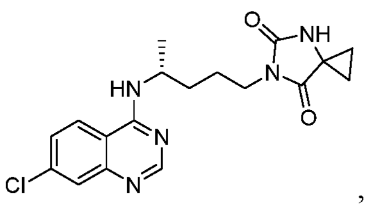, or 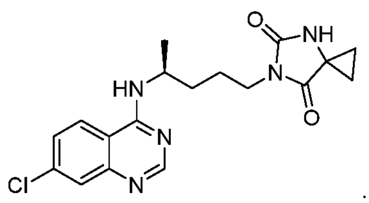.

The present disclosure also provides a method for preparing the quinazoline compound of formula I, and the method is any one of the following methods:

method 1, comprising the following steps: in a solvent, in the presence of a base and an iodine salt, reacting a compound of formula II-1 with a compound of formula II-2;

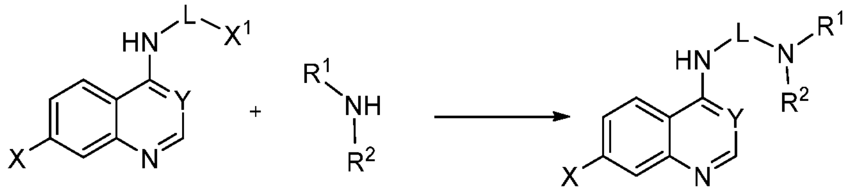

II-1 II-2 I ;

wherein $X^1$ is halogen, such as F, Cl, Br, or I, preferably Cl; Yis CH; other substituents in the above formulas are each defined as above;

the solvent can be a solvent commonly used for such reactions in the art, such as a nitrile solvent, preferably acetonitrile;

the base can be a base commonly used for such reactions in the art, such as an alkali metal carbonate, preferably potassium carbonate;

the iodine salt can be an iodine salt commonly used for such reactions in the art, such as KI;

method 2: in a solvent, in the presence of a base and a quaternary ammonium salt, reacting a compound of formula II-1 with a compound of formula II-2;

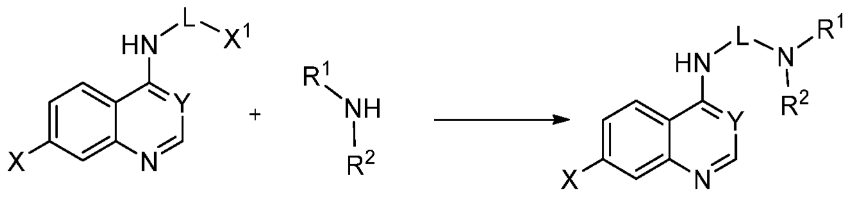

II-1 II-2 I

wherein $X^1$ is halogen, such as F, Cl, Br, or I, preferably Cl; Y is N; other substituents in the above formulas are each defined as above;

the solvent can be a solvent commonly used for such reactions in the art, such as a nitrile solvent, preferably acetonitrile;

the base can be a base commonly used for such reactions in the art, such as an alkali metal carbonate, preferably potassium carbonate;

the quaternary ammonium salt can be a quaternary ammonium salt commonly used for such reactions in the art, such as *n*-$Bu_4NBr$;

method 3, comprising the following steps: in a solvent, in the presence of a base, reacting a compound of formula II-3 with a compound of formula II-4;

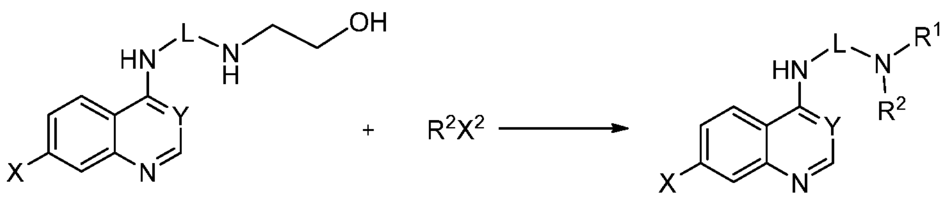

II-3 II-4 I

wherein in formula II-4, $X^2$ is halogen, such as F, Cl, Br, or I, preferably Cl; in formula I, $R^1$ is

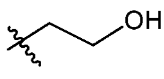

;

other substituents in the above formulas are each defined as above;

the solvent can be a solvent commonly used for such reactions in the art, such as a nitrile solvent, preferably acetonitrile;

the base can be a base commonly used for such reactions in the art, such as an alkali metal carbonate, preferably potassium carbonate.

The present disclosure also provides a pharmaceutical composition comprising a substance X and a pharmaceutical excipient; the substance X is the quinazoline compound of formula I, the pharmaceutically acceptable salt thereof, the solvate thereof, or the solvate of the pharmaceutically acceptable salt thereof.

The present disclosure also provides a use of a substance Y in the manufacture of a medicament; the substance Y is the quinazoline compound of formula I, the pharmaceutically acceptable salt thereof, the solvate thereof, the solvate of the pharmaceutically acceptable salt thereof, or the pharmaceutical composition, and the medicament is a medicament having an inhibitory effect on 1, 2 or 3 of cytokines TNF-$\alpha$, IL-6, and IL-1$\beta$.

The present disclosure also provides a use of a substance Y in the manufacture of an inhibitor; the substance Y is the quinazoline compound of formula I, the pharmaceutically acceptable salt thereof, the solvate thereof, the solvate of the pharmaceutically acceptable salt thereof, or the pharmaceutical composition, and the inhibitor is 1, 2, or 3 of a TNF-$\alpha$ inhibitor, an IL-6 inhibitor, and an IL-1$\beta$ inhibitor.

In a certain embodiment, the inhibitor is an inhibitor having an inhibitory effect *in vitro* on 1, 2, or 3 of TNF-$\alpha$, IL-6, and IL-1$\beta$.

The present disclosure also provides a use of a substance Y in the manufacture of a medicament; the substance Y is the quinazoline compound of formula I, the pharmaceutically acceptable salt thereof, the solvate thereof, the solvate of the pharmaceutically acceptable salt thereof, or the pharmaceutical composition; the medicament is used for preventing and/or treating a disease related to 1, 2, or 3 of TNF-$\alpha$, IL-6, and IL-1$\beta$.

The present disclosure also provides a use of a substance Y in the manufacture of a medicament; the substance Y is the quinazoline compound of formula I, the pharmaceutically acceptable salt thereof, the solvate thereof, the solvate of the pharmaceutically acceptable salt thereof, or the pharmaceutical composition; the medicament is used for preventing and/or treating autoimmune diseases (such as rheumatoid arthritis, systemic lupus erythematosus, cutaneous lupus erythematosus, diabetes, Sjögren’s syndrome, multiple sclerosis), malaria, infectious diseases (such as AIDS, viral hepatitis, coronaviruses), allergic diseases (such as asthma), cardiovascular diseases, anti-rejection reactions after organ transplantation, chronic obstructive pulmonary disease, tumors, and other diseases related to the abnormal function and response state of the body’s immune system.

The term "halogen" refers to fluorine, chlorine, bromine, or iodine.

The term "alkyl" refers to a straight or branched alkyl with a specified number of carbon atoms (e.g., $C_1$-$C_4$). Alkyl groups include, but are not limited to, methyl, ethyl, *n*-propyl, isopropyl, *n*-butyl, *tert*-butyl, isobutyl, or *sec*-butyl, etc.

The term "alkoxy" refers to the group $R^X$-O-, wherein $R^X$ represents the alkyl group as defined above.

The term "oxo" refers to the group

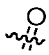

.

The term "hydrocarbon ring" refers to a cyclic, saturated, monocyclic group with a specified number of carbon atoms (e.g., $C_{3-6}$). Examples of hydrocarbon rings include, but are not limited to, cyclopropane, cyclobutane, cyclopentane, and cyclohexane.

The term "heterocycloalkyl" refers to a cyclic group with a specified number of ring atoms (e.g., 5- to 10-membered), a specified number of heteroatoms (e.g., 1, 2, or 3), and a specified type of heteroatoms (one or more than one of nitrogen (N), oxygen (O), and sulfur (S)). It can be a monocyclic, bridged, or spiro ring, and each ring is saturated. Heterocycloalkyl groups include, but are not limited to, azetidinyl, tetrahydropyrrolyl, tetrahydrofuran, morpholinyl, piperidinyl, etc.

The term "heterocycle" refers to a ring with a specified number of ring atoms (e.g., 3-to 6-membered), a specified number of heteroatoms (e.g., 1, 2, or 3), and a specified type of heteroatoms (one or more than one of nitrogen (N), oxygen (O), and sulfur (S)). It is a monocyclic ring. Examples of heterocycles include, but are not limited to, oxetane, tetrahydropyrrole, and tetrahydropyran.

The term "aryl" refers to a cyclic group composed solely of carbon atoms, with a specified number of carbon atoms (e.g., $C_6$-$C_{10}$). It can be monocyclic or polycyclic, with at least one ring being aromatic (conforming to Huckel’s rule). Aryl groups are connected to other moieties in the molecule either through an aromatic or a non-aromatic ring. Examples of aryl groups include, but are not limited to, phenyl, naphthyl, etc.

The term "heteroaryl" refers to a cyclic group with a specified number of ring atoms (e.g., 5- to 10-membered), a specified number of heteroatoms (e.g., 1, 2, or 3), and a specified type of heteroatoms (one or more than one of nitrogen (N), oxygen (O), and sulfur (S)). It can be monocyclic or polycyclic, with at least one ring being aromatic (conforming to Huckel’s rule). Heteroaryl groups are connected to other moieties in the molecule either through an aromatic or a non-aromatic ring. Heteroaryl groups include, but are not limited to, furanyl, pyrrolyl, thiophenyl, pyrazolyl, imidazolyl, oxazolyl, thiazolyl, pyridyl, pyrimidinyl, indolyl, etc. In a certain embodiment, the heteroaryl is monocyclic.

The "-" at the end of a group indicates that the group is connected at that point to other moieties in the molecule. For example, $CH_3$-C(=O)- represents an acetyl group.

In structural moieties,

"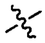"

indicates that the structural moiety is connected at that point to other moieties in the molecule. For example,

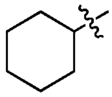

represents a cyclohexyl group.

When any variable (e.g., the group $R^{1-1}$) appears multiple times in the definition of a compound, their definitions are independent of each other and do not affect each other. For example, $C_6$-$C_{10}$ aryl substituted by 3 $R^{1-1}$ means that the $C_6$-$C_{10}$ aryl is substituted by 3 $R^{1-1}$, and the definitions of these 3 $R^{1-1}$ are independent and do not affect each other.

The term "pharmaceutically acceptable salt" refers to salts derived from the reaction of a compound with a pharmaceutically acceptable acid or base, which are relatively non-toxic, safe, and suitable for patient use. When a compound contains relatively acidic functional groups, the corresponding base addition salts can be obtained by contacting the free form of the compound with a sufficient amount of pharmaceutically acceptable base in an appropriate inert solvent. Pharmaceutically acceptable base addition salts include, but are not limited to, sodium salts, potassium salts, calcium salts, aluminum salts, magnesium salts, bismuth salts, ammonium salts, etc. When a compound contains relatively basic functional groups, acid addition salts can be obtained by contacting the free form of the compound with a sufficient amount of pharmaceutically acceptable acid in an appropriate inert solvent. Pharmaceutically acceptable acid addition salts include, but are not limited to, hydrochloride salts, sulfate salts, methanesulfonate salts, etc. For details, refer to Handbook of Pharmaceutical Salts: Properties, Selection, and Use (P. Heinrich Stahl, 2002).

The term "solvate" refers to a substance formed when a compound crystallizes with a solvent, which includes, but is not limited to, water, methanol, ethanol, etc. Solvates are classified into stoichiometric solvates and non-stoichiometric solvates.

The term "solvate of the pharmaceutically acceptable salt" refers to a substance formed by the combination of a compound with a pharmaceutically acceptable (relatively non-toxic, safe, and suitable for patient use) acid or base, and a solvent (including but not limited to water, methanol, ethanol, etc.). Here, the "pharmaceutically acceptable salt" carries the same meaning as defined above, and the solvent can be either stoichiometric or non-stoichiometric. The solvate of the pharmaceutically acceptable salt include, but is not limited to, hydrochloride monohydrate.

The term "therapeutically effective amount" refers to an amount of a compound administered to a patient that is sufficient to effectively treat a disease. The therapeutically effective amount will vary depending on the compound, type of disease, severity of the disease, patient's age, etc., but can be adjusted by those skilled in the art as required.

The term "pharmaceutical excipient" refers to the formulating agents and additives used in the manufacture of drugs and the compounding of prescriptions. They are substances contained in a pharmaceutical formulation, apart from the active ingredients. For specific reference, see the Pharmacopoeia of the People's Republic of China (2020 Edition) or the Handbook of Pharmaceutical Excipients (Raymond C Rowe, 2009).

The term "treat/treatment" refers to any of the following situations: (1) alleviating one or more biological manifestations of a disease; (2) interfering with one or more points in the biological cascade that initiates the disease; (3) slowing the progression of one or more biological manifestations of the disease.

The term "prevent/prevention" refers to reducing the risk of developing a disease.

The term "patient" refers to any animal who has already received or is about to receive treatment, preferably a mammal, most preferably a human. Mammals include, but are not limited to, cattle, horses, sheep, pigs, cats, dogs, mice, rats, rabbits, guinea pigs, monkeys, and humans.

In accordance with common knowledge in the art, the above various preferred conditions can be arbitrarily combined to obtain the various preferred embodiments of the present disclosure.

The reagents and raw materials used in the present disclosure are commercially available.

The advantageous progressive effect of the present disclosure is that the quinazoline compound of the present disclosure is novel in structure and exhibits good inhibitory effects on cytokines TNF-$\alpha$, IL-6, and IL-1$\beta$.

DETAILED DESCRIPTION OF THE PREFERRED EMBODIMENT

The present disclosure is further described through examples below, but is not limited to the scope of the examples provided. In the following examples, experimental methods without specified conditions are carried out according to conventional methods and conditions, or are selected according to the product instructions.

Example 1: 4-(4-((7-Chloroquinolin-4-yl)amino)pentyl)thiomorpholine-1,1-dioxide (compound 1)

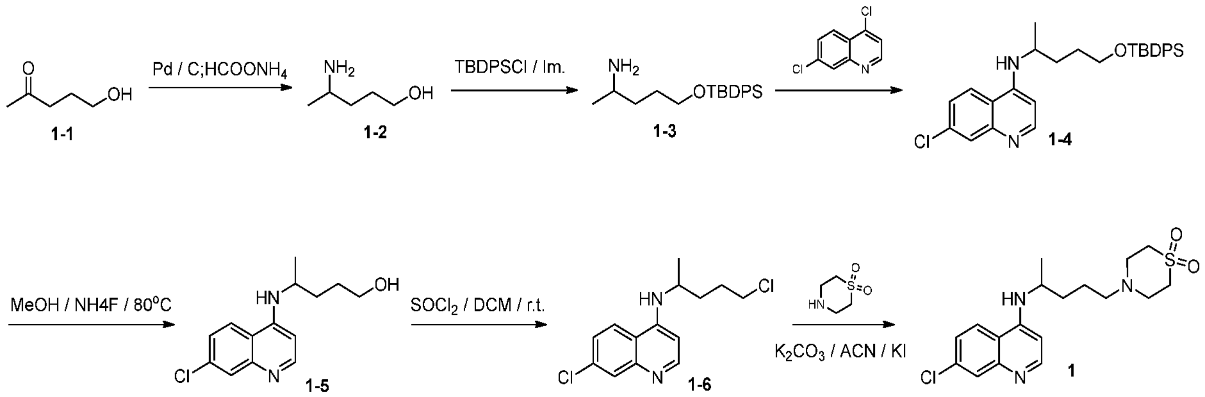


Step 1: Preparation of 4-amino-1-pentanol (compound 1-2)

Under a hydrogen atmosphere at room temperature, 5-hydroxy-2-pentanone (5.0 g, 48.96 mmol) and ammonium formate (10.0 g, 158.58 mmol) were added to a mixture of palladium on carbon (1 g) in methanol (200 mL). The reaction mixture was stirred at room temperature for 48 hours. The reaction mixture was concentrated under reduced pressure, and the residual liquid was slurried with ethyl acetate (200 mL). The mixture was filtered and concentrated under reduced pressure to dryness to obtain the crude 4-amino-1-pentanol (4 g, yield of 79.22%). LC-MS: m/z 104.1 $[M+H]^+$.

Step 2: Preparation of 5-((*tert*-butyldiphenylsilyl)oxy)pentan-2-amine (compound 1-3)

Under an argon atmosphere at room temperature, 4-amino-1-pentanol (4.0 g, 38.83 mmol), imidazole (5.28 g, 77.67 mmol), and *tert*-butyldiphenylchlorosilane (16.02 g, 58.25 mmol) were added to a solution of dichloromethane (200 mL). The reaction mixture was stirred at room temperature for 18 hours. After the reaction was completed, the reaction mixture was concentrated, and then water and ethyl acetate were added thereto. The mixture was extracted with ethyl acetate three times. The organic phase was dried, filtered, and concentrated. The residue was separated by column chromatography to obtain the product 5-((*tert*-butyldiphenylsilyl)oxy)pentan-2-amine (5.4 g, yield of 40.79%). LC-MS: m/z 342.2 $[M+H]^+$.

Step 3: Preparation of *N*-(5-((*tert*-butyldiphenylsilyl)oxy)pentan-2-yl)-7-chloroquinolin-4-amine (compound 1-4)

The starting material 5-((*tert*-butyldiphenylsilyl)oxy)pentan-2-amine (3.8 g, 11.14 mmol) was dissolved in 40 mL of 1,4-dioxane. $Pd_2(dba)_3$ (50 mg), BINAP (40 mg), $Cs_2CO_3$ (7.25 g, 22.25 mmol), and 4,7-dichloroquinoline (2.0 g, 10.1 mmol) were added thereto, respectively. The reaction mixture was heated to 110°C under an argon atmosphere and reacted for 18 hours. After the reaction was completed, the reaction mixture was concentrated. The residue was separated by column chromatography to obtain the product (4 g, yield of 78.72%). LC-MS: m/z 503.2 $[M+H]^+$.

Step 4: Preparation of 4-((7-chloroquinolin-4-yl)amino)pentan-1-ol (compound 1-5)

The starting material *N*-(5-((*tert*-butyldiphenylsilyl)oxy)pentan-2-yl)-7-chloroquinolin-4-amine (4.0 g, 7.95 mmol) was dissolved in 40 mL of methanol. Ammonium fluoride (2.0 g, 54.05 mmol) was added thereto, and the reaction mixture was heated to reflux under an argon atmosphere and reacted for 2 hours. After the reaction was completed, the reaction mixture was concentrated. The residue was separated by preparative liquid chromatography to obtain the product (1.6 g, yield of 76.19%). LC-MS: m/z 265.1 $[M+H]^+$.

Step 5: Preparation of 7-chloro-*N*-(5-chloropentan-2-yl)quinolin-4-amine (compound 1-6)

The starting material 4-((7-chloroquinolin-4-yl)amino)pentan-1-ol (2.0 g, 7.58 mmol) was dissolved in 40 mL of dichloromethane. Thionyl chloride (2.0 g, 16.81 mmol) and a catalytic amount of *N,N*-dimethylformamide (0.1 mL) were added thereto. The reaction mixture was reacted at room temperature under an argon atmosphere for 2 hours. After the

reaction was completed, the reaction mixture was concentrated. The residue was quenched with sodium bicarbonate solution and extracted with ethyl acetate. The organic phase was dried, filtered, and concentrated to obtain the intermediate product (2.1 g, yield of 97.95%), which was used directly in the next reaction step. LC-MS: m/z 283.1 $[M+H]^+$.

Step 6: Preparation of 4-(4-((7-chloroquinolin-4-yl)amino)pentyl)thiomorpholine-1,1-dioxide (compound 1)

The starting material 7-chloro-*N*-(5-chloropentan-2-yl)quinolin-4-amine (80 mg, 0.28 mmol) was dissolved in 20 mL of acetonitrile. KI (10 mg), $K_2CO_3$ (100 mg, 0.73 mmol), and thiomorpholine-1,1-dioxide (150 mg, 1.11 mmol) were added thereto. The reaction mixture was heated to 60 to 70°C under an argon atmosphere and reacted for 48 hours. After the reaction was completed, the reaction mixture was concentrated. The residue was separated by column chromatography to obtain the product (15 mg, yield of 13.89%). $^1H$ NMR (400 MHz, $CDCl_3$) δ 8.47 (d, *J* = 5.6 Hz, 1H), 7.98 (d, *J* = 1.8 Hz, 1H), 7.87 (d, *J* = 9.0 Hz, 1H), 7.35 (dd, *J* = 8.9, 2.0 Hz, 1H), 6.45 (d, *J* = 5.7 Hz, 1H), 5.57 (s, 1H), 3.78 (dd, *J* = 13.4, 6.8 Hz, 1H), 3.05 (d, *J* = 6.1 Hz, 4H), 2.99 (d, *J* = 5.9 Hz, 4H), 2.56 (t, *J* = 6.9 Hz, 2H), 1.86 - 1.76 (m, 1H), 1.76 - 1.56 (m, 4H), 1.39 (d, *J* = 6.3 Hz, 3H). LC-MS: m/z 382.1 $[M+H]^+$.

Examples 2 to 12

To prepare the compounds in examples 2 to 12, the same method as described in example 1 was applied. Specific experimental data for these compounds are shown in Table 1.

Table 1: Experimental data for compounds 1 to 12

| Example Number | Compound structural formula and number | Naming and structural identification data |
|---|---|---|
| 1 | 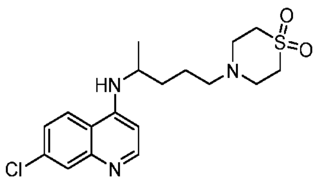 | 4-(4-((7-Chloroquinolin-4-yl)amino)pentyl)thiomorpholine-1,1-dioxide<br>$^1H$ NMR (400 MHz, $CDCl_3$) δ 8.47 (d, *J* = 5.6 Hz, 1H), 7.98 (d, *J* = 1.8 Hz, 1H), 7.87 (d, *J* = 9.0 Hz, 1H), 7.35 (dd, *J* = 8.9, 2.0 Hz, 1H), 6.45 (d, *J* = 5.7 Hz, 1H), 5.57 (s, 1H), 3.78 (dd, *J* = 13.4, 6.8 Hz, 1H), 3.05 (d, *J* = 6.1 Hz, 4H), 2.99 (d, *J* = 5.9 Hz, 4H), 2.56 (t, *J* = 6.9 Hz, 2H), 1.86 - 1.76 (m, 1H), 1.76 - 1.56 (m, 4H), 1.39 (d, *J* = 6.3 Hz, 3H). LC-MS: m/z 382.1 $[M+H]^+$. |
| **2** | 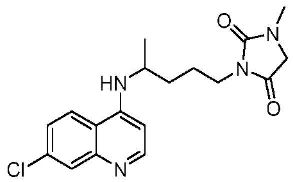 | 3-(4-((7-Chloroquinolin-4-yl)amino)pentyl)-1-methylimidazolidine-2,4-dione<br>$^1H$ NMR (400 MHz, $CDCl_3$) δ 8.51 (d, *J* = 4.3 Hz, 1H), 7.97 (s, 1H), 7.80 (d, *J* = 8.7 Hz, 1H), 7.36 (d, *J* = 8.3 Hz, 1H), 6.42 (d, *J* = 4.2 Hz, 1H), 5.29 (d, *J* = 5.8 Hz, 1H), 3.90 - 3.71 (m, 3H), 3.58 (d, *J* = 6.9 Hz, 2H), 2.97 (s, 3H), 1.89 - 1.63 (m, 4H), 1.32 (d, *J*= 5.5 Hz, 3H). LC-MS: m/z 361.1 $[M+H]^+$. |
| **3** | 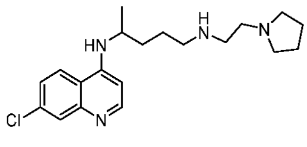 | *N*[4]-(7-Chloroquinolin-4-yl)-*N*[1]-(2-(pyrrolidin-1-yl)ethyl)pentane-1,4-diamine<br>$^1H$ NMR (400 MHz, $CDCl_3$) δ 8.52 (d, *J* = 5.4 Hz, 1H), 7.96 (d, *J* = 2.1 Hz, 1H), 7.77 (d, *J* = 8.9 Hz, 1H), 7.36 (dd, *J* = 8.9, 2.2 Hz, 1H), 6.42 (d, *J* = 5.5 Hz, 1H), 5.58 (s, 1H), 3.73 (s, 1H), 2.77 (t, *J* = 6.0 Hz, 2H), 2.74 - 2.64 (m, 4H), 2.57 (d, *J* = 6.5 Hz, 4H), 1.89 - 1.77 (m, 5H), 1.75 - 1.62 (m, 3H), 1.34 (d, *J* = 6.3 Hz, 3H). LC-MS: m/z 361.0 $[M+H]^+$. |

(continued)

| Example Number | Compound structural formula and number | Naming and structural identification data |
|---|---|---|
| **4** | 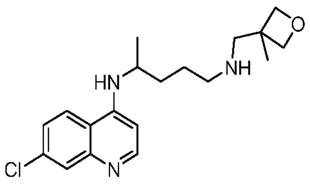 | *N*1-(7-Chloroquinolin-4-yl)-*N*1-((3-methyloxetan-3-yl)methyl) pentane-1,4-diamine<br>$^1$H NMR (400 MHz, $CDCl_3$) δ 8.53 (d, *J* = 5.4 Hz, 1H), 7.98 (d, *J* = 2.1 Hz, 1H), 7.68 (d, *J* = 9.0 Hz, 1H), 7.37 (dd, *J* = 8.9, 2.1 Hz, 1H), 6.44 (d, *J* = 5.5 Hz, 1H), 5.08 (d, *J* = 6.8 Hz, 1H), 4.48 (d, *J* = 5.7 Hz, 2H), 4.39 (d, *J* = 5.7 Hz, 2H), 3.75 (dd, *J* = 12.7, 6.5 Hz, 1H), 2.82 (s, 2H), 2.71 (t, *J* = 6.6 Hz, 2H), 1.77 (ddd, *J* = 22.8, 13.3, 6.5 Hz, 4H), 1.39 - 1.32 (m, 6H). LC-MS: m/z 348.0 $[M+H]^+$. |
| **5** | 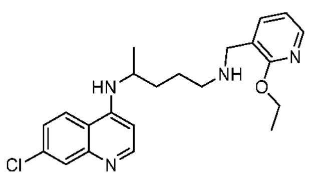 | *N*4-(7-Chloroquinolin-4-yl)-*N*1-((2-ethoxypyridin-3-yl)methyl) pentane-1,4-diamine<br>$^1$H NMR (400 MHz, MeOD) δ 8.40 (dd, *J* = 7.5, 6.4 Hz, 2H), 8.16 (dd, *J* = 5.1, 1.8 Hz, 1H), 7.85 (d, *J* = 2.0 Hz, 1H), 7.78 (dd, *J* = 7.3, 1.8 Hz, 1H), 7.52 (dd, *J* = 9.1, 2.1 Hz, 1H), 6.97 (dd, *J* = 7.3, 5.1 Hz, 1H), 6.74 (d, *J* = 6.4 Hz, 1H), 4.42 (dd, *J* = 11.5, 4.4 Hz, 2H), 4.00 (dd, *J* = 12.4, 6.1 Hz, 1H), 3.25 (s, 2H), 3.06 (t, *J* = 7.5 Hz, 2H), 1.97 - 1.74 (m, 4H), 1.42 - 1.38 (m, 7H). LC-MS: m/z 399.0 $[M+H]^+$. |
| **6** | 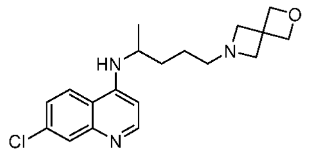 | N (5-(2-Oxa-6-azaspiro[3.3]heptan-6-yl)pentan-2-yl)-7-chloroquinolin-4-amine<br>$^1$H NMR (400 MHz, $CDCl_3$) δ 8.47 (d, *J* = 5.4 Hz, 1H), 7.93 (d, *J* = 2.1 Hz, 1H), 7.70 (d, *J* = 9.0 Hz, 1H), 7.32 (dd, *J* = 8.9, 2.1 Hz, 1H), 6.38 (d, *J* = 5.5 Hz, 1H), 5.57 (d, *J* = 6.2 Hz, 1H), 4.71 (s, 4H), 3.66 (dt, *J* = 12.3, 6.1 Hz, 1H), 3.31 (s, 4H), 2.46 - 2.32 (m, 2H), 1.65 (tdd, *J* = 13.9, 11.4, 6.4 Hz, 2H), 1.52 - 1.40 (m, 2H), 1.28 (d, *J* = 6.3 Hz, 3H). LC-MS: m/z 346.0 $[M+H]^+$. |
| **7** | 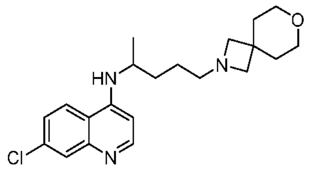 | N (5-(7-Oxa-2-azaspiro[3.5]nonan-2-yl)pentan-2-yl)-7-chloroquinolin-4-amine<br>$^1$H NMR (400 MHz, $CDCl_3$) δ 8.48 (d, *J* = 5.5 Hz, 1H), 7.95 (d, *J* = 1.8 Hz, 1H), 7.83 (d, *J* = 8.9 Hz, 1H), 7.33 (dd, *J* = 8.9, 1.9 Hz, 1H), 6.41 (d, *J* = 5.5 Hz, 1H), 5.77 (s, 1H), 3.71 (d, *J* = 4.8 Hz, 1H), 3.63 - 3.54 (m, 4H), 3.14 (s, 4H), 2.67 - 2.51 (m, 2H), 1.85 - 1.74 (m, 5H), 1.67 (dd, *J* = 13.3, 7.1 Hz, 1H), 1.63 - 1.52 (m, 2H), 1.31 (d, *J* = 6.3 Hz, 3H). LC-MS: m/z 374.0 $[M+H]^+$. |
| **8** | 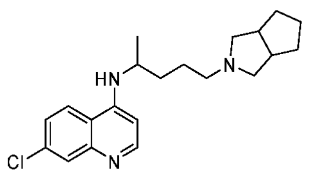 | 7-Chloro-*N*-(5-(hexahydrocyclopenta[*c*]pyrrol-2(1*H*-yl)pentan-2-yl) quinolin-4-amine<br>$^1$H NMR (400 MHz, $CDCl_3$) δ 8.49 (d, *J* = 5.4 Hz, 1H), 7.93 (d, *J* = 2.1 Hz, 1H), 7.73 (d, *J* = 9.0 Hz, 1H), 7.32 (dd, *J* = 8.9, 2.2 Hz, 1H), 6.41 (d, *J* = 5.5 Hz, 1H), 5.48 (d, *J* = 6.6 Hz, 1H), 3.70 (dd, *J* = 11.7, 5.9 Hz, 1H), 2.90 - 2.84 (m, 1H), 2.84 - 2.76 (m, 1H), 2.61 (d, *J* = 2.4 Hz, 2H), 2.44 (dt, *J* = 12.1, 7.1 Hz, 1H), 2.41 - 2.32 (m, 1H), 2.02 - 1.91 (m, 2H), 1.86 - 1.74 (m, 1H), 1.69 - 1.49 (m, 7H), 1.41 (ddd, *J* = 15.3, 6.0, 3.2 Hz, 2H), 1.31 (d, *J* = 6.3 Hz, 3H). LC-MS: m/z 358.0 $[M+H]^+$. |

(continued)

| Example Number | Compound structural formula and number | Naming and structural identification data |
|---|---|---|
| **9** | 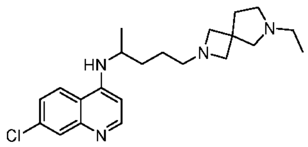 | 7-Chloro-N-(5-(6-ethyl-2,6-diazaspiro[3.4]octan-2-yl)pentan-2-yl) quinolin-4-amine<br>$^1$H NMR (400 MHz, $CDCl_3$) δ 8.48 (d, *J* = 5.5 Hz, 1H), 7.93 (d, *J* = 2.1 Hz, 1H), 7.78 (d, *J* = 8.9 Hz, 1H), 7.33 (dd, *J* = 8.9, 2.1 Hz, 1H), 6.39 (d, *J* = 5.6 Hz, 1H), 3.68 (d, *J* = 5.9 Hz, 1H), 3.31 - 3.25 (m, 2H), 3.17 (d, *J* = 7.7 Hz, 2H), 2.74 (d, *J* = 10.5 Hz, 2H), 2.62 - 2.54 (m, 2H), 2.54 - 2.40 (m, 4H), 2.03 (dd, *J* = 8.9, 5.2 Hz, 2H), 1.75 (dt, *J* = 14.3, 7.2 Hz, 1H), 1.70 - 1.59 (m, 1H), 1.58 - 1.48 (m, 2H), 1.28 (d, *J* = 6.3 Hz, 3H), 1.11 (t, *J* = 7.2 Hz, 3H). LC-MS: m/z 387.0[M+H]$^+$. |
| **10** | 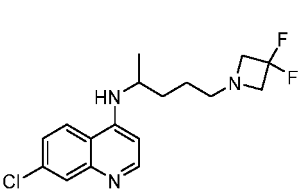 | 7-Chloro-*N*-(5-(3,3-difluoroazetidin-1-yl)pentan-2-yl)quinolin-4-amine<br>$^1$H NMR (400 MHz, $CDCl_3$) δ 8.51 (d, *J* = 5.4 Hz, 1H), 7.97 (s, 1H), 7.74 (d, *J* = 8.9 Hz, 1H), 7.37 (d, *J* = 8.8 Hz, 1H), 6.43 (d, *J* = 5.4 Hz, 1H), 5.52 (d, *J* = 6.1 Hz, 1H), 3.73 (dt, *J* = 12.7, 6.3 Hz, 1H), 3.57 (t, *J* = 11.9 Hz, 4H), 2.69 - 2.53 (m, 2H), 1.75 (qd, *J* = 14.1, 7.5 Hz, 2H), 1.63 - 1.51 (m, 2H), 1.34 (d, *J* = 6.3 Hz, 3H). LC-MS: m/z 340.0 [M+H]$^+$. |
| **11** | 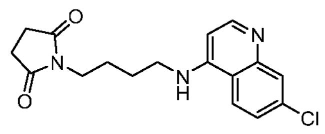 | 1-(4-((7-Chloroquinolin-4-yl)amino)butyl)pyrrolidine-2,5-dione<br>$^1$H NMR (400 MHz, *d*-DMSO) δ 8.45 - 8.34 (d, *J* = 5.2 Hz, 1H), 8.27 (d, *J* = 9.0 Hz, 1H), 7.78 (d, *J* = 2.2 Hz, 1H), 7.46 (dd, *J* = 8.8, 2.4 Hz, 1H), 7.37 - 7.24 (m, 1H), 6.48 (d, *J* = 5.5 Hz, 1H), 3.42 (t, *J* = 6.4 Hz, 2H), 3.28 (m, 2H), 2.61(s,4H), 1.69 - 1.51 (m, 4H). LC-MS: m/z: 332.1 [M+H]$^+$. |
| **12** | 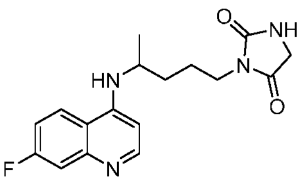 | 3-(4-((7-Fluoroquinolin-4-yl)amino)pentyl)imidazolidine-2,4-dione<br>$^1$H NMR (400 MHz, $CDCl_3$) δ 8.52 (d, *J* = 5.5 Hz, 1H), 7.86 (dd, *J* = 9.2, 5.7 Hz, 1H), 7.62 (dd, *J* = 10.3, 2.6 Hz, 1H), 7.26 - 7.15 (m, 1H), 6.43 (d, *J* = 5.6 Hz, 1H), 5.63 (s, 1H), 5.27 (d, *J* = 6.8 Hz, 1H), 3.97 (s, 2H), 3.81 (dt, *J* = 13.5, 6.7 Hz, 1H), 3.67 - 3.53 (m, 2H), 1.82 (dd, *J* = 13.9, 6.4 Hz, 2H), 1.71 (dt, *J* = 14.0, 6.4 Hz, 2H), 1.34 (d, *J* = 6.4 Hz, 3H). LC-MS: m/z 331.0 [M+H]$^+$. |

Example 13: 3-(4-((7-Chloroquinazolin-4-yl)amino)pentyl)imidazolidine-2,4-dione (compound 13)

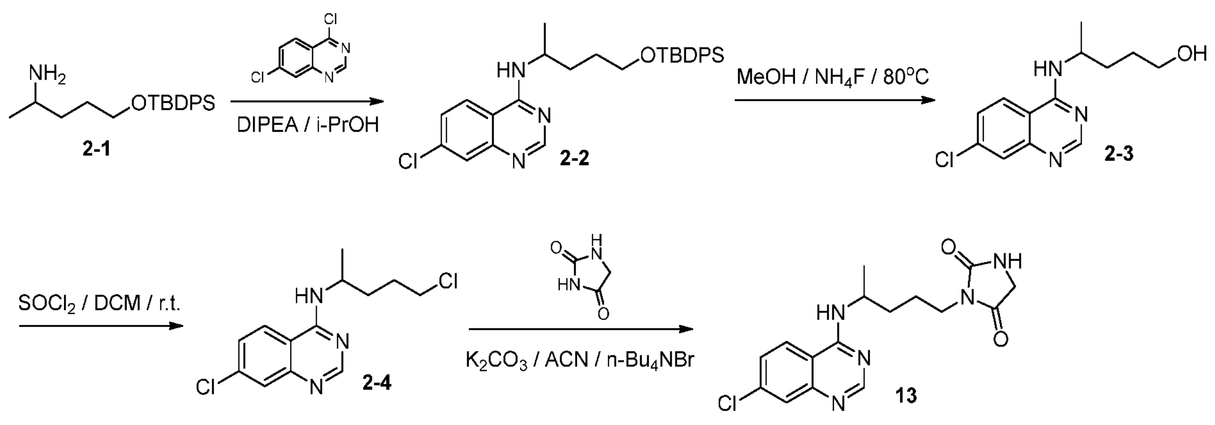

Step 1: Preparation of *N*-(5-((*tert*-butyldiphenylsilyl)oxy)pentan-2-yl)-7-chloroquinazolin-4-amine (compound 2-2)

The starting material 5-((*tert*-butyldiphenylsilyl)oxy)pentan-2-amine (2.8 g, 8.20 mmol) was dissolved in 40 mL of isopropanol. DIPEA (3.5 g, 27.08 mmol) and 4,7-dichloroquinazoline (2.0 g, 10.05 mmol) were then added thereto. The reaction mixture was heated to 90°C under an argon atmosphere and reacted for 18 hours. After the reaction was completed, the reaction mixture was concentrated. The residue was subjected to preparative separation to obtain the product (3.1 g, yield of 75.01%). LC-MS: m/z 504.2 $[M+H]^+$.

Step 2: Preparation of 4-((7-chloroquinazolin-4-yl)amino)pentan-1-ol (compound 2-3)

The starting material *N*-(5-((*tert*-butyldiphenylsilyl)oxy)pentan-2-yl)-7-chloroquinazolin-4-amine (3.1 g, 6.15 mmol) was dissolved in 40 mL of methanol. Ammonium fluoride (2.0 g, 54.05 mmol) was added thereto, and the reaction mixture was heated to reflux under an argon atmosphere and reacted for 2 hours. After the reaction was completed, the reaction mixture was concentrated. The residue was separated by preparative liquid chromatography to obtain the product (1.3 g, yield of 79.75%). LC-MS: m/z 266.1 $[M+H]^+$.

Step 3: Preparation of 7-chloro-*N*-(5-chloropentan-2-yl)quinazolin-4-amine (compound 2-4)

The starting material 4-((7-chloroquinolin-4-yl)amino)pentan-1-ol (1.0 g, 3.76 mmol) was dissolved in 20 mL of dichloromethane. Thionyl chloride (1.0 g, 8.40 mmol) and a catalytic amount of *N,N*-dimethylformamide (0.1 mL) were added thereto. The reaction mixture was reacted at room temperature under an argon atmosphere for 2 hours. After the reaction was completed, the reaction mixture was concentrated. The residue was quenched with sodium bicarbonate solution and extracted with ethyl acetate. The organic phase was dried, filtered, and concentrated to obtain the intermediate product (1.0 g, yield of 93.66%), which was used directly in the next reaction step. LC-MS: m/z 284.1 $[M+H]^+$.

Step 4: Preparation of 3-(4-((7-chloroquinazolin-4-yl)amino)pentyl)imidazolidine-2,4-dione (compound 13)

The starting material 7-chloro-*N*-(5-chloropentan-2-yl)quinazolin-4-amine (100 mg, 0.35 mmol) was dissolved in 20 mL of acetonitrile. *n*-$Bu_4NBr$ (10 mg), $K_2CO_3$ (100 mg, 0.73 mmol), and imidazolidine-2,4-dione (100 mg, 1.00 mmol) were added thereto, respectively. The reaction mixture was heated to 60 to 70°C under an argon atmosphere and reacted for 48 hours. After the reaction was completed, the reaction mixture was concentrated. The residue was separated by column chromatography to obtain the product (54 mg, yield of 44.12%). $^1H$ NMR (400 MHz, $CDCl_3$) δ 8.62 (s, 1H), 7.81 (t, *J* = 5.5 Hz, 2H), 7.41 (dd, *J* = 8.8, 2.1 Hz, 1H), 6.13 (d, *J* = 8.0 Hz, 1H), 5.74 (s, 1H), 4.66 - 4.52 (m, 1H), 4.01 (d, *J* = 0.9 Hz, 2H), 3.68 - 3.56 (m, 2H), 1.90 - 1.80 (m, 2H), 1.72 - 1.60 (m, 2H), 1.32 (d, *J* = 6.5 Hz, 3H). LC-MS: m/z 348.1 $[M+H]^+$.

Example 14

To prepare the compounds in example 14, the same method as described in example 13 was applied. Specific experimental data are shown in Table 2.

Table 2: Experimental data for compounds 13 and 14

| | | |
|---|---|---|
| **13** | 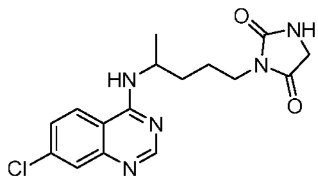 | 3-(4-((7-Chloroquinazolin-4-yl)amino)pentyl)imidazolidine-2,4-dione<br>$^1H$ NMR(400 MHz, $CDCl_3$)δ 8.62 (s, 1H), 7.81 (t, *J* = 5.5 Hz, 2H), 7.41 (dd, *J* = 8.8, 2.1 Hz, 1H), 6.13 (d, *J* = 8.0 Hz, 1H), 5.74 (s, 1H), 4.66 - 4.52 (m, 1H), 4.01 (d, *J* = 0.9 Hz, 2H), 3.68 - 3.56 (m, 2H), 1.90 - 1.80 (m, 2H), 1.72 - 1.60 (m, 2H), 1.32 (d, *J* = 6.5 Hz, 3H). LC-MS: m/z 348.1 $[M+H]^+$. |
| **14** | 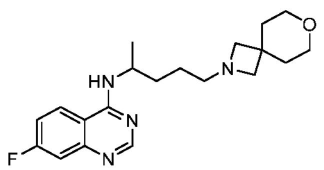 | *N*-(5-(7-Oxa-2-azaspiro[3.5]nonan-2-yl)pentan-2-yl)-7-fluoroquinazolin-4-amine<br>$^1H$ NMR (400 MHz, $CDCl_3$) δ 8.58 (s, 1H), 8.16 (dd, *J* = 9.0, 5.8 Hz, 1H), 7.42 (dd, *J* = 9.8, 2.1 Hz, 1H), 7.21 (dd, *J* = 11.7, 5.4 Hz, 1H), 6.70 (s, 1H), 4.55 (s, 1H), 3.63 - 3.55 (m, 4H), 3.51 (s, 4H), 3.01 - 2.85 (m, 2H), 1.96 (dd, *J* = 14.7, 6.4 Hz, 1H), 1.91 - 1.80 (m, 4H), 1.72 (d, *J* = 5.5 Hz, 3H), 1.34 (d, *J* = 6.5 Hz, 3H). LC-MS: m/z 359.2 $[M+H]^+$. |

Example 15: 1-(4-((7-Chloroquinazolin-4-yl)amino)pentyl)pyrrolidine-2,5-dione (compound 15)

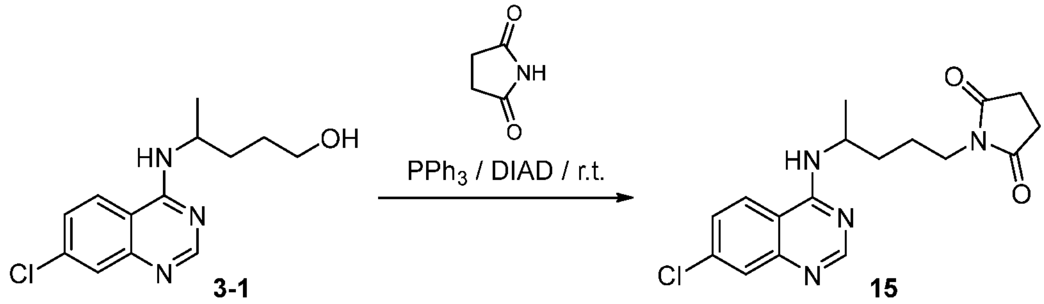


3-1 15

Step 1: Preparation of 1-(4-((7-chloroquinazolin-4-yl)amino)pentyl)pyrrolidine-2,5-dione (compound 15)

The starting material 4-((7-chloroquinazolin-4-yl)amino)pentan-1-ol (50 mg, 0.19 mmol) was dissolved in 20 mL of anhydrous tetrahydrofuran. $PPh_3$ (280 mg, 1.07 mmol), DIAD (240 mg, 1.19 mmol), and pyrrolidin-2,5-dione (100 mg, 1.01 mmol) were added thereto, respectively. The reaction mixture was reacted at room temperature under an argon atmosphere for 48 hours. After the reaction was completed, the reaction mixture was concentrated. The residue was separated by column chromatography to obtain the product (57 mg, yield of 87.10%). $^1$H NMR (400 MHz, $CDCl_3$) δ 8.63 (s, 1H), 7.82 (d, *J* = 2.0 Hz, 1H), 7.78 (d, *J* = 8.8 Hz, 1H), 7.42 (dd, *J* = 8.8, 2.1 Hz, 1H), 5.97 (d, *J* = 7.9 Hz, 1H), 4.63 - 4.51 (m, 1H), 3.67 - 3.52 (m, 2H), 2.74 (s, 4H), 1.76 - 1.60 (m, 4H), 1.32 (d, *J* = 6.5 Hz, 3H). LC-MS: m/z 348 $[M+H]^+$.

Table 3: Experimental data for compound 15

| | | |
|---|---|---|
| **15** | 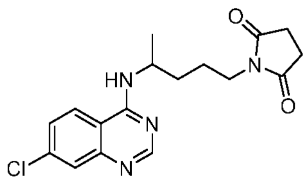 | 1-(4-((7-Chloroquinazolin-4-yl)amino)pentyl)pyrrolidine-2,5-dione<br>$^1$H NMR (400 MHz, $CDCl_3$) δ 8.63 (s, 1H), 7.82 (d, *J* = 2.0 Hz, 1H), 7.78 (d, *J* = 8.8 Hz, 1H), 7.42 (dd, *J* = 8.8, 2.1 Hz, 1H), 5.97 (d, *J* = 7.9 Hz, 1H), 4.63 - 4.51 (m, 1H), 3.67 - 3.52 (m, 2H), 2.74 (s, 4H), 1.76 - 1.60 (m, 4H), 1.32 (d, *J* = 6.5 Hz, 3H). LC-MS: m/z 348 $[M+H]^+$. |

Example 16: 2-((4-((7-Chloroquinazolin-4-yl)amino)pentyl)(oxazol-2-ylmethyl)amino)ethan-1-ol (compound 16)

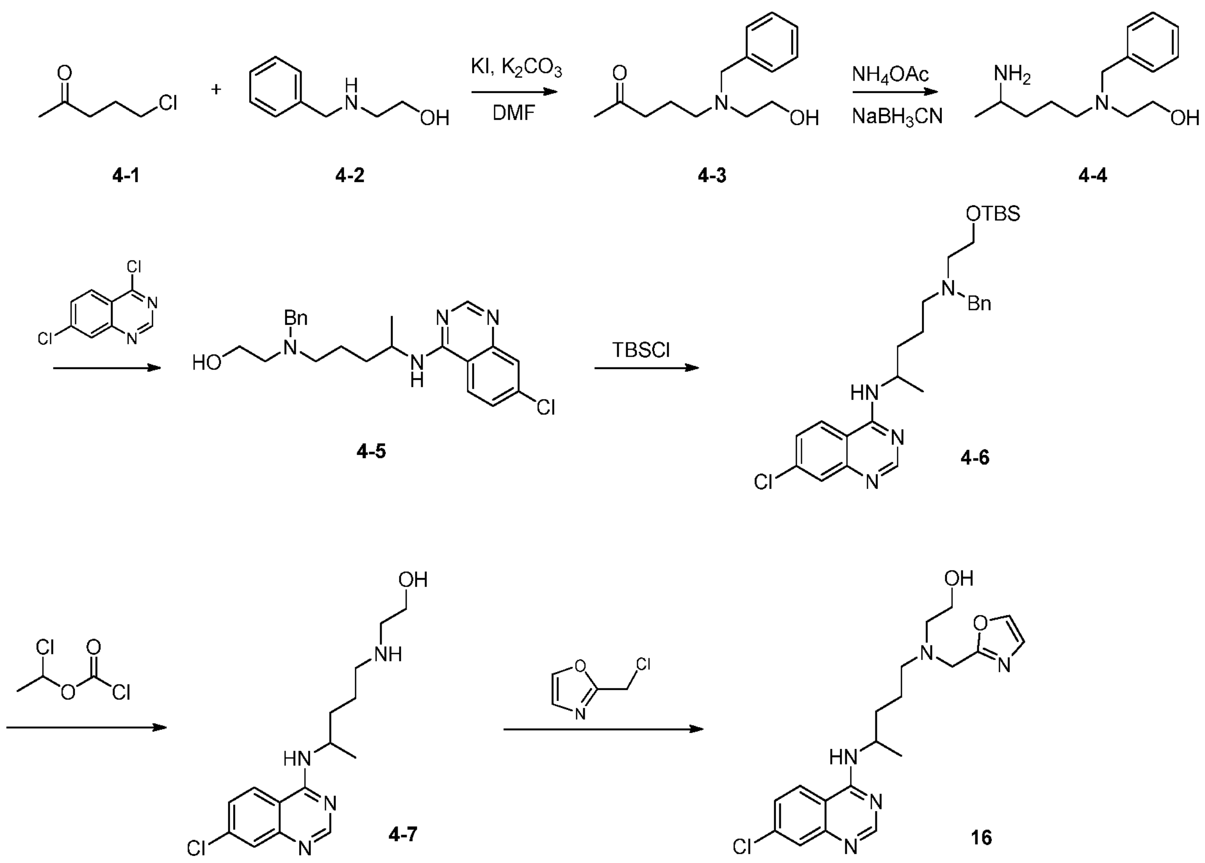


4-1 4-2 4-3 4-4

4-5 4-6

4-7 16

Step 1: Preparation of 5-(benzyl(2-hydroxyethyl)amino)pentan-2-one (compound 4-3)

At room temperature, the starting materials 2-(benzylamino)ethanol (17.5 g, 116 mmol), 5-chloropentan-2-one (28 g, 232 mmol), potassium carbonate (24 g, 174 mmol), and potassium iodide (1.9 g, 12 mmol) were added to *N,N*-dimethylformamide (300 mL). The reaction mixture was reacted overnight at 120°C under an argon atmosphere. After *N,N*-dimethylformamide was evaporated until dryness, water was added thereto, and the mixture was extracted with dichloromethane three times. The organic phase was washed with saturated brine, dried over anhydrous sodium sulfate, filtered, and then subjected to rotary evaporation until dryness. The residue was purified by column chromatography to obtain a yellow oil, 5-(benzyl(2-hydroxyethyl)amino)pentan-2-one (23 g, 98 mmol, yield of 84.5%). LC-MS: m/z 236 $[M+H]^+$.

Step 2: Preparation of 2-[4-aminopentyl(benzyl)amino]ethanol (compound 4-4)

The starting material 5-(benzyl(2-hydroxyethyl)amino)pentan-2-one (9 g, 38 mmol) was dissolved in methanol (200 mL). Ammonium acetate (17.7 g, 230 mmol) was added at room temperature and the reaction mixture was stirred for 30 minutes. Subsequently, sodium cyanoborohydride (5 g, 80 mmol) was added thereto, and the reaction mixture was stirred at room temperature overnight. After methanol was evaporated until dryness, 40% sodium hydroxide aqueous solution (200 mL) was added thereto, and the mixture was extracted three times with dichloromethane. The organic phase was washed with saturated brine, dried over anhydrous sodium sulfate, filtered, and then subjected to rotary evaporation until dryness. The residue was purified by column chromatography to obtain a yellow oil, 2-[4-aminopentyl(benzyl)amino]ethanol (2.45 g, 10.4 mmol, yield of 27.1%). LC-MS: m/z 237 $[M+H]^+$.

Step 3: Preparation of 2-(benzyl(4-((7-chloroquinazolin-4-yl)amino)pentyl)amino)ethanol (compound 4-5)

The starting materials 2-[4-aminopentyl(benzyl)amino]ethanol (2.4 g, 10 mmol) and 4,7-dichloroquinazoline (2.26 g, 11.4 mmol) were added to *N,N*-diisopropylethylamine (7.5 g). The reaction mixture was stirred at 90°C overnight. After *N,N-diisopropylethylamine* was evaporated until dryness, water was added thereto, and the mixture was extracted with dichloromethane three times. The organic phase was washed with saturated brine, dried over anhydrous sodium sulfate, filtered, and then subjected to rotary evaporation until dryness. The residue was purified by column chromatography to obtain a yellow oil, 2-(benzyl(4-((7-chloroquinazolin-4-yl)amino)pentyl)amino)ethanol (2.2 g, 5.5 mmol, yield of 54%). LC-MS: m/z 399 $[M+H]^+$.

Step 4: Preparation of *N*1-benzyl-*N*1-(2-((*tert*-butyldimethylsilyl)oxy)ethyl)-*N*4-(7-chloroquinazolin-4-yl)pentane-1,4-diamine (compound 4-6)

The starting materials 2-(benzyl(4-((7-chloroquinazolin-4-yl)amino)pentyl)amino)ethanol (2.2 g, 5.5 mmol) and imidazole (0.752 g, 11.0 mmol) were dissolved in anhydrous dichloromethane (30 mL). *tert*-Butyldimethylchlorosilane (1 g, 6.6 mmol) was added dropwise under an ice bath, and then the reaction mixture was stirred at room temperature overnight. The reaction mixture was washed with water three times, dried over anhydrous sodium sulfate, filtered, and then subjected to rotary evaporation until dryness. The residue was purified by column chromatography to obtain a white solid, *N*1-benzyl-*N*1-(2-((*tert*-butyldimethylsilyl)oxy)ethyl)-*N*4-(7-chloroquinazolin-4-yl)pentane-1,4-diamine (1.4 g, 2.7 mmol, yield of 50%). LC-MS: m/z 513 $[M+H]^+$.

Step 5: Preparation of 2-((4-((7-chloroquinazolin-4-yl)amino)pentyl)amino)ethanol (compound 4-7)

The starting material *N*1-benzyl-*N*1-(2-((*tert*-butyldimethylsilyl)oxy)ethyl)-*N*4-(7-chloroquinazolin-4-yl)pentane-1,4-diamine (1.4 g, 2.7 mmol) was dissolved in anhydrous dichloromethane (30 mL). 1-Chloroethyl chloroformate (0.51 g, 3.6 mmol) was added dropwise under an ice bath, and the reaction mixture was stirred at room temperature for 2 hours. The dichloromethane was evaporated, and the residue was added with methanol and refluxed for 2 hours. After the methanol was evaporated, the residue was purified by column chromatography to obtain a yellow oil, 2-((4-((7-chloroquinazolin-4-yl)amino)pentyl)amino)ethanol (0.388 g, 1.26 mmol, yield of 46%). LC-MS: m/z 309 $[M+H]^+$.

Step 6: Preparation of 2-((4-((7-chloroquinazolin-4-yl)amino)pentyl)(oxazol-2-ylmethyl)amino)ethan-1-ol (compound 16)

The starting materials 2-((4-((7-chloroquinazolin-4-yl)amino)pentyl)amino)ethanol (65 mg, 0.21 mmol), 2-(chloromethyl)oxazole (30 mg, 0.26 mmol), and potassium carbonate (44 mg, 0.32 mmol) were added to acetonitrile (5 mL). The reaction mixture was stirred at 80°C overnight. After the solvent was evaporated until dryness, water was added thereto, and the mixture was extracted three times with dichloromethane. The organic phase was washed with saturated brine, dried over anhydrous sodium sulfate, filtered, and then subjected to rotary evaporation until dryness. The residue was purified by a silica gel preparative plate to obtain a yellow oil, 2-((4-((7-chloroquinazolin-4-yl)amino)pentyl)(oxazol-2-ylmethyl)amino)ethan-1-ol (39 mg, 0.10 mmol, yield of 47.52%). $^1H$ NMR (400 MHz, $CDCl_3$) $\delta$ 8.62 (s, 1H), 7.85 (d, *J* = 2.0 Hz, 1H), 7.82 (d, *J* = 8.9 Hz, 1H), 7.63 (s, 1H), 7.42 (dd, *J* = 8.8, 2.0 Hz, 1H), 7.08 (s, 1H), 6.00 (d, *J* = 6.4 Hz, 1H), 4.58 - 4.47 (m, 1H), 3.90 (s, 2H), 3.65 (t, *J* = 5.1 Hz, 2H), 2.79 (t, *J* = 4.4 Hz, 2H), 2.73-2.64 (m, 2H), 1.85-1.80 (m, 2H), 1.71-1.62 (m, 3H), 1.34 (d, *J* = 6.5 Hz, 3H). LC-MS: m/z 390 $[M+H]^+$.

Examples 17 to 43

To prepare the compounds in examples 17 to 43, the same method as described in example 16 was applied. Specific experimental data are shown in Table 4.

Table 4: Experimental data for compounds 16 to 43

| | | |
|---|---|---|
| **16** | 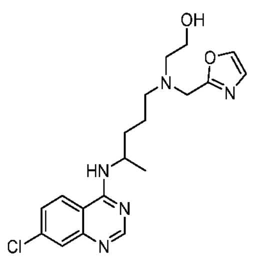 | 2-((4-((7-Chloroquinazolin-4-yl)amino)pentyl)(oxazol-2-ylmethyl)amino) ethan-1-ol<br>$^1H$ NMR (400 MHz, $CDCl_3$) $\delta$ 8.62 (s, 1H), 7.85 (d, *J* = 2.0 Hz, 1H), 7.82 (d, *J* = 8.9 Hz, 1H), 7.63 (s, 1H), 7.42 (dd, *J* = 8.8, 2.0 Hz, 1H), 7.08 (s, 1H), 6.00 (d, *J* = 6.4 Hz, 1H), 4.58 - 4.47 (m, 1H), 3.90 (s, 2H), 3.65 (t, *J* = 5.1 Hz, 2H), 2.79 (t, *J* = 4.4 Hz, 2H), 2.73-2.64 (m, 2H), 1.85-1.80 (m, 2H), 1.71-1.62 (m, 3H), 1.34 (d, *J* = 6.5 Hz, 3H). LC-MS: m/z 390 $[M+H]^+$. |

(continued)

| | | |
|---|---|---|
| **17** | 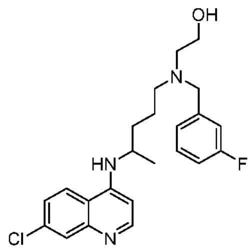 | 2-((4-((7-Chloroquinolin-4-yl)amino)pentyl)(3-fluorobenzyl)amino)ethan-1-ol<br>$^1$H NMR (400 MHz, $CDCl_3$) δ 8.49 (d, *J* = 5.5 Hz, 1H), 8.00 (d, *J* = 2.1 Hz, 1H), 7.74 (d, *J* = 9.0 Hz, 1H), 7.38 (dd, *J* = 8.9, 2.2 Hz, 1H), 7.28 - 7.23 (m, 1H), 7.09 - 7.04 (m, 1H), 7.03 (dd, *J* = 9.7, 2.2 Hz, 1H), 6.97 (td, *J* = 8.4, 1.9 Hz, 1H), 6.37 (d, *J* = 5.6 Hz, 1H), 5.09 (s, 1H), 3.74 - 3.55 (m, 5H), 2.74 - 2.61 (m, 2H), 2.54 (td, *J* = 7.0, 2.9 Hz, 2H), 1.74 - 1.59 (m, 5H), 1.32 (d, *J* = 6.4 Hz, 3H). LC-MS: m/z 416 $[M+H]^+$. |
| **18** | 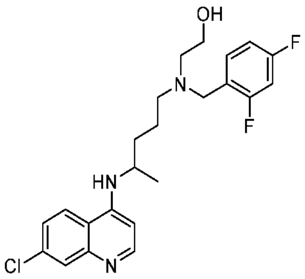 | 2-((4-((7-Chloroquinolin-4-yl)amino)pentyl)(2,4-difluorobenzyl)amino) ethan-1-ol<br>$^1$H NMR (400 MHz, $CDCl_3$) δ 8.48 (d, *J* = 5.6Hz, 1H), 8.01 (d, *J* = 2.1 Hz, 1H), 7.79 (d, *J* = 9.0 Hz, 1H), 7.39 (dd, *J* = 8.9, 2.1 Hz, 1H), 7.28 - 7.23 (m, 1H), 6.85 (dd, *J* = 7.9, 5.1 Hz, 1H), 6.80 (dd, *J* = 11.9, 3.0 Hz, 1H), 6.38 (d, *J* = 5.7 Hz, 1H), 5.27 (s, 1H), 3.72-3.60 (m, 5H), 2.74 - 2.61 (m, 2H), 2.53 (t, *J* = 5.9 Hz, 2H), 1.71-1.60 (m, 5H), 1.33 (d, *J* = 6.4 Hz, 3H). LC-MS: m/z 434 $[M+H]^+$. |
| **19** | 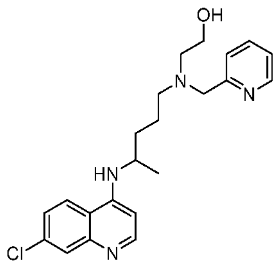 | 2-((4-((7-Chloroquinolin-4-yl)amino)pentyl)(pyridin-2-ylmethyl)amino) ethan-1 -ol<br>$^1$H NMR (400 MHz, $CDCl_3$) δ 8.56 (d, *J* = 4.8 Hz, 1H), 8.47 (d, *J* = 5.6 Hz, 1H), 8.02 (d, *J* = 2.1 Hz, 1H), 7.80 (d, *J* = 9.0 Hz, 1H), 7.63 (td, *J* = 7.7, 1.8 Hz, 1H), 7.36 (dd, *J* = 9.0, 2.1 Hz, 1H), 7.26 (d, *J* = 7.8 Hz, 1H), 7.19 (dd, *J* = 7.5, 4.9 Hz, 1H), 6.38 (d, *J* = 5.7 Hz, 1H), 5.38 (s, 1H), 3.82 (q, *J* = 14.7 Hz, 2H), 3.72 - 3.61 (m, 3H), 2.83 - 2.74 (m, 2H), 2.70-2.64 (m, 2H), 1.85 - 1.57 (m, 5H), 1.30 (d, *J* = 6.4 Hz, 3H). LC-MS: m/z 399 $[M+H]^+$. |
| **20** | 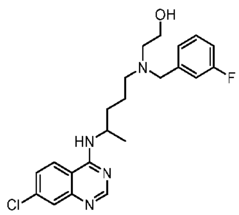 | 2-((4-((7-Chloroquinazolin-4-yl)amino)pentyl)(3-fluorobenzyl)amino) ethan-1-ol<br>$^1$H NMR (400 MHz, $CDCl_3$) δ 8.63 (s, 1H), 7.84 (d, *J* = 2.1 Hz, 1H), 7.70 (d, *J* = 8.8 Hz, 1H), 7.43 (dd, *J* = 8.8, 2.1 Hz, 1H), 7.26 (dd, *J* = 7.9, 5.9 Hz, 1H), 7.09 (d, *J* = 7.7 Hz, 1H), 7.04 (d, *J* = 9.6 Hz, 1H), 6.96 (td, *J* = 8.5, 2.1 Hz, 1H), 5.62 (d, *J* = 8.2 Hz, 1H), 4.55 - 4.46 (m, 1H), 3.64 (dd, *J* = 9.8, 4.5 Hz, 4H), 2.68 (td, *J* = 4.9, 1.8 Hz, 2H), 2.59 (td, *J* = 6.0, 1.6 Hz, 2H), 1.79-1.73 (m, 1H), 1.70 - 1.54 (m, 4H), 1.32 (d, *J* = 6.4 Hz, 3H). LC-MS: m/z 417 $[M+H]^+$. |
| **21** | 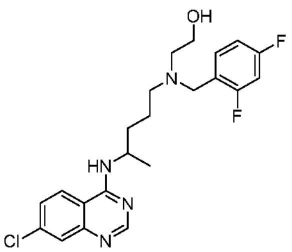 | 2-((4-((7-Chloroquinazolin-4-yl)amino)pentyl)(2,4-difluorobenzyl)amino) ethan-1-ol<br>$^1$H NMR (400 MHz, $CDCl_3$) δ 8.63 (s, 1H), 7.84 (d, *J* = 2.1 Hz, 1H), 7.77 (d, *J* = 8.3 Hz, 1H), 7.43 (dd, *J* = 8.8, 2.1 Hz, 1H), 7.36 (s, 1H), 6.88 - 6.78 (m, 2H), 5.74 (s, 1H), 4.54 - 4.46 (m, 1H), 3.79-3.62 (m, 4H), 2.71 (s, 2H), 2.61 (s, 2H), 1.80-1.60 (m, 5H), 1.32 (d, *J* = 6.4 Hz, 3H). LC-MS: m/z 435 $[M+H]^+$. |

(continued)

| | | |
|---|---|---|
| **22** | 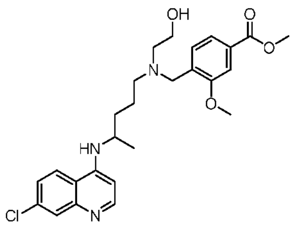 | Methyl 4-(((4-((7-chloroquinolin-4-yl)amino)pentyl)(2-hydroxyethyl) amino)methyl)-3-methoxybenzoate<br>$^1$H NMR (400 MHz, $CDCl_3$) δ 8.47 (d, *J* = 5.6 Hz, 1H), 8.00 (d, *J* = 2.0 Hz, 1H), 7.77 (d, *J* = 9.0 Hz, 1H), 7.63 (dd, *J* = 7.7, 1.4 Hz, 1H), 7.55 (d, *J* = 1.2 Hz, 1H), 7.38 (dd, *J* = 8.9, 2.1 Hz, 1H), 7.30 (d, *J* = 7.8 Hz, 1H), 6.30 (d, *J* = 5.6 Hz, 1H), 5.15 (s, 1H), 3.95 (s, 3H), 3.89 (s, 3H), 3.78 (d, *J* = 13.4 Hz, 1H), 3.74-3.69 (m, 1H), 3.67-3.63 (m, 1H), 3.59 (dd, *J* = 12.8, 6.8 Hz, 2H), 2.78-2.71 (m, 1H), 2.68 - 2.61 (m, 1H), 2.52 (dd, *J* = 10.7, 6.1 Hz, 2H), 1.67 - 1.51 (m, 5H), 1.26 (d, *J* = 6.3 Hz, 3H). LC-MS: m/z 486 $[M+H]^+$. |
| **23** | 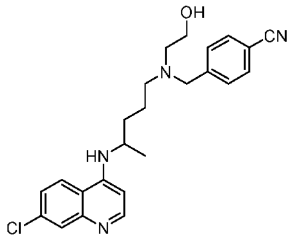 | 4-(((4-((7-Chloroquinolin-4-yl)amino)pentyl)(2-hydroxyethyl)amino) methyl)benzonitrile<br>$^1$H NMR (400 MHz, $CDCl_3$) δ 8.45 (d, *J* = 5.7 Hz, 1H), 8.00 (d, *J* = 2.1 Hz, 1H), 7.85 (d, *J* = 9.1 Hz, 1H), 7.62 (d, *J* = 8.2 Hz, 2H), 7.43 (d, *J* = 8.2 Hz, 2H), 7.37 (dd, *J* = 9.0, 2.1 Hz, 1H), 6.40 (d, *J* = 5.8 Hz, 1H), 5.52 (s, 1H), 3.74-3.63 (m, 5H), 2.73-2.63 (m, 2H), 2.55 (t, *J* = 6.5 Hz, 2H), 1.78-1.58 (m, 5H), 1.36 (d, *J* = 6.4 Hz, 3H). LC-MS: m/z 423 $[M+H]^+$. |
| **24** | 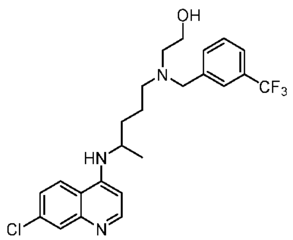 | 2-((4-((7-Chloroquinolin-4-yl)amino)pentyl)(3-(trifluoromethyl)benzyl) amino)ethan-1-ol<br>$^1$H NMR (400 MHz, $CDCl_3$) δ 8.45 (d, *J* = 5.7 Hz, 1H), 8.01 (d, *J* = 2.1 Hz, 1H), 7.80 (d, *J* = 9.1 Hz, 1H), 7.58 (s, 1H), 7.54 (d, *J* = 7.6 Hz, 1H), 7.49 (d, *J* = 7.8 Hz, 1H), 7.43 (t, *J* = 7.7 Hz, 1H), 7.38 (dd, *J* = 9.0, 2.1 Hz, 1H), 6.37 (d, *J* = 5.8 Hz, 1H), 5.35 (s, 1H), 3.75-3.64 (m, 5H), 2.76-2.64 (m, 2H), 2.54 (t, *J* = 6.0 Hz, 2H), 1.74-1.59 (m, 5H), 1.33 (d, *J* = 6.4 Hz, 3H). LC-MS: m/z 466 $[M+H]^+$. |
| **25** | 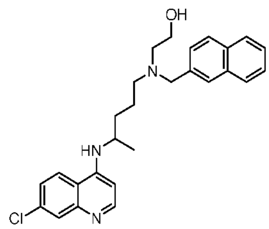 | 2-((4-((7-Chloroquinolin-4-yl)amino)pentyl)(naphthalen-2-ylmethyl) amino)ethan-1-ol<br>$^1$H NMR (400 MHz, $CDCl_3$) δ 8.36 (d, *J* = 5.6 Hz, 1H), 7.98 (d, *J* = 2.1 Hz, 1H), 7.86-7.78 (m, 3H), 7.72 (d, *J* = 8.7 Hz, 2H), 7.55 - 7.49 (m, 2H), 7.46 (dd, *J* = 8.4, 1.5 Hz, 1H), 7.34 (dd, *J* = 8.9, 2.1 Hz, 1H), 6.20 (d, *J* = 5.7 Hz, 1H), 5.15 (s, 1H), 3.85 (d, *J* = 13.4 Hz, 1H), 3.75 (d, *J* = 13.4 Hz, 1H), 3.70 - 3.65 (m, 2H), 3.61 - 3.54 (m, 1H), 2.80 - 2.68 (m, 2H), 2.59 (dd, *J* = 13.1, 6.8 Hz, 2H), 1.77 - 1.54 (m, 5H), 1.27 (d, *J* = 6.3 Hz, 3H). LC-MS: m/z 448 $[M+H]^+$. |
| **26** | 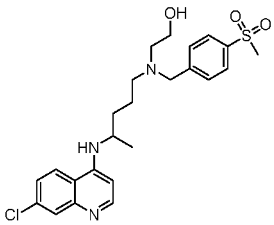 | 2-((4-((7-Chloroquinolin-4-yl)amino)pentyl)(4-(methylsulfonyl)benzyl) amino)ethan-1-ol<br>$^1$H NMR (400 MHz, $CDCl_3$) δ 8.47 (d, *J* = 5.6 Hz, 1H), 7.98 (d, *J* = 2.1 Hz, 1H), 7.90 (d, *J* = 8.3 Hz, 2H), 7.80 (d, *J* = 9.0 Hz, 1H), 7.52 (d, *J* = 8.3 Hz, 2H), 7.37 (dd, *J* = 9.0, 2.1 Hz, 1H), 6.40 (d, *J* = 5.7 Hz, 1H), 5.30 (s, 1H), 3.77 - 3.62 (m, 5H), 3.07 (s, 3H), 2.75 - 2.63 (m, 2H), 2.55 (t, *J* = 6.4 Hz, 2H), 1.83-1.51 (m,5H), 1.34 (d, *J* = 6.4Hz, 3H). LC-MS: m/z 476 $[M+H]^+$. |
| **27** | 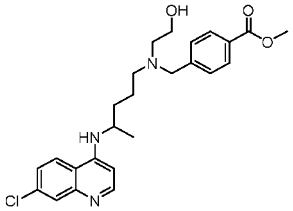 | Methyl 4-(((4-((7-chloroquinolin-4-yl)amino)pentyl)(2-hydroxyethyl) amino)methyl)benzoate<br>$^1$H NMR (400 MHz, $CDCl_3$) δ 8.47 (d, *J* = 5.6Hz, 1H), 8.03 - 7.97 (m, 3H), 7.77 (d, *J* = 8.9 Hz, 1H), 7.40-7.37 (m, 3H), 6.36 (d, *J* = 5.7 Hz, 1H), 5.23 (s, 1H), 3.94 (s, 3H), 3.74-3.63 (m, 5H), 2.74 - 2.64 (m, 2H), 2.54 (t, *J* = 5.7 Hz, 2H), 1.73-1.59 (m, 5H), 1.32 (d, *J* = 6.4 Hz, 3H). LC-MS: m/z 456 $[M+H]^+$. |

(continued)

| | | |
|---|---|---|
| **28** | 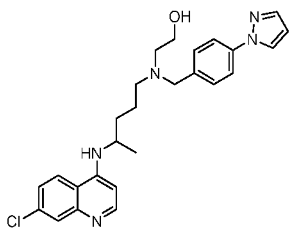 | 2-((4-(1*H*-Pyrazol-1-yl)benzyl)(4-((7-chloroquinolin-4-yl)amino)pentyl) amino)ethan-1-ol<br>$^{1}H$ NMR (400 MHz, $CDCl_3$) δ 8.41 (d, *J* = 5.9 Hz, 1H), 8.04 (d, *J* = 2.0 Hz, 1H), 7.92 (d, *J* = 2.4 Hz, 1H), 7.90 (s, 1H), 7.76 (d, *J* = 1.6 Hz, 1H), 7.65 (d, *J* = 8.5 Hz, 2H), 7.43 - 7.36 (m, 3H), 6.52 - 6.49 (m, 1H), 6.37 (d, *J* = 2.9 Hz, 1H), 5.70 (s, 1H), 3.77 - 3.65 (m, 5H), 2.80-2.71 (m, 2H), 2.61 (t, *J* = 6.6 Hz, 2H), 1.82 - 1.63 (m, 5H), 1.34 (d, *J* = 6.4 Hz, 3H). LC-MS: m/z 464 $[M+H]^+$. |
| **29** | 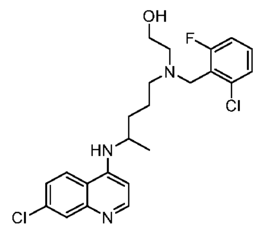 | 2-((2-Fluoro-6-chlorobenzyl)(4-((7-chloroquinolin-4-yl)amino)pentyl) amino)ethan-1-ol<br>$^{1}H$ NMR (400 MHz, $CDCl_3$) δ 8.48 (d, *J* = 5.6 Hz, 1H), 8.03 (d, *J* = 2.1 Hz, 1H), 7.77 (d, *J* = 9.0 Hz, 1H), 7.39 (dd, *J* = 8.9, 2.1 Hz, 1H), 7.23 - 7.18 (m, 2H), 7.00-6.95 (m, 1H), 6.36 (d, *J* = 5.7 Hz, 1H), 5.20 (s, 1H), 3.85-3.76 (m, 2H), 3.69 - 3.59 (m, 3H), 2.75-2.64 (m, 2H), 2.55 (t, *J* = 6.4 Hz, 2H), 1.72 - 1.54 (m, 5H), 1.30 (d, *J* = 6.4 Hz, 3H). LC-MS: m/z 450 $[M+H]^+$. |
| **30** | 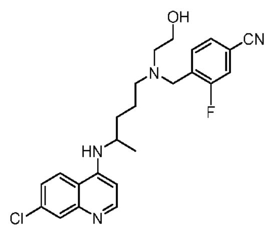 | 4-(((4-((7-Chloroquinolin-4-yl)amino)pentyl)(2-hydroxyethyl)amino) methyl)-3-fluorobenzonitrile<br>$^{1}H$ NMR (400 MHz, $CDCl_3$) δ 8.48 (d, *J* = 5.6 Hz, 1H), 7.99 (d, *J* = 2.1 Hz, 1H), 7.79 (d, *J* = 9.0 Hz, 1H), 7.50 (t, *J* = 7.4 Hz, 1H), 7.43 (dd, *J* = 7.9, 1.5 Hz, 1H), 7.39-7.33 (m, 2H), 6.40 (d, *J* = 5.7 Hz, 1H), 5.25 (s, 1H), 3.79-3.70 (m, 3H), 3.67-3.63 (m, 2H), 2.74 - 2.63 (m, 2H), 2.55 (t, *J* = 6.6 Hz, 2H), 1.81 - 1.56 (m, 5H), 1.34 (d, *J* = 6.4 Hz, 3H). LC-MS: m/z 441 $[M+H]^+$. |
| **31** | 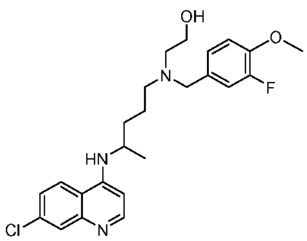 | 2-((4-((7-Chloroquinolin-4-yl)amino)pentyl)(3-fluoro-4-methoxybenzyl) amino)ethan-1-ol<br>$^{1}H$ NMR (400 MHz, $CDCl_3$) δ 8.49 (d, *J* = 5.7Hz, 1H), 8.02 (d, *J* = 2.1 Hz, 1H), 7.79 (d, *J* = 9.0 Hz, 1H), 7.39 (dd, *J* = 8.9, 2.1 Hz, 1H), 7.05 (dd, *J* = 12.0, 1.9 Hz, 1H), 6.98 (d, *J* = 8.3 Hz, 1H), 6.88 (t, *J* = 8.4 Hz, 1H), 6.38 (d, *J* = 5.7 Hz, 1H), 5.24 (s, 1H), 3.89 (s, 3H), 3.73-3.54 (m, 5H), 2.73-2.64 (m, 2H), 2.57-2.53 (m, 2H), 1.74-1.59 (m, 5H), 1.34 (d, *J* = 6.4 Hz, 3H). LC-MS: m/z 446 $[M+H]^+$. |
| **32** | 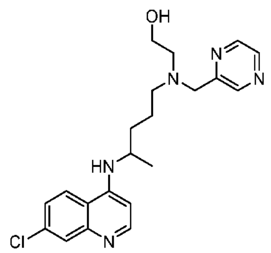 | 2-((4-((7-Chloroquinolin-4-yl)amino)pentyl)(pyrazin-2-ylmethyl)amino) ethan-1 -ol<br>$^{1}H$ NMR (400 MHz, $CDCl_3$) δ 8.61 (d, *J* = 1.3 Hz, 1H), 8.54 - 8.51 (m, 1H), 8.50 - 8.46 (m, 2H), 8.02 (d, *J* = 1.8 Hz, 1H), 7.82 (d, *J* = 9.0 Hz, 1H), 7.38 (dd, *J* = 9.0, 2.0 Hz, 1H), 6.40 (d, *J* = 5.7 Hz, 1H), 5.37 (s, 1H), 3.90-3.82 (m, 2H), 3.73 - 3.64 (m, 3H), 2.82-2.74 (m, 2H), 2.70-2.62 (m, 2H), 1.79 - 1.59 (m, 5H), 1.32 (d, *J* = 6.4 Hz, 3H). LC-MS: m/z 400 $[M+H]^+$. |
| **33** | 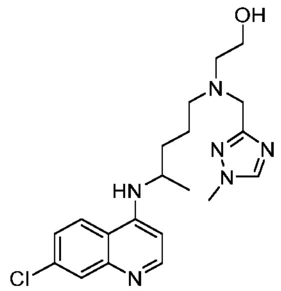 | 2-((4-((7-Chloroquinolin-4-yl)amino)pentyl)((1-methyl-1*H*-1,2,4-triazol-3-yl)methyl)amino)ethan-1-ol<br>$^{1}H$ NMR (400 MHz, $CDCl_3$) δ 8.48 (d, *J* = 5.7 Hz, 1H), 8.03 (d, *J* = 2.1 Hz, 1H), 7.96 (s, 1H), 7.87 (d, *J* = 9.1 Hz, 1H), 7.37 (dd, *J* = 8.9, 2.1 Hz, 1H), 6.44 (d, *J* = 5.8 Hz, 1H), 5.56 (s, 1H), 3.87 (s, 3H), 3.81 (d, *J* = 3.0 Hz, 2H), 3.78 - 3.72 (m, 1H), 3.63 (t, *J* = 5.2 Hz, 2H), 2.82 - 2.73 (m, 2H), 2.70-2.65 (m, 2H), 1.89-1.64 (m, 5H), 1.35 (d, *J* = 6.4 Hz, 3H). LC-MS: m/z 403 $[M+H]^+$. |

(continued)

| | | |
|---|---|---|
| **34** | 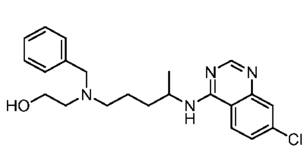 | 2-(Benzyl(4-((7-chloroquinazolin-4-yl)amino)pentyl)amino)ethan-1-ol<br>$^1$H NMR (400 MHz, $CDCl_3$) δ 8.62 (s, 1H), 7.83 (d, *J* = 2.1 Hz, 2H), 7.43 (dd, *J* = 8.8, 2.1 Hz, 1H), 7.33 (s, 5H), 5.90 (s, 1H), 4.54-4.47 (m, 1H), 3.79 (s, 2H), 3.69 (s, 2H), 2.78 (s, 2H), 2.70 (s, 2H), 1.76-1.58 (m, 5H), 1.33 (d, *J* = 6.5 Hz, 3H). LC-MS: m/z 399 $[M+H]^+$. |
| **35** | 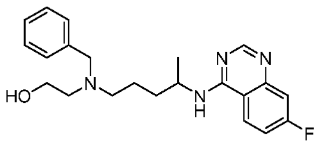 | 2-(Benzyl(4-((7-fluoroquinazolin-4-yl)amino)pentyl)amino)ethan-1-ol<br>$^1$H NMR (400 MHz, $CDCl_3$) δ 8.62 (s, 1H), 7.90 (s, 1H), 7.47 (dd, *J* = 9.9, 2.5 Hz, 1H), 7.33 (s, 5H), 7.26 - 7.21 (m, 1H), 5.78 (s, 1H), 4.55-4.48 (m, 1H), 3.85-3.62 (m, 4H), 2.85-2.62 (m, 4H), 1.80 - 1.56 (m, 5H), 1.33 (d, *J* = 6.5 Hz, 3H). LC-MS: m/z 383 $[M+H]^+$. |
| **36** | 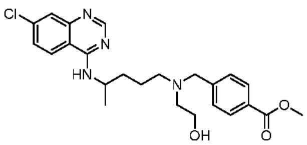 | Methyl 4-(((4-((7-chloroquinazolin-4-yl)amino)pentyl)(2-hydroxyethyl) amino)methyl)benzoate<br>$^1$H NMR (400 MHz, *d*-DMSO) δ 8.41 (s, 1H), 8.34 (d, *J* = 8.9 Hz, 1H), 8.02 - 7.94 (d, *J* = 8.0 Hz, 1H), 7.82 (d, *J* = 8.1 Hz, 2H), 7.69 (d, *J* = 2.1 Hz, 1H), 7.56 (dd, *J* = 8.8, 2.0 Hz, 1H), 7.41 (d, *J* = 8.2 Hz, 2H), 4.44 - 4.36 (m, 1H), 4.34 (t, *J* = 5.2 Hz, 1H), 3.84 (s, 3H), 3.62 (s, 2H), 3.44 (q, *J* = 5.7 Hz, 2H), 2.40-2.50 (m, 4H), 1.66 - 1.52 (m, 2H), 1.52 - 1.41 (m, 2H), 1.19 (d, *J* = 6.5 Hz, 3H). LC-MS: m/z: 457.2 $[M+H]^+$. |
| **37** | 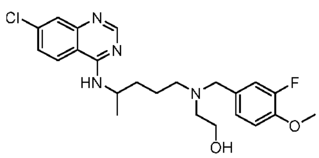 | 2-((4-((7-Chloroquinazolin-4-yl)amino)pentyl)(3-fluoro-4-methoxyphenyl)amino)ethan-1-ol<br>$^1$H NMR (400 MHz, *d*-DMSO) δ 8.42 (s, 1H), 8.36 (d, *J* = 8.9 Hz, 1H), 8.00 (d, *J* = 8.1 Hz, 1H), 7.69 (d, *J* = 2.1 Hz, 1H), 7.55 (dd, *J* = 8.8, 2.1 Hz, 1H), 7.11 (d, *J* = 12.7 Hz, 1H), 7.00 (d, *J* = 4.9 Hz, 2H), 4.35-4.46 (m, 1H), 4.31 (t, *J* = 5.3 Hz, 1H), 3.80 (s, 3H), 3.47 (s, 2H), 3.42 (q, *J* = 6.4 Hz, 2H), 2.2.38-2.46 (q, *J* = 6.7 Hz, 4H), 1.50-1.62 (m, 2H), 1.42-1.50 (m, 2H), 1.20 (d, *J* = 6.5 Hz, 3H). LC-MS: m/z: 447.2 $[M+H]^+$. |
| **38** | 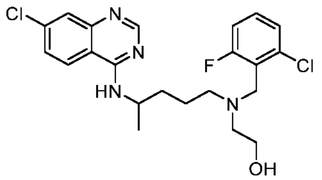 | 2-((2-Chloro-6-fluorophenyl)(4-((7-chloroquinazolin-4-yl)amino)pentyl) amino)ethan-1-ol<br>$^1$H NMR (400 MHz, *d*-DMSO) δ 8.42 (s, 1H), 8.35 (d, *J* = 8.9 Hz, 1H), 7.97 (d, *J* = 7.7 Hz, 1H), 7.69 (d, *J* = 2.1 Hz, 1H), 7.55 (dd, *J* = 8.8, 2.1 Hz, 1H), 7.35 - 7.30 (m, 1H), 7.27 (t, *J* = 7.3 Hz, 1H), 7.16 (t, *J* = 9.3 Hz, 1H), 4.41 - 4.30 (m, 1H), 4.24 (t, *J* = 5.2 Hz, 1H), 3.67 (s, 2H), 3.43 (q, *J* = 6.4 Hz, 2H), 2.45-2.50 (m, 2H), 1.52-1.60 (m, 2H), 1.42-1.50 (m, 2H), 1.17 (d, *J* = 6.5 Hz, 3H). LC-MS: m/z: 451.1 $[M+H]^+$. |
| **39** | 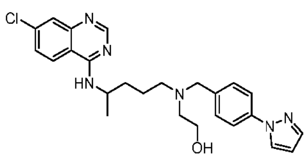 | 2-((4-(1*H*-Pyrazol-1-yl)phenyl)(4-((7-chloroquinazolin-4-yl)amino)pentyl) amino)ethan-1-ol<br>$^1$H NMR (400 MHz, *d*-DMSO) δ 8.45 - 8.43 (d, *J* = 2.4 Hz, 1H), 8.42 (s, 1H), 8.35 (d, *J* = 8.4 Hz, 1H), 8.04 - 7.96 (d, *J* = 8.0 Hz, 1H), 7.72 (d, *J* = 8.6 Hz, 3H), 7.68 (d, *J* = 2.2 Hz, 1H), 7.58 - 7.50 (m, 1H), 7.38 (d, *J* = 8.4 Hz, 2H), 6.56 - 6.50 (m, 1H), 4.49 - 4.36 (m, 1H), 4.34 - 4.28 (m, 1H), 3.58 (s, 2H), 3.50 - 3.39 (m, 2H), 2.47 (d, *J* = 7.0 Hz, 4H), 1.69 - 1.56 (m, 2H), 1.55 - 1.43 (m, 2H), 1.21 (d, *J* = 6.6 Hz, 3H). LC-MS: m/z: 465.2 $[M+H]^+$. |
| **40** | 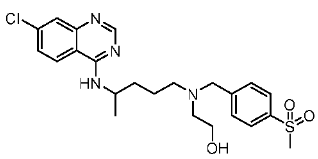 | 2-((4-((7-Chloroquinazolin-4-yl)amino)pentyl)(4-(methylsulfonyl)phenyl) amino)ethan-1-ol<br>$^1$H NMR (400 MHz, *d*-DMSO) δ 8.42 (s, 1H), 8.36 (d, *J* = 8.9 Hz, 1H), 8.06 - 7.96 (d, *J* = 8.4 Hz, 1H), 7.82 (d, *J* = 8.3 Hz, 2H), 7.69 (d, *J* = 2.0 Hz, 1H), 7.56 (d, *J* = 8.3 Hz, 3H), 4.48 - 4.37 (m, 1H), 4.35 (t, *J* = 5.2 Hz, 1H), 3.66 (s, 2H), 3.45 (d, *J* = 5.6 Hz, 2H), 3.19 (s, 3H), 2.47 (d, *J* = 3.5 Hz, 4H), 1.71 - 1.55 (m, 2H), 1.55 - 1.42 (m, 2H), 1.20 (d, *J* = 6.6 Hz, 3H). LC-MS: m/z: 477.2 $[M+H]^+$. |

(continued)

| | | |
|---|---|---|
| **41** | 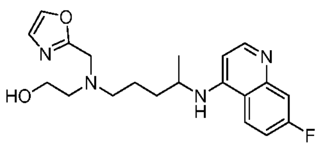 | 2-((4-((7-Fluoroquinazolin-4-yl)amino)pentyl)(oxazol-2-ylmethyl)amino) ethan-1 -ol<br>$^1$H NMR (400 MHz, *d*-DMSO) δ 8.43 (dd, *J* = 9.4, 6.2 Hz, 1H), 8.37 (s, 1H), 8.01 (d, *J* = 0.8 Hz, 1H), 7.45 (dd, *J* = 10.7, 2.7 Hz, 1H), 7.39 - 7.30 (m, 1H), 7.13 (s, 1H), 7.02 (d, *J* = 7.8 Hz, 1H), 6.50 (d, *J* = 5.8 Hz, 1H), 4.37 (t, *J* = 5.3 Hz, 1H), 3.79 (s, 2H), 3.77 - 3.68 (m, 1H), 3.46 (dd, *J* = 11.6, 6.3 Hz, 2H), 3.31 (s, 2H), 2.55 (t, *J* = 6.5 Hz, 2H), 1.76 - 1.62 (m, 1H), 1.52 (dd, *J* = 14.2, 8.2 Hz, 3H), 1.23 (d, *J* = 6.4 Hz, 3H). LC-MS: m/z: 373.2 [M+H]$^+$. |
| **42** | 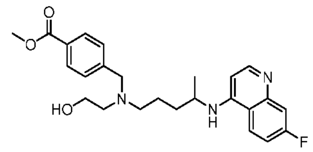 | Methyl 4-(((4-((7-fluoroquinazolin-4-yl)amino)pentyl)(2-hydroxyethyl) amino)methyl)benzoate<br>$^1$H NMR (400 MHz, *d*-DMSO) δ 8.38 (dd, *J* = 9.4, 6.3 Hz, 1H), 8.32 (d, *J* = 5.5 Hz, 1H), 7.86 (d, *J* = 8.3 Hz, 2H), 7.44 (dd, *J* = 10.8, 2.6 Hz, 3H), 7.31 (td, *J* = 9.2, 2.7 Hz, 1H), 6.86 (d, *J* = 8.0 Hz, 1H), 6.36 (d, *J* = 5.7 Hz, 1H), 4.35 (t, *J* = 5.3 Hz, 1H), 3.64 (s, 3H), 3.40-3.50 (m, 2H), 2.40-2.50 (m, 4H), 1.65-1.70 (m, 1H), 1.58 - 1.45 (m, 3H), 1.20 (d, *J* = 6.3 Hz, 3H). LC-MS: m/z: 440.2 [M+H]$^+$. |
| **43** | 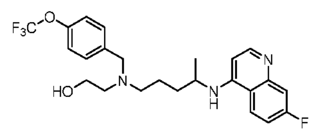 | 2-((4-((7-Fluoroquinazolin-4-yl)amino)pentyl)(4-(trifluoromethoxy) benzyl)amino)ethan-1-ol<br>$^1$H NMR (400 MHz, *d*-DMSO) δ 8.38 (dd, *J* = 9.4, 6.2 Hz, 1H), 8.33 (d, *J* = 5.5 Hz, 1H), 7.46 - 7.42 (m, 1H), 7.41 (d, *J* = 8.9 Hz, 2H), 7.31 (td, *J* = 9.3, 2.8 Hz, 1H), 7.24 (d, *J* = 7.9 Hz, 2H), 6.84 (d, *J* = 8.1 Hz, 1H), 6.37 (d, *J* = 5.6 Hz, 1H), 4.34 (t, *J* = 5.3 Hz, 1H), 3.62 (s, 1H), 3.58 (d, *J* = 5.0 Hz, 2H), 3.46 (dd, *J* = 11.9, 6.4 Hz, 2H), 2.49 - 2.39 (m, 4H), 1.66 (s, 1H), 1.54 (d, *J* = 6.3 Hz, 3H), 1.20 (d, *J* = 6.3 Hz, 3H). LC-MS: m/z: 466.2 [M+H]$^+$. |

Example 44: Methyl 4-(((5-((7-chloroquinazolin-4-yl)amino)pentyl)(2-hydroxyethyl)amino)methyl)benzoate (compound 44)

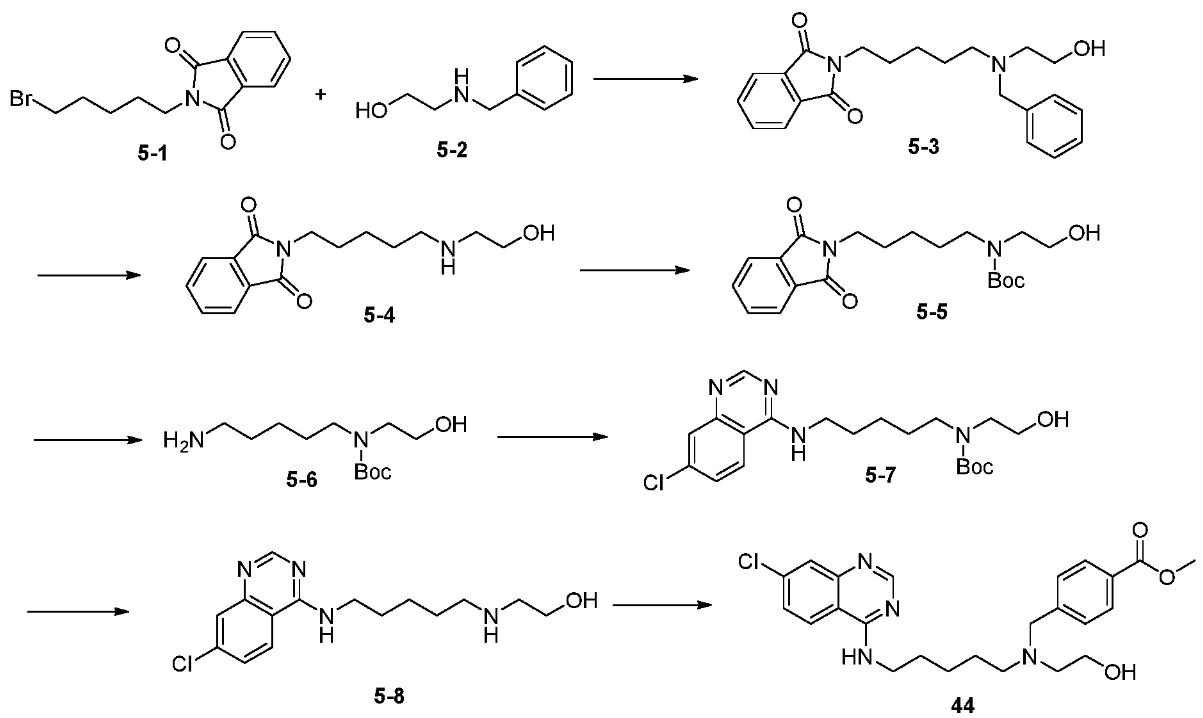

Step 1: Preparation of 2-(5-(benzyl(2-hydroxyethyl)amino)pentyl)isoindole-1,3-dione (compound 5-3)

At room temperature, potassium carbonate (2.07 g, 15 mmol) and 2-(5-bromopentyl)isoindole-1,3-dione (2.96 g, 10 mmol) were added to a solution of 2-(benzylamino)ethan-1-ol (1.51 g, 10 mmol) in acetonitrile (30 mL). The reaction mixture was heated to 80°C and stirred for 16 hours. Subsequently, the reaction mixture was cooled to room temperature, filtered, and the filtrate was concentrated under reduced pressure until dryness to obtain a pale yellow liquid, 2-(5-(benzyl(2-hydroxyethyl)amino)pentyl)isoindole-1,3-dione (3.66 g, 10 mmol, yield of 100%). LC-MS: m/z 367.2 $[M+H]^+$.

Step 2: Preparation of 2-(5-((2-hydroxyethyl)amino)pentyl)isoindole-1,3-dione (compound 5-4)

2-(5-(Benzyl(2-hydroxyethyl)amino)pentyl)isoindole-1,3-dione (3.66 g, 10 mmol) was dissolved in methanol (40 mL), then 10% Pd/C (0.9 g) was added thereto. The reaction was conducted under 1 atom of Hz pressure at 30°C for 48 hours. LCMS showed that the reaction was completed, and the reaction mixture was filtered to remove Pd/C, and the mother liquor was concentrated under reduced pressure until dryness to obtain a pale yellow liquid, 2-(5-((2-hydroxyethyl)amino)pentyl)isoindole-1,3-dione (2.76 g, 10 mmol, yield of 99%), which was used directly in the next reaction step. LC-MS: m/z 277.2 $[M+H]^+$.

Step 3: Preparation of *tert*-butyl (5-(1,3-dioxoisoindol-2-yl)pentyl)(2-hydroxyethyl)carbonate (compound 5-5)

2-(5-((2-Hydroxyethyl)amino)pentyl)isoindole-1,3-dione (2.76 g, 10 mmol) was dissolved in dichloromethane (30 mL). Di-*tert*-butyl dicarbonate (2.62 g, 12 mmol) and triethylamine (1.5 g, 15 mmol) were then added thereto. After the reaction mixture was stirred at room temperature for 16 hours, LCMS showed that the reaction was completed. The reaction mixture was concentrated under reduced pressure using a rotary evaporator, and the residue was purified by a Biotage preparative chromatography column (25 g silica gel, MeOH/DCM = 0 to 5%, v/v) to obtain *tert*-butyl (5-(1,3-dioxoisoindol-2-yl)pentyl)(2-hydroxyethyl)carbonate (3.0 g, 7.958 mmol, yield of 79.58%) as a yellow oil. LC-MS: m/z 377.2 $[M+H]^+$.

Step 4: Preparation of *tert*-butyl (5-aminopentyl)(2-hydroxyethyl)carbonate (compound 5-6)

*tert*-Butyl (5-(1,3-dioxoisoindol-2-yl)pentyl)(2-hydroxyethyl)carbonate (3.0 g, 7.958 mmol) was dissolved in methanol (20 mL), then hydrazine hydrate (10 mL, 50%) was added thereto. After the reaction mixture was stirred at 30°C for 6 hours, LCMS showed that the reaction was completed. The reaction mixture was concentrated using a rotary evaporator, and then 1 M NaOH solution was added to the residue. The mixture was extracted with dichloromethane (20 mL × 3), and the organic phases were combined, dried over anhydrous $Na_2SO_4$, and concentrated until dryness to obtain *tert*-butyl (5-aminopentyl)(2-hydroxyethyl)carbonate (1.1 g, 4.453 mmol, yield of 55.96%) as a yellow oil. LC-MS: m/z 247.3 $[M+H]^+$.

Step 5: Preparation of *tert*-butyl (5-((7-chloroquinazolin-4-yl)amino)pentyl)(2-hydroxyethyl)carbonate (compound 5-7)

At room temperature, *tert*-butyl (5-aminopentyl)(2-hydroxyethyl)carbonate (1.1 g, 4.453 mmol) and DIPEA (1.29 g, 10.00 mmol) were added to a solution of 4,7-dichloroquinazoline (0.88 g, 4.453 mmol) in isopropanol (10 mL). The reaction mixture was stirred at 80°C for 16 hours and then concentrated until dryness. The reaction mixture was added with saturated sodium bicarbonate aqueous solution (100 mL), then extracted with dichloromethane (100 mL × 2). The organic phases were combined, dried over anhydrous sodium sulfate, and concentrated until dryness. The residue was purified by a silica gel column (eluent: petroleum ether: ethyl acetate = 10:1) to obtain *tert*-butyl (5-((7-chloroquinazolin-4-yl)amino)pentyl)(2-hydroxyethyl)carbonate (1.2 g, 2.934 mmol, yield of 65.89%) as a yellow oil. LC-MS: m/z 409.2 $[M+H]^+$.

Step 6: Preparation of 2-((5-((7-chloroquinazolin-4-yl)amino)pentyl)amino)ethan-1-ol (compound 5-8)

At room temperature, a solution of hydrochloric acid in 1,4-dioxane (10 mL) was added to a solution of *tert*-butyl (5-((7-chloroquinazolin-4-yl)amino)pentyl)(2-hydroxyethyl)carbonate (1.2 g, 2.94 mmol) in methanol (5 mL). The reaction mixture was stirred at room temperature for 1.5 hours and then concentrated until dryness to obtain 2-((5-((7-chloroquinazolin-4-yl)amino)pentyl)amino)ethan-1-ol (0.905 g, 2.94 mmol, yield of 100%) as a yellow solid. LC-MS: m/z 309.2 $[M+H]^+$.

Step 7: Preparation of methyl 4-(((5-((7-chloroquinazolin-4-yl)amino)pentyl)(2-hydroxyethyl)amino)methyl)benzoate (compound 44)

2-((5-((7-Chloroquinazolin-4-yl)amino)pentyl)amino)ethan-1-ol (0.08 g, 0.2591 mmol) and methyl 4-(bromomethyl)benzoate (0.060 g, 0.2619 mmol) were dissolved in acetonitrile (3 mL), and potassium carbonate (0.04 g, 0.2899 mmol) was added thereto. The reaction mixture was stirred at 80°C for 16 hours. The reaction mixture was cooled to room temperature, concentrated until dryness, and purified by a preparative thin-layer silica gel plate (eluent: dichloromethane: methanol = 10:1) to obtain a yellow oily product, methyl 4-(((5-((7-chloroquinazolin-4-yl)amino)pentyl)(2-hydroxyethyl)amino)methyl)benzoate (23 mg, 0.05033 mmol, yield of 19.43%). $^1$H NMR (400 MHz, *d*-DMSO) δ 8.45 (s, 1H), 8.32-8.39 (t, *J* = 6.0 Hz, 1H), 8.27 (d, *J* = 8.9 Hz, 1H), 7.85 (d, *J* = 8.2 Hz, 2H), 7.70 (d, *J* = 2.2 Hz, 1H), 7.55 (dd, *J* = 8.8, 2.2 Hz, 1H), 7.43 (d, *J* = 8.1 Hz, 2H), 4.33 (t, *J* = 5.2 Hz, 1H), 3.84 (s, 3H), 3.63 (s, 2H), 3.51 (q, *J* = 6.8 Hz, 2H), 3.45 (q, *J* =6.4 Hz, 2H), 2.44 (m, 4H), 1.67 - 1.54 (m, 2H), 1.53 - 1.40 (m, 2H), 1.38 - 1.28 (m, 2H). LC-MS: m/z 457.2 [M+H]$^+$.

Examples 45 to 49

To prepare the compounds in examples 45 to 49, the same method as described in example 44 was applied. Specific experimental data are shown in Table 5.

Table 5: Experimental data for compounds 44 to 49

| | | |
|---|---|---|
| **44** | 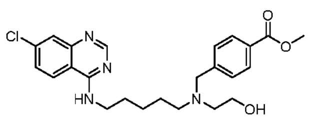 | Methyl 4-(((5-((7-chloroquinazolin-4-yl)amino)pentyl)(2-hydroxyethyl) amino)methyl)benzoate<br>$^1$H NMR (400 MHz, *d*-DMSO) δ 8.45 (s, 1H), 8.32-8.39 (t, *J* = 6.0 Hz, 1H), 8.27 (d, *J* = 8.9 Hz, 1H), 7.85 (d, *J* = 8.2 Hz, 2H), 7.70 (d, *J* = 2.2 Hz, 1H), 7.55 (dd, *J* = 8.8, 2.2 Hz, 1H), 7.43 (d, *J* = 8.1 Hz, 2H), 4.33 (t, *J* = 5.2 Hz, 1H), 3.84 (s, 3H), 3.63 (s, 2H), 3.51 (q, *J* = 6.8 Hz, 2H), 3.45 (q, *J* =6.4 Hz, 2H), 2.44 (m, 4H), 1.67 - 1.54 (m, 2H), 1.53 - 1.40 (m, 2H), 1.38 - 1.28 (m, 2H). LC-MS: m/z: 457.2 [M+H]$^+$. |
| **45** | 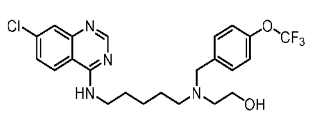 | 2-((5-((7-Chloroquinazolin-4-yl)amino)pentyl)(4-(trifluoromethoxy) benzyl)amino)ethan-1-ol<br>$^1$H NMR (400 MHz, *d*-DMSO) δ 8.45 (s, 1H), 8.40 - 8.33 (t, *J* = 6.0 Hz, 1H), 8.27 (d, *J* = 8.9 Hz, 1H), 7.70 (d, *J* = 2.1 Hz, 1H), 7.59 - 7.52 (dd, *J* = 8.8, 2.0 Hz, 1H), 7.40 (d, *J* = 8.5 Hz, 2H), 7.23 (d, *J* = 8.3 Hz, 2H), 4.32 (t, *J* = 5.2 Hz, 1H), 3.58 (s, 2H), 3.55 - 3.48 (q, *J* = 5.8 Hz, 2H), 3.45 (q, *J* = 5.8 Hz, 2H), 2.49 - 2.39 (m, 4H), 1.67 - 1.55 (m, 2H), 1.53 - 1.40 (m, 2H), 1.38 - 1.28 (m, 2H). LC-MS: m/z: 483.2 [M+H]$^+$. |
| **46** | 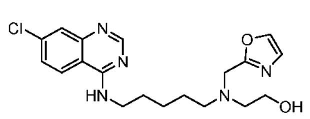 | 2-((5-((7-Chloroquinazolin-4-yl)amino)pentyl)(oxazol-2-ylmethyl)amino) ethan-1 -ol<br>$^1$H NMR (400 MHz, *d*-DMSO) δ 8.46 (s, 1H), 8.38 (s, 1H), 8.35-8.40 (t, *J* = 5.2 Hz, 1H), 8.03 (d, *J* = 0.8 Hz, 1H), 7.70 (d, *J* = 2.2 Hz, 1H), 7.56 (dd, *J* = 8.8, 2.2 Hz, 1H), 7.14 (d, *J* = 0.8 Hz, 1H), 4.36 (t, *J* = 5.4 Hz, 1H), 3.78 (s, 2H), 3.51 (t, 7.0 Hz, 2H), 3.45 (t, 6.5 Hz, 2H), 2.55 (t, *J* = 6.5 Hz, 2H), 2.49 - 2.42 (m, 2H), 1.68 - 1.57 (m, 2H), 1.53 - 1.39 (m, 2H), 1.38 - 1.27 (m, 2H). LC-MS: m/z: 390.2 [M+H]$^+$. |
| **47** | 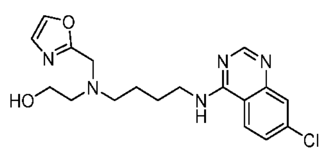 | 2-((4-((7-Chloroquinazolin-4-yl)amino)butyl)(oxazol-2-ylmethyl)amino) ethan-1 -ol<br>$^1$H NMR (400 MHz, *d*-DMSO) δ 8.46 (s, 1H), 8.38 (s, 1H), 8.29 (d, *J* = 8.9 Hz, 1H), 8.03 (s, 1H), 7.71 (d, *J* = 2.2 Hz, 1H), 7.56 (dd, *J* = 8.8, 2.2 Hz, 1H), 7.13 (s, 1H), 4.37 (t, *J* = 5.4 Hz, 1H), 3.80 (s, 2H), 3.52 (dd, *J* = 11.9, *J* = 5.8 Hz, 2H), 3.46 (dd, *J* = 11.9, 6.4 Hz, 2H), 2.55 (dd, *J* = 11.4, 4.9 Hz, 3H), 1.62 (m, 2H), 1.51 (m, 2H). LC-MS: m/z: 376.2 [M+H]$^+$. |

(continued)

| | | |
|---|---|---|
| **48** | 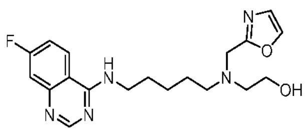 | 2-((5-((7-Fluoroquinazolin-4-yl)amino)pentyl)(oxazol-2-ylmethyl)amino) ethan-1 -ol<br>$^1$H NMR (400 MHz, $CDCl_3$) δ 8.64 (s, 1H), 7.88 (dd, *J* = 9.1, 2.0 Hz, 1H), 7.63 (s, 1H), 7.48 (dd, *J* = 9.8, 2.5 Hz, 1H), 7.23 (td, *J* = 8.7, 2.6 Hz, 1H), 7.06 (s, 1H), 6.16 (s, 1H), 3.90 (s, 2H), 3.71-3.64 (m, 4H), 2.80 (t, *J* = 5.2 Hz, 2H), 2.65 (t, *J* = 6.8 Hz, 2H), 1.80-1.73 (m, 2H), 1.66-1.59 (m, 2H), 1.55-1.49 (m, 2H). LC-MS: m/z 374.2 $[M+H]^+$. |
| **49** | 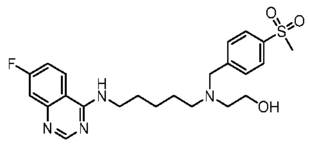 | 2-((5-((7-Fluoroquinazolin-4-yl)amino)pentyl)(4-(methylsulfonyl)phenyl) amino)ethan-1-ol<br>$^1$H NMR (400 MHz, $CDCl_3$) δ 8.63 (s, 1H), 7.91 (d, *J* = 8.2 Hz, 2H), 7.82 (dd, *J* = 9.1, 1.6 Hz, 1H), 7.55 (d, *J* = 8.2 Hz, 2H), 7.47 (dd, *J* = 9.8, 2.0 Hz, 1H), 7.23 (td, *J* = 8.8, 2.4 Hz, 1H), 5.95 (s, 1H), 3.75 (s, 2H), 3.68-3.62 (m, 4H), 3.09 (s, 3H), 2.72 (t, *J* = 5.3 Hz, 2H), 2.55 (t, *J* = 7.1 Hz, 2H), 1.70 - 1.58 (m, 4H), 1.47-1.42 (m, 2H). LC-MS: m/z 461.2 $[M+H]^+$. |

Example 50: 3-(5-((7-Fluoroquinazolin-4-yl)amino)pentyl)imidazolidine-2,4-dione (compound 50)

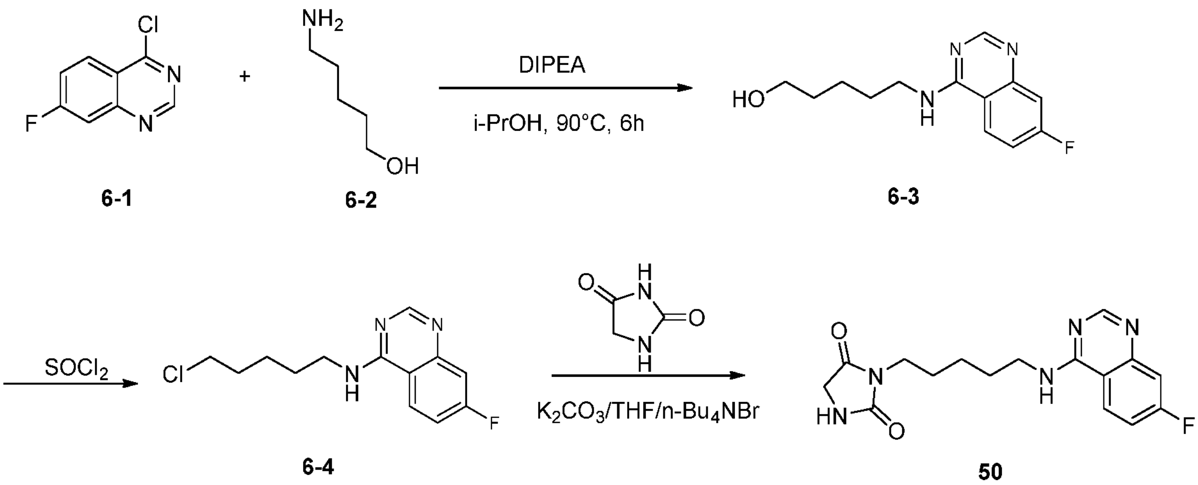


6-1 6-2 6-3 6-4 50

Step 1: Preparation of 5-((7-fluoroquinazolin-4-yl)amino)pentan-1-ol (compound 6-3)

The raw materials 4-chloro-7-fluoroquinazoline (0.9 g, 5 mmol) and 5-aminopentan-1-ol (0.5 g, 5 mmol) were dissolved in 10 mL of isopropanol. *N,N*-Diisopropylethylamine (0.7 g, 5 mmol) was added to the reaction mixture, and the reaction mixture was heated to 90°C under an argon atmosphere and reacted for 6 hours. After the reaction was completed, the reaction mixture was concentrated. The residue was separated by column chromatography to obtain the product (622 mg, yield of 50%). LC-MS: m/z 250.1 $[M+H]^+$.

Step 2: Preparation of *N*-(5-chloropentyl)-7-fluoroquinazolin-4-amine (compound 6-4)

The raw material 5-((7-fluoroquinazolin-4-yl)amino)pentan-1-ol (0.6 g, 2.4 mmol) was dissolved in 20 mL of anhydrous dichloromethane. Then, sulfonyl chloride (1.2 g, 10 mmol) and a catalytic amount of *N*,*N*-dimethylformamide (0.3 mL) were added thereto. The reaction mixture was reacted at room temperature under an argon atmosphere for 4 hours. After the reaction was completed, the reaction mixture was concentrated. The residue was adjusted to a pH of 8 with an aqueous solution of sodium bicarbonate and extracted with dichloromethane. The organic phase was dried, filtered, and concentrated. The residue was then separated by column chromatography to obtain the product (493 mg, yield of 76.5%). LC-MS: m/z 268.0 $[M+H]^+$.

Step 3: Preparation of 3-(5-((7-fluoroquinazolin-4-yl)amino)pentyl)imidazolidine-2,4-dione (compound 50)

The raw material *N*-(5-chloropentyl)-7-fluoroquinazolin-4-amine (80 mg, 0.3 mmol) was dissolved in 5 mL of dry tetrahydrofuran. Imidazolidine-2,4-dione (60 mg, 0.6 mmol), potassium carbonate (130 mg, 0.9 mmol), and tetrabutylammonium hydrogen sulfate (20 mg, 0.06 mmol) were added thereto. The reaction mixture was heated to 60°C under an argon atmosphere and reacted overnight. After the reaction was completed, the reaction mixture was concentrated. The residue was separated by a preparative silica gel plate to obtain the product (42 mg, yield of 42.4%). $^1$H NMR (400 MHz, MeOD) δ 8.45 (s, 1H), 8.23 (dd, *J* = 9.9, 5.8 Hz, 1H), 7.37 - 7.32 (m, 2H), 3.93 (s, 2H), 3.64 (t, *J* = 7.1 Hz, 2H), 3.52 (t, *J* = 7.1 Hz, 2H), 1.83 - 1.75 (m, 2H), 1.74 - 1.66 (m, 2H), 1.50 - 1.41 (m, 2H). LC-MS: m/z 332.0 [M+H]$^+$.

Examples 51 to 77

To prepare the compounds in examples 51 to 77, the same method as described in example 50 was applied. Specific experimental data are shown in Table 6.

Table 6: Experimental data for compounds 50 to 77

| | | |
|---|---|---|
| **50** | 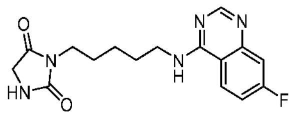 | 3-(5-((7-Fluoroquinazolin-4-yl)amino)pentyl)imidazolidine-2,4-dione<br>$^1$H NMR (400 MHz, MeOD) δ 8.45 (s, 1H), 8.23 (dd, *J* = 9.9, 5.8 Hz, 1H), 7.37 - 7.32 (m, 2H), 3.93 (s, 2H), 3.64 (t, *J* = 7.1 Hz, 2H), 3.52 (t, *J* = 7.1 Hz, 2H), 1.83 - 1.75 (m, 2H), 1.74 - 1.66 (m, 2H), 1.50 - 1.41 (m, 2H). LC-MS: m/z 332.0 [M+H]$^+$. |
| **51** | 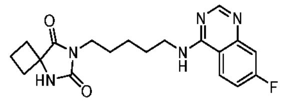 | 7-(5-((7-Fluoroquinazolin-4-yl)amino)pentyl)-5,7-diazaspiro[3.4]octane-6,8-dione<br>$^1$H NMR (400 MHz, MeOD) δ 8.45 (s, 1H), 8.23 (dd, *J* = 9.8, 5.8 Hz, 1H), 7.37 - 7.31 (m, 2H), 3.64 (t, *J* = 7.0 Hz, 2H), 3.48 (t, *J* = 7.0 Hz, 2H), 2.42 - 2.30 (m, 4H), 2.09 - 2.02 (m, 1H), 1.85 - 1.74 (m, 3H), 1.72- 1.64 (m, 2H), 1.46 - 1.38 (m, 2H). LC-MS: m/z 372.0 [M+H]$^+$. |
| **52** | 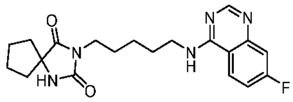 | 3-(5-((7-Fluoroquinazolin-4-yl)amino)pentyl)-1,3-diazaspiro[4.4]nonane-2,4-dione<br>$^1$H NMR (400 MHz, MeOD) δ 8.44 (s, 1H), 8.22 (dd, J = 9.8, 5.8 Hz, 1H), 7.36 - 7.31 (m, 2H), 3.64 (t, J = 7.0 Hz, 2H), 3.51 (t, J = 6.9 Hz, 2H), 2.04 - 1.96 (m, 2H), 1.83 - 1.76 (m, 6H), 1.74 -1.66 (m, 4H), 1.46 - 1.38 (m, 2H). LC-MS: m/z 386.2 [M+H]$^+$. |
| **53** | 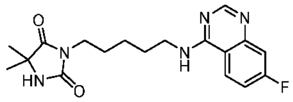 | 3-(5-((7-Fluoroquinazolin-4-yl)amino)pentyl)-5,5-dimethylimidazoline-2,4-dione<br>$^1$H NMR (400 MHz, MeOD) δ 8.44 (s, 1H), 8.22 (dd, J = 9.8, 5.8 Hz, 1H), 7.37 - 7.31 (m, 2H), 3.64 (t, J = 7.0 Hz, 2H), 3.50 (t, J = 7.0 Hz, 2H), 1.83 - 1.74 (m, 2H), 1.73 - 1.65 (m, 2H), 1.46 - 1.39 (m, 2H), 1.33 (s, 6H). LC-MS: m/z 360.2 [M+H]$^+$. |
| **54** | 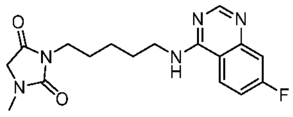 | 3-(5-((7-Fluoroquinazolin-4-yl)amino)pentyl)-1-methylimidazoline-2,4-dione<br>$^1$H NMR (400 MHz, MeOD) δ 8.44 (s, 1H), 8.23 (dd, *J* = 9.8, 5.8 Hz, 1H), 7.37 - 7.31 (m, 2H), 3.93 (s, 2H), 3.64 (t, *J* = 7.1 Hz, 2H), 3.51 (t, *J* = 7.0 Hz, 2H), 2.95 (s, 3H), 1.82 - 1.74 (m, 2H), 1.73 - 1.65 (m, 2H), 1.49 - 1.41 (m, 2H). LC-MS: m/z 346.3 [M+H]$^+$. |
| **55** | 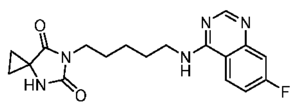 | 6-(5-((7-Fluoroquinazolin-4-yl)amino)pentyl)-4,6-diazaspiro[2.4]heptane-5,7-dione<br>$^1$H NMR (400 MHz, MeOD) δ 8.45 (s, 1H), 8.24 (dd, *J* = 9.9, 5.8 Hz, 1H), 7.37 - 7.32 (m, 2H), 3.65 (t, *J* = 7.0 Hz, 2H), 3.57 (t, *J* = 7.0 Hz, 2H), 1.83 - 1.78 (m, 2H), 1.76 - 1.69 (m, 2H), 1.50 - 1.44 (m, 2H), 1.35 - 1.30 (m, 4H). LC-MS: m/z 358.2 [M+H]$^+$. |

(continued)

| | | |
|---|---|---|
| **56** | 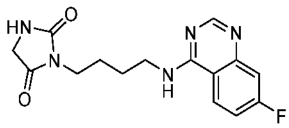 | 3-(4-((7-Fluoroquinazolin-4-yl)amino)butyl)imidazoline-2,4-dione<br>$^1$H NMR (400 MHz, MeOD) δ 8.48 (s, 1H), 8.24 (dd, *J* = 9.9, 5.8 Hz, 1H), 7.40 - 7.34 (m, 2H), 3.95 (s, 2H), 3.70 (t, *J* = 6.6 Hz, 2H), 3.57 (t, *J* = 6.7 Hz, 2H), 1.80 - 1.72 (m, 4H). LC-MS: m/z 318.0 $[M+H]^+$. |
| **57** | 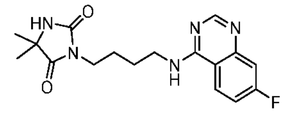 | 3-(4-((7-Fluoroquinazolin-4-yl)amino)butyl)-5,5-dimethylimidazoline-2,4-dione<br>$^1$H NMR (400 MHz, MeOD) δ 8.44 (s, 1H), 8.23 - 8.18 (m, 1H), 7.34 (d, *J* = 9.2 Hz, 1H), 7.32 (dd, *J* = 7.6, 2.4 Hz, 1H), 3.66 (t, *J* = 6.2 Hz, 2H), 3.55 (t, *J* = 6.2 Hz, 2H), 1.77 - 1.71 (m, 4H), 1.37 (s, 6H). LC-MS: m/z 346.2 $[M+H]^+$. |
| **58** | 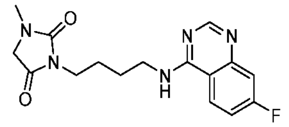 | 3-(4-((7-Fluoroquinazolin-4-yl)amino)butyl)-1-methylimidazoline-2,4-dione<br>$^1$H NMR (400 MHz, MeOD) δ 8.45 (s, 1H), 8.21 (dd, *J* = 9.7, 5.9 Hz, 1H), 7.37 - 7.31 (m, 2H), 3.94 (s, 2H), 3.67 (t, *J* = 6.5 Hz, 2H), 3.56 (t, *J* = 6.6 Hz, 2H), 2.95 (s, 3H), 1.78 - 1.71 (m, 4H). LC-MS: m/z 332.2 $[M+H]^+$. |
| **59** | 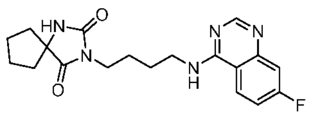 | 3-(4-((7-Fluoroquinazolin-4-yl)amino)butyl)-1,3-diazaspiro[4.4]nonane-2,4-dione<br>$^1$H NMR (400 MHz, MeOD) δ 8.44 (s, 1H), 8.23 - 8.19 (m, 1H), 7.36 - 7.31 (m, 2H), 3.66 (t, *J* = 6.0 Hz, 2H), 3.56 (t, *J* = 5.8 Hz, 2H), 2.09 - 2.01 (m, 2H), 1.87 - 1.73 (m, 10H). LC-MS: m/z 372.2 $[M+H]^+$. |
| **60** | 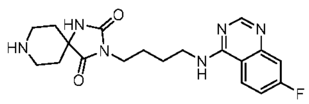 | 3-(4-((7-Fluoroquinazolin-4-yl)amino)butyl)-1,3,8-triazaspiro[4.5]decane-2,4-dione<br>$^1$H NMR (400 MHz, MeOD) δ 8.78 (s, 1H), 8.48 (dd, *J* = 9.2, 5.3 Hz, 1H), 7.65 - 7.60 (m, 1H), 7.51 (dd, *J* = 8.7, 2.5 Hz, 1H), 3.90 (t, *J* = 6.8 Hz, 2H), 3.63 - 3.56 (m, 4H), 3.32 - 3.24 (m, 2H), 2.28 - 2.19 (m, 2H), 2.03 - 1.96 (m, 2H), 1.86 - 1.72 (m, 4H). LC-MS: m/z 387.2 $[M+H]^+$. |
| **61** | 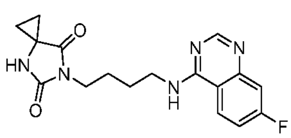 | 6-(4-((7-Fluoroquinazolin-4-yl)amino)butyl)-4,6-diazaspiro[2.4]heptane-5,7-dione<br>$^1$H NMR (400 MHz, MeOD) δ 8.45 (s, 1H), 8.24 - 8.19 (m, 1H), 7.38 - 7.31 (m, 2H), 3.68 (t, *J* = 6.4 Hz, 2H), 3.62 (t, *J* = 6.6 Hz, 2H), 1.83 - 1.73 (m, 4H), 1.39 - 1.35 (m, 2H), 1.32 - 1.29 (m, 2H). LC-MS: m/z 344.2 $[M+H]^+$. |
| **62** | 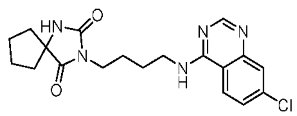 | 3-(4-((7-Chloroquinazolin-4-yl)amino)butyl)-1,3-diazaspiro[4.4]nonane-2,4-dione<br>1H NMR (400 MHz, MeOD) δ 8.45 (s, 1H), 8.12 (d, J = 8.8 Hz, 1H), 7.70 (d, J = 2.0 Hz, 1H), 7.51 (dd, J = 8.9, 2.2 Hz, 1H), 3.66 (t, *J* = 5.9 Hz, 2H), 3.56 (t, *J* = 5.9 Hz, 2H), 2.07 - 2.02 (m, 2H), 1.87 - 1.81 (m, 6H), 1.76 - 1.72 (m, 4H). LC-MS: m/z 388.6 $[M+H]^+$. |
| **63** | 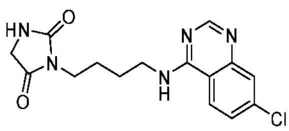 | 3-(4-((7-Chloroquinazolin-4-yl)amino)butyl)imidazoline-2,4-dione<br>1H NMR (400 MHz, DMSO) δ 8.47 (s, 1H), 8.40 (t, J = 5.3 Hz, 1H), 8.28 (d, *J* = 8.9 Hz, 1H), 8.00 (s, 1H), 7.71 (d, *J* = 2.1 Hz, 1H), 7.56 (dd, *J* = 8.8, 2.1 Hz, 1H), 3.89 (s, 2H), 3.56 - 3.52 (m, 2H), 3.41 - 3.38 (m, 2H), 1.65 - 1.56 (m, 4H). LC-MS: m/z 334.6 $[M+H]^+$. |
| 64 | 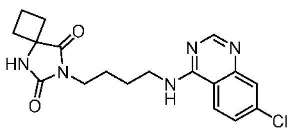 | 7-(4-((7-Chloroquinazolin-4-yl)amino)butyl)-5,7-diazaspiro[3.4]octane-6,8-dione<br>$^1$H NMR (400 MHz, MeOD) δ 8.42 (s, 1H), 8.09 (d, *J* = 8.8 Hz, 1H), 7.66 (s, 1H), 7.48 (d, J = 8.8 Hz, 1H), 3.63 (s, 2H), 3.50 (s, 2H), 2.49 - 2.30 (m, 4H), 2.12 - 1.99 (m, 1H), 1.83 (s, 1H), 1.70 (s, 4H). LC-MS: m/z 374.6 $[M+H]^+$. |

(continued)

| | | |
|---|---|---|
| 65 | 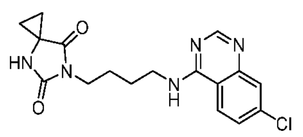 | 6-(4-((7-Chloroquinazolin-4-yl)amino)butyl)-4,6-diazaspiro[2.4]heptane-5,7-dione<br>$^1$H NMR (400 MHz, MeOD) δ 8.47 (s, 1H), 8.14 (d, *J* = 8.9 Hz, 1H), 7.71 (d, *J* = 2.0 Hz, 1H), 7.53 (dd, *J* = 8.9, 2.1 Hz, 1H), 3.68 (t, *J* = 6.3 Hz, 2H), 3.62 (t, *J* = 5.7 Hz, 2H), 1.80 - 1.74 (m, 4H), 1.39 - 1.35 (m, 2H), 1.32 - 1.27 (m, 2H). LC-MS: m/z 360.6 $[M+H]^+$. |
| 66 | 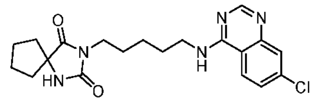 | 3 -(5-((7-Chloroquinazolin-4-yl)amino)pentyl)-1,3-diazaspiro[4.4]nonane-2,4-dione<br>1H NMR (400 MHz, MeOD) δ 8.45 (s, 1H), 8.14 (d, J = 8.9 Hz, 1H), 7.70 (d, J = 2.1 Hz, 1H), 7.52 (dd, J = 8.9, 2.1 Hz, 1H), 3.65 (t, *J* = 7.0 Hz, 2H), 3.51 (t, *J* = 6.9 Hz, 2H), 2.03 - 1.95 (m, 2H), 1.84 - 1.78 (m, 6H), 1.74 - 1.66 (m, 4H), 1.45 - 1.38 (m, 2H). LC-MS: m/z 402.6 $[M+H]^+$. |
| 67 | 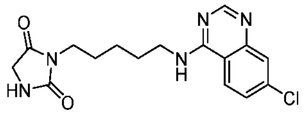 | 3-(5-((7-Chloroquinazolin-4-yl)amino)pentyl)imidazoline-2,4-dione<br>$^1$H NMR (400 MHz, DMSO) δ 8.46 (s, 1H), 8.39 (t, *J* = 5.0 Hz, 1H), 8.29 (d, *J* = 8.9 Hz, 1H), 8.00 (s, 1H), 7.71 (d, *J* = 2.1 Hz, 1H), 7.56 (dd, *J* = 8.8, 2.2 Hz, 1H), 3.89 (s, 2H), 3.53 - 3.48 (m, 2H), 3.37 - 3.33 (m, 2H), 1.70 - 1.62 (m, 2H), 1.59 - 1.52 (m, 2H), 1.37 - 1.29 (m, 2H). LC-MS: m/z 348.6 $[M+H]^+$. |
| 68 | 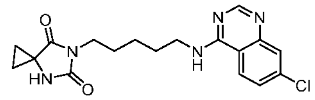 | 6-(5-((7-Chloroquinazolin-4-yl)amino)pentyl)-4,6-diazaspiro[2.4]heptane-5,7-dione<br><br>$^1$H NMR (400 MHz, MeOD) δ 8.46 (s, 1H), 8.15 (d, *J* = 8.9 Hz, 1H), 7.70 (d, *J* = 2.1 Hz, 1H), 7.53 (dd, *J* = 8.9, 2.1 Hz, 1H), 3.65 (t, *J* = 7.0 Hz, 2H), 3.57 (t, *J* = 7.0 Hz, 2H), 1.83 - 1.76 (m, 2H), 1.76 - 1.68 (m, 2H), 1.49 - 1.43 (m, 2H), 1.36 - 1.33 (m, 2H), 1.30 - 1.26 (m, 2H). LC-MS: m/z 374.6 $[M+H]^+$. |
| 69 | 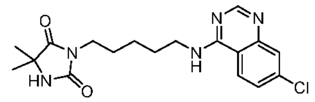 | 3-(5-((7-Chloroquinazolin-4-yl)amino)pentyl)-5,5-dimethylimidazoline-2,4-dione<br>$^1$H NMR (400 MHz, MeOD) δ 8.45 (s, 1H), 8.14 (d, *J* = 8.9 Hz, 1H), 7.69 (d, *J* = 2.1 Hz, 1H), 7.52 (dd, *J* = 8.9, 2.1 Hz, 1H), 3.64 (t, *J* = 7.0 Hz, 2H), 3.50 (t, *J* = 7.0 Hz, 2H), 1.82 - 1.75 (m, 2H), 1.72 - 1.66 (m, 2H), 1.46 - 1.39 (m, 2H), 1.33 (s, 6H). LC-MS: m/z 376.6 $[M+H]^+$. |
| 70 | 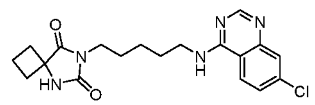 | 7-(5-((7-Chloroquinazolin-4-yl)amino)pentyl)-5,7-diazaspiro[3.4]octane-6,8-dione<br>$^1$H NMR (400 MHz, MeOD) δ 8.45 (s, 1H), 8.14 (d, *J* = 8.8 Hz, 1H), 7.70 (d, *J* = 2.1 Hz, 1H), 7.52 (dd, *J* = 8.9, 2.1 Hz, 1H), 3.64 (t, *J* = 7.0 Hz, 2H), 3.48 (t, *J* = 7.0 Hz, 2H), 2.45 - 2.30 (m, 4H), 2.08 - 2.00 (m, 1H), 1.88 - 1.81 (m, 1H), 1.80 - 1.74 (m, 2H), 1.72 - 1.64 (m, 2H), 1.45 - 1.38 (m, 2H). LC-MS: m/z 388.6 $[M+H]^+$. |
| 71 | 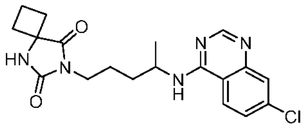 | 7-(4-((7-Chloroquinazolin-4-yl)amino)pentyl)-5,7-diazaspiro[3.4]octane-6,8-dione<br>$^1$H NMR (400 MHz, MeOD) δ 8.45 (s, 1H), 8.21 (d, *J* = 8.9 Hz, 1H), 7.69 (d, *J* = 2.1 Hz, 1H), 7.51 (dd, *J* = 8.9, 2.1 Hz, 1H), 4.62 - 4.56 (m, 1H), 3.51 (t, *J* = 6.6 Hz, 2H), 2.48 - 2.33 (m, 4H), 2.12 - 2.03 (m, 1H), 1.89 - 1.81 (m, 1H), 1.75 - 1.63 (m, 4H), 1.32 (d, *J* = 6.6 Hz, 3H). LC-MS: m/z 388.0 $[M+H]^+$. |
| 72 | 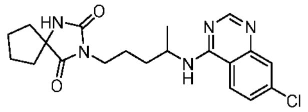 | 3 -(4-((7-Chloroquinazolin-4-yl)amino)pentyl)-1,3-diazaspiro[4.4]nonane-2,4-dione $^1$H NMR (400 MHz, MeOD) δ 8.45 (s, 1H), 8.21 (d, *J* = 8.8 Hz, 1H), 7.69 (d, *J* = 2.1 Hz, 1H), 7.51 (dd, *J* = 8.9, 2.1 Hz, 1H), 4.60 - 4.56 (m, 1H), 3.53 (t, *J* = 6.3 Hz, 2H), 2.08 - 2.02 (m, 2H), 1.86 - 1.64 (m, 10H), 1.32 (d, *J* = 6.6 Hz, 3H). LC-MS: m/z 402.0 $[M+H]^+$. |

(continued)

| | | |
|---|---|---|
| **73** | 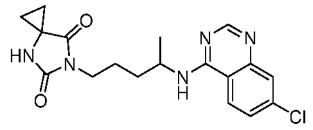 | 6-(4-((7-Chloroquinazolin-4-yl)amino)pentyl)-4,6-diazaspiro[2.4]heptane-5,7-dione<br>$^1$H NMR (400 MHz, MeOD) δ 8.45 (s, 1H), 8.21 (d, *J* = 8.9 Hz, 1H), 7.69 (d, *J* = 2.2 Hz, 1H), 7.51 (dd, *J* = 8.9, 2.1 Hz, 1H), 4.62 - 4.56 (m, 1H), 3.59 (t, *J* = 6.4 Hz, 2H), 1.78 - 1.67 (m, 4H), 1.39 - 1.34 (m, 2H), 1.32 (d, *J* = 6.6 Hz, 3H), 1.30 - 1.27 (m, 2H). LC-MS: m/z 374.6 [M+H]$^+$. |
| 74 | 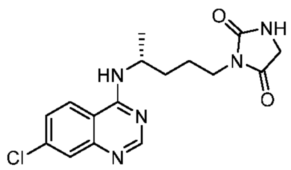 | (R)-3-(4-((7-Chloroquinazolin-4-yl)amino)pentyl)imidazolidine-2,4-dione<br>$^1$H NMR (400 MHz, MeOD) δ 8.43 (s, 1H), 8.18 (d, *J* = 8.9 Hz, 1H), 7.67 (d, *J* = 2.1 Hz, 1H), 7.48 (dd, *J* = 8.9, 2.1 Hz, 1H), 4.60 (d, *J* = 6.6 Hz, 1H), 3.93 (s, 2H), 3.57 - 3.49 (m, 2H), 1.78 - 1.63 (m, 4H), 1.31 (d, *J* = 6.6 Hz, 3H). LC-MS: m/z 348.1 [M+H]$^+$. |
| **75** | 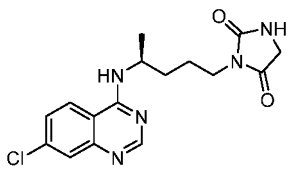 | (*S*)-3-(4-((7-Chloroquinazolin-4-yl)amino)pentyl)imidazolidine-2,4-dione<br>$^1$H NMR (400 MHz, MeOD) δ 8.44 (s, 1H), 8.19 (d, *J* = 8.9 Hz, 1H), 7.68 (d, *J* = 2.1 Hz, 1H), 7.49 (dd, *J* = 8.9, 2.2 Hz, 1H), 4.58 (d, *J* = 6.5 Hz, 1H), 3.93 (s, 2H), 3.58 - 3.49 (m, 2H), 1.76 - 1.64 (m, 4H), 1.32 (d, *J* = 6.6 Hz, 3H). LC-MS: m/z 348.1 [M+H]$^+$. |
| **76** | 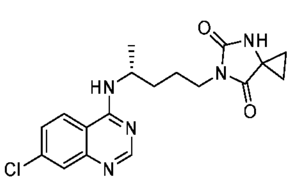 | (R)-6-(4-((7-Chloroquinazolin-4-yl)amino)pentyl)-4,6-diazaspiro[2.4] heptane-5,7-dione<br>$^1$H NMR (400 MHz, MeOD) δ 8.43 (s, 1H), 8.20 (d, *J* = 8.9 Hz, 1H), 7.67 (d, *J* = 2.1 Hz, 1H), 7.50 (dd, *J* = 8.9, 2.1 Hz, 1H), 4.61 - 4.54 (m, 1H), 3.57 (t, *J* = 6.4 Hz, 2H), 1.79 - 1.69 (m, 4H), 1.38 - 1.34 (m, 2H), 1.33 (d, *J* = 6.5 Hz, 3H), 1.32 - 1.26 (m, 2H). LC-MS: m/z 374.6 [M+H]$^+$. |
| **77** | 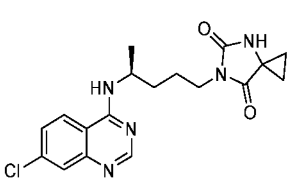 | (*S*)-6-(4-((7-Chloroquinazolin-4-yl)amino)pentyl)-4,6-diazaspiro[2.4] heptane-5,7-dione<br>$^1$H NMR (400 MHz, MeOD) δ 8.45 (s, 1H), 8.22 (d, *J* = 8.9 Hz, 1H), 7.70 (d, *J* = 2.2 Hz, 1H), 7.52 (dd, *J* = 8.9, 2.2 Hz, 1H), 4.65 - 4.58 (m, 1H), 3.60 (t, *J* = 6.5 Hz, 2H), 1.78 - 1.68 (m, 4H), 1.38 - 1.35 (m, 2H), 1.34 (d, *J* = 6.6 Hz, 3H), 1.31 - 1.27 (m, 2H). LC-MS: m/z 374.6 [M+H]$^+$. |

Effect example 1: Determination of $IC_{50}$ values for inhibitory effects of compounds on cytokines

Experimental methods and steps

(1) Cell culture and inoculation: A stock solution of PMA at 1 mg/mL was diluted with serum-free RP1640 medium to prepare a 100 ng/mL PMA solution. Normal cultured THP-1 cells were centrifuged, resuspended in a complete medium (RP1640 medium containing 10% FBS), and counted. The cell density was adjusted to $2\times10^6$ cells/mL. An equal volume of 100 ng/mL PMA solution was then added, resulting in a cell density of $1\times10^6$ cells/mL and a final PMA concentration of 50 ng/mL. 100 μL of the cell suspension was seeded into each well of a 96-well cell culture plate with $1\times10^5$ cells per well. The 96-well cell culture plate was then incubated at 37°C in a 5% $CO_2$ atmosphere for 24 hours.

(2) Drug treatment of cells: On the second day, LPS was added to the complete medium to adjust the final concentration to 100 ng/mL, and the compound was prepared using this LPS-containing medium. The compound was added to the appropriate cell wells according to the prepared concentration gradient. The positive control group (LPS+) was a culture medium solution containing a final concentration of 100 ng/mL LPS and 0.1% DMSO without drugs, while the negative control group (LPS-) was a culture medium solution containing 0.1% DMSO without LPS. The volume in each well was 100 μL.

(3) Test sample collection: The mixture was centrifuged at 1000 rpm for 5 minutes and the cell supernatant and cell precipitate were collected separately. The cell supernatant was used to detect TNF-α and IL-6, and the cell precipitate was collected for IL-1β detection. After collecting the cell supernatant, the remaining liquid in the wells was discarded

and 100 μL of cell lysis solution (20 mM Tris 8.0; 2 mM EDTA; 0.5% Triton X-100) was added to each well for complete lysis.

(4) Sample detection: The enzyme-linked immunosorbent assay (ELISA) was used to detect the content of each cytokine. The specific steps were performed according to the instructions provided in the corresponding kit manuals. The catalog numbers of kits are as follows:

Human TNF-alpha Quantikine ELISA Kit, Catalog # STA00D
Human IL-1 beta/IL-1F2 Quantikine ELISA Kit, Catalog # SLB50
Human IL-6 Quantikine ELISA Kit, Catalog # S6050.

(5) Data analysis: The *in vitro* inhibitory activity of the compounds was calculated using the following formula:

$$\text{Inhibition rate (\%)}=[1-(OD450_{\text{with drug}}-OD450_{LPS-})/(OD450_{LPS+}-OD450_{LPS-})] \times 100\%.$$

In this assay, the compounds of the present disclosure exhibited $IC_{50}$ values below 2000 μM. Some compounds showed $IC_{50}$ values below 10 μM. Tables 7 and 8 provide the specific $IC_{50}$ values of the compounds of the present disclosure tested in this assay.

Table 7: Specific $IC_{50}$ values of the compounds of the present disclosure in this assay

| Example | TNF-α $IC_{50}$ (μM) | IL-6 $IC_{50}$ (μM) | IL-1β $IC_{50}$ (μM) |
|---|---|---|---|
| 1 | 31.66 | 0.86 | 23.08 |
| 2 | 10.43 | 1.17 | 17.92 |
| 3 | 56.51 | 5.53 | / |
| 4 | 5.54 | 1.28 | 14.40 |
| 5 | 25.56 | 3.95 | 19.53 |
| 6 | 32.69 | 23.94 | / |
| 7 | 12.17 | 2.06 | 30.06 |
| 8 | 18.39 | 3.29 | 34.13 |
| 9 | 28.27 | 10.49 | 48.26 |
| 10 | 8.91 | 0.38 | 15.02 |
| 11 | 29.95 | 10.64 | 24.29 |
| 12 | / | 20.5 | / |
| 13 | 7.83 | 1.80 | 28.63 |
| 14 | / | 2.98 | / |
| 15 | 17.43 | 1.24 | / |
| 16 | 53.16 | 0.59 | 23.42 |
| 17 | 4.72 | 0.641 | 23.59 |
| 18 | 3.26 | 0.226 | 2.938 |
| 19 | 14.32 | 0.81 | 2.2 |
| 20 | 6.4 | 0.47 | 5.9 |
| 21 | 1.41 | 0.19 | 0.65 |
| 22 | 6.91 | 2.06 | 9.79 |
| 23 | 2.79 | 0.38 | 1.36 |
| 24 | 4.77 | 1.2 | 4.46 |

(continued)

| Example | TNF-α $IC_{50}$ (μM) | IL-6 $IC_{50}$ (μM) | IL-1β $IC_{50}$ (μM) |
|---|---|---|---|
| 25 | 5.07 | 1.32 | 4.36 |
| 26 | 3.82 | 0.33 | 14.02 |
| 27 | 2.17 | 0.53 | 5.78 |
| 28 | 11.19 | 0.68 | 10.86 |
| 29 | 2.63 | 0.36 | 4.108 |
| 30 | 0.97 | 0.12 | 1.337 |
| 31 | 7.13 | 1.31 | 8.639 |
| 32 | / | 0.63 | / |
| 33 | / | 3.85 | / |
| 34 | 33.35 | 3.16 | 36.61 |
| 35 | 20.12 | 0.82 | 5.66 |
| 36 | 6.86 | 1.05 | 17.17 |
| 37 | 4.88 | 0.18 | 41.75 |
| 38 | 7.28 | 0.12 | / |
| 39 | 7.89 | 0.77 | 7.4 |
| 40 | 10.1 | 0.6 | 0.7 |
| 41 | / | 30.4 | / |
| 42 | 6.56 | 2.84 | 3.31 |
| 43 | 4.01 | 1.57 | 33.75 |
| 44 | 19.91 | 0.77 | 21.12 |
| 45 | 12.6 | 1.09 | 10.29 |
| 46 | 56.7 | 0.27 | / |
| 47 | 58.06 | 1.94 | / |
| 48 | 14.79 | 0.91 | 49.59 |
| 49 | 18.54 | 1.15 | 48.24 |
| HCQ | 69.33 | 32.00 | 62.2 |

Table 8: Specific $IC_{50}$ values of the compounds of the present disclosure in this assay

| Example | TNF-α $IC_{50}$ (μM) | IL-6 $IC_{50}$ (μM) | IL-1β $IC_{50}$ (μM) |
|---|---|---|---|
| 50 | / | 3.99 | 121.50 |
| 51 | 3.96 | 0.04 | 3.52 |
| 52 | 28.32 | 0.28 | 21.03 |
| 53 | / | 0.52 | 118.40 |
| 54 | 113.70 | 1.05 | 154.30 |
| 55 | 34.90 | 0.34 | 63.83 |
| 56 | 158.90 | 18.75 | 16.27 |
| 57 | 29.43 | 3.22 | 53.92 |

(continued)

| Example | TNF-α $IC_{50}$ (μM) | IL-6 $IC_{50}$ (μM) | IL-1β $IC_{50}$ (μM) |
|---|---|---|---|
| 58 | 90.71 | 2.58 | 86.47 |
| 59 | 12.12 | 0.37 | 80.52 |
| 60 | / | 34.03 | / |
| 61 | / | 5.84 | 134.00 |
| 62 | 7.18 | 0.75 | 51.61 |
| 63 | 79.37 | 14.38 | 23.05 |
| 64 | 34.90 | 0.34 | 63.83 |
| 65 | / | 2.02 | / |
| 66 | 3.41 | 0.30 | 37.38 |
| 67 | 65.15 | 4.6 | 105 |
| 68 | / | 0.42 | / |
| 69 | 11.65 | 0.58 | 13.66 |
| 70 | / | 0.46 | / |
| 71 | 12.72 | 0.64 | 16.12 |
| 72 | 7.5 | 0.23 | 12.78 |
| 73 | 33.53 | 1.50 | 38.11 |
| 74 | / | 12.41 | / |
| 75 | 45.57 | 4.28 | 53.78 |
| 76 | 96.06 | 19.91 | 109.90 |
| 77 | 22.68 | 1.37 | 26.55 |
| HCQ | 90.54 | 123.20 | 164.40 |

Although the specific embodiments of the present disclosure have been described above, those skilled in the art should understand that these are merely illustrative examples. Various changes or modifications can be made to these embodiments without departing from the principles and essence of the present disclosure. Therefore, the scope of protection for the present disclosure is defined by the appended claims.

## Claims

**1.** A quinazoline compound of formula I, a pharmaceutically acceptable salt thereof, a solvate thereof, or a solvate of the pharmaceutically acceptable salt thereof,

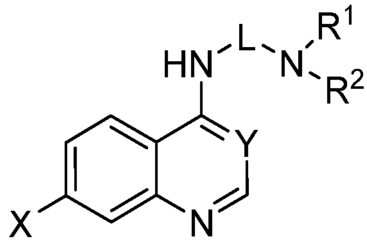

I

wherein X is halogen;

Y is CH or N;
L is $-(CH_2)_m-$ or $-(CH_2)_m-$ substituted by 1, 2, or 3 $C_{1-4}$ alkyl groups; m is 4, 5, or 6;
$R^1$ is H or

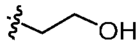 ;

$R^2$ is $C_{1-4}$ alkyl or $C_{1-4}$ alkyl substituted by 1, 2, or 3 $R^{2-1}$;
$R^{2-1}$ is 3- to 7-membered heterocycloalkyl, 5- to 6-membered heteroaryl, $C_{6-12}$ aryl, 3- to 7-membered heterocycloalkyl substituted by 1, 2, or 3 $R^{2-1-1}$, 5- to 6-membered heteroaryl substituted by 1, 2, or 3 $R^{2-1-2}$, or $C_{6-12}$ aryl substituted by 1, 2, or 3 $R^{2-1-3}$; in the 5- to 6-membered heteroaryl and the 5- to 6-membered heteroaryl substituted by 1, 2, or 3 $R^{2-1-2}$, the heteroatom in the 5- to 6-membered heteroaryl is 1, 2, or 3 heteroatoms selected from one or more than one of oxygen, sulfur, and nitrogen; in the 3- to 7-membered heterocycloalkyl and the 3- to 7-membered heterocycloalkyl substituted by 1, 2, or 3 $R^{2-1-1}$, the heteroatom in the 3-to 7-membered heterocycloalkyl is 1, 2, or 3 heteroatoms selected from one or more than one of oxygen, sulfur, and nitrogen;
$R^{2-1-1}$ is independently $C_{1-4}$ alkyl;
$R^{2-1-2}$ is independently $C_{1-4}$ alkyl or $C_{1-4}$ alkoxy;
$R^{2-1-3}$ is independently halogen, cyano, $C_{1-4}$ alkoxy, $C_{1-4}$ alkyl substituted by 1, 2, or 3 halogens, $C_{1-4}$ alkoxy substituted by 1, 2, or 3 halogens, $C_{6-12}$ aryl, 5- to 6-membered heteroaryl,

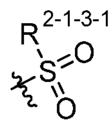 , or 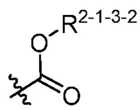 ;

the heteroatom in the 5- to 6-membered heteroaryl is 1, 2, or 3 heteroatoms selected from one or more than one of oxygen, sulfur, and nitrogen;
$R^{2-1-3-1}$ is independently $C_{1-4}$ alkyl;
$R^{2-1-3-2}$ is independently $C_{1-4}$ alkyl;
alternatively, $R^1$ and $R^2$, together with the nitrogen atom between them, form a ring A, and the ring A is 4- to 12-membered heterocycloalkyl or 4- to 12-membered heterocycloalkyl substituted by 1, 2, or 3 $R^{2-2}$; in the case where ring A is 4- to 12-membered heterocycloalkyl or 4- to 12-membered heterocycloalkyl substituted by 1, 2, or 3 $R^{2-2}$, the heteroatom in the 4-to 12-membered heterocycloalkyl is 1, 2, or 3 heteroatoms selected from one or more than one of oxygen, sulfur, and nitrogen;
$R^{2-2}$ is independently oxo, $C_{1-4}$ alkyl, or halogen.

2. The quinazoline compound of formula I, the pharmaceutically acceptable salt thereof, the solvate thereof, or the solvate of the pharmaceutically acceptable salt thereof according to claim 1, wherein the quinazoline compound of formula I satisfies 1, 2, or 3 of the following conditions:

i: $R^1$ is

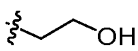 ;

ii: $R^2$ is $C_{1-4}$ alkyl substituted by 1, 2, or 3 $R^{2-1}$, and $R^{2-1}$ is 3- to 7-membered heterocycloalkyl, 5- to 6-membered heteroaryl, 3- to 7-membered heterocycloalkyl substituted by 1, 2, or 3 $R^{2-1-1}$, or 5- to 6-membered heteroaryl substituted by 1, 2, or 3 $R^{2-1-2}$;
iii: $R^1$ and $R^2$, together with the nitrogen atom between them, form a ring A, and the ring A is a spiro or bridged ring; alternatively, $R^1$ and $R^2$, together with the nitrogen atom between them, form a ring A, and the ring A is a monocyclic ring, wherein the ring A is 4- to 12-membered heterocycloalkyl substituted by 2 or 3 $R^{2-2}$; $R^{2-2}$ is independently oxo, $C_{1-4}$ alkyl, or halogen, and when substituents in the 4- to 12-membered heterocycloalkyl contain oxo, the number of oxo groups is 2 or 3.

3. The quinazoline compound of formula I, the pharmaceutically acceptable salt thereof, the solvate thereof, or the solvate of the pharmaceutically acceptable salt thereof according to claim 1, wherein the compound satisfies one or more than one of the following conditions:

(1) L is $-(CH_2)_m-$ or $-(CH_2)_m-$ substituted by 1 methyl group; m is 4 or 5;
(2) $R^2$ is $C_{1-4}$ alkyl substituted by 1, 2, or 3 $R^{2-1}$, preferably methyl substituted by 1 or 2 $R^{2-1}$ or ethyl substituted by 1 or 2 $R^{2-1}$;
(3) $R^{2-1-3}$ is independently halogen, cyano, $C_{1-4}$ alkoxy, $C_{1-4}$ alkyl substituted by 1, 2, or 3 halogens, $C_{1-4}$ alkoxy substituted by 1, 2, or 3 halogens, 5- to 6-membered heteroaryl,

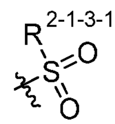 ,

or

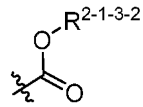 ;

the heteroatom in the 5- to 6-membered heteroaryl is 1, 2, or 3 heteroatoms selected from one or more than one of oxygen, sulfur, and nitrogen;
(4) $R^1$ and $R^2$, together with the nitrogen atom between them, form a ring A, wherein the ring A is 5- to 12-membered heterocycloalkyl $A_1$, and the heterocycloalkyl $A_1$ is a bridged ring; or the ring A is 5- to 10-memebered heterocycloalkyl $A_2$ substituted by 2 or 3 $R^{2-2-1}$, and the heterocycloalkyl $A_3$ is a monocyclic ring; or the ring A is 8- to 12-membered heterocycloalkyl $A_3$ substituted by 1, 2, or 3 $R^{2-2-2}$, and the heterocycloalkyl $A_3$ is a spiro ring; $R^{2-2-1}$ is independently oxo, $C_{1-4}$ alkyl, or halogen; $R^{2-2-2}$ is independently oxo, $C_{1-4}$ alkyl, or halogen; in $A_2$, when substituents in the 5- to 10-membered heterocycloalkyl contain oxo, the number of oxo groups is 2 or 3.

**4.** The quinazoline compound of formula I, the pharmaceutically acceptable salt thereof, the solvate thereof, or the solvate of the pharmaceutically acceptable salt thereof according to claim 1, wherein when $R^1$ and $R^2$, together with the nitrogen atom between them, form a ring A, the ring A is

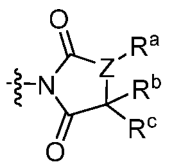 or 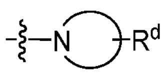 ;

wherein Z is CH or N;
$R^a$, $R^b$, and $R^c$ are each independently H or $C_{1-4}$ alkyl; preferably, $R^a$ is H, $R^b$ and $R^c$ are each independently $C_{1-4}$ alkyl; alternatively, $R^b$ and $R^c$, together with the atom to which they are attached, form a $C_{3-6}$ hydrocarbon ring or a 3- to 6-membered heterocycle; the heteroatom in the 3- to 6-membered heterocycle is 1 or 2 heteroatoms selected from one or more than one of oxygen, sulfur, and nitrogen;
the structural moiety

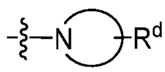

represents 3- to 6-membered nitrogen-containing heterocycloalkyl substituted by 2 or 3 $R^d$; aside from nitrogen, the heteroatom in the heterocyloalkyl is 1, 2, or 3 heteroatoms selected from one or more than one of oxygen, sulfur, and nitrogen;
$R^d$ is H, oxo, or halogen; or adjacent two $R^d$, together with the atom to which they are attached, form a $C_{3-6}$ hydrocarbon ring, a 3- to 6-membered heterocycle, a $C_{3-6}$ hydrocarbon ring substituted by 1, 2, or 3 $R^{d-1}$, or a 3- to 6-membered heterocycle substituted by 1, 2, or 3 $R^{d-1}$; or two $R^d$ linking to the same atom, together with the atom to which they are attached, form a $C_{3-6}$ hydrocarbon ring, a 3- to 6-membered heterocycle, a $C_{3-6}$ hydrocarbon ring substituted by 1, 2, or 3 $R^{d-1}$, or a 3- to 6-membered heterocycle substituted by 1, 2, or 3 $R^{d-1}$;

the heteroatom in the heterocycle is 1, 2, or 3 heteroatoms selected from one or more than one of oxygen, sulfur, and nitrogen;
$R^{d-1}$ is independently $C_{1-4}$ alkyl.

**5.** The quinazoline compound of formula I, the pharmaceutically acceptable salt thereof, the solvate thereof, or the solvate of the pharmaceutically acceptable salt thereof according to at least one of claims 1 to 4, wherein the compound satisfies one or more than one of the following conditions:

(1) L is

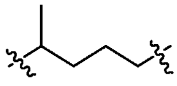, 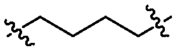, or 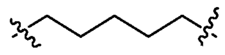, such as 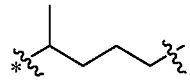,

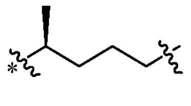, 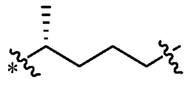, 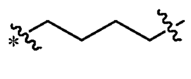, or 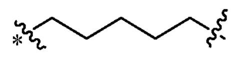,

wherein the side marked with * indicates connection to the N in the NH structure of the parent nucleus;
(2) X is F or Cl;
(3) $R^2$ is

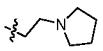, 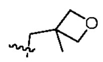, 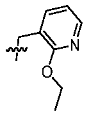, 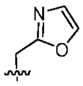, 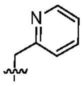, 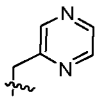, 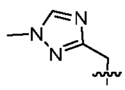, 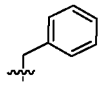,

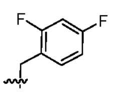, 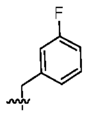, 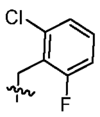, 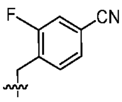, 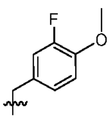, 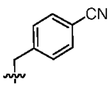, 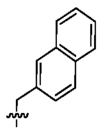, 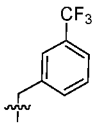, 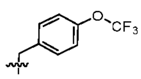,

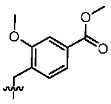, 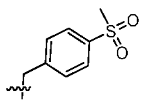, 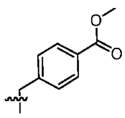, or 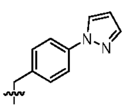;

(4) when $R^1$ and $R^2$, together with the nitrogen atom between them, form a ring A, and the ring A is

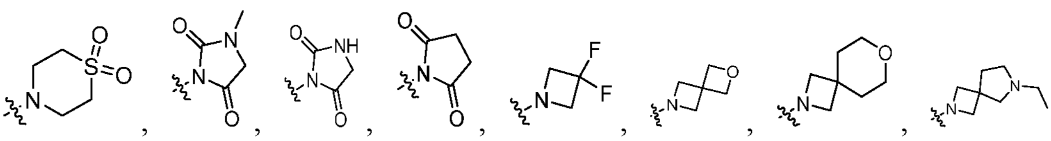

,

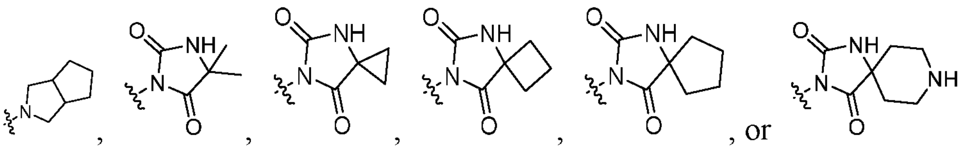

, or .

**6.** The quinazoline compound of formula I, the pharmaceutically acceptable salt thereof, the solvate thereof, or the solvate of the pharmaceutically acceptable salt thereof according to at least one of claims 1 to 3, wherein the compound satisfies one or more than one of the following conditions:

(1) in $R^2$, in the $C_{1-4}$ alkyl and the $C_{1-4}$ alkyl substituted by 1, 2, or 3 $R^{2-1}$, the $C_{1-4}$ alkyl is independently methyl, ethyl, n-propyl, isopropyl, n-butyl, tert-butyl, isobutyl, or sec-butyl, such as methyl or ethyl;
(2) in $R^{2-1}$, in the 3- to 7-membered heterocycloalkyl and the 3- to 7-membered heterocycloalkyl substituted by 1, 2, or 3 $R^{2-1-1}$, the number of heteroatoms in the 3- to 7-membered heterocycloalkyl is independently 1;
(3) in $R^{2-1}$, in the 3- to 7-membered heterocycloalkyl and the 3- to 7-membered heterocycloalkyl substituted by 1, 2, or 3 $R^{2-1-1}$, the heteroatom in the 3- to 7-membered heterocycloalkyl is independently N or O;
(4) in $R^{2-1}$, in the 3- to 7-membered heterocycloalkyl and the 3- to 7-membered heterocycloalkyl substituted by 1, 2, or 3 $R^{2-1-1}$, the 3- to 7-membered heterocycloalkyl is independently 4- to 5-membered heterocycloalkyl;
(5) in $R^{2-1}$, in the 5- to 6-membered heteroaryl and the 5- to 6-membered heteroaryl substituted by 1, 2, or 3 $R^{2-1-2}$, the type of heteroatoms in the 5- to 6-membered heteroaryl is selected from one or both of N and O;
(6) in $R^{2-1-1}$ and $R^{2-1-2}$, the $C_{1-4}$ alkyl is independently methyl, ethyl, n-propyl, isopropyl, n-butyl, tert-butyl, isobutyl, or sec-butyl, such as methyl;
(7) in $R^{2-1-2}$, the $C_{1-4}$ alkoxy is independently methoxy, ethoxy, or propoxy, such as ethoxy;
(8) in $R^{2-1-3}$, the halogen is independently F, Cl, Br, or I, such as F or Cl;
(9) in $R^{2-1-1}$ the $C_{1-4}$ alkoxy is independently methoxy, ethoxy, or propoxy, such as methoxy;
(10) in $R^{2-1-1}$, the $C_{1-4}$ alkyl substituted by 1, 2, or 3 halogens is independently trifluoromethyl, trifluoroethyl, or trifluoropropyl, such as trifluoromethyl;
(11) in $R^{2-1-3}$, the $C_{1-4}$ alkoxy substituted by 1, 2, or 3 halogens is independently trifluoromethoxy, trifluoroethoxy, or trifluoropropoxy, such as trifluoromethoxy;
(12) in $R^{2-1-3}$, the $C_{6-12}$ aryl is phenyl or naphthyl;
(13) in $R^{2-1-3}$, the 5- to 6-membered heteroaryl is pyrazolyl, pyrazinyl, pyridazinyl, pyridyl, pyrimidinyl, or pyrrolyl, such as pyrazolyl, also such as

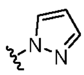 ;

(14) in $R^{2-1-3-1}$ and $R^{2-1-3-2}$, the $C_{1-4}$ alkyl is independently methyl, ethyl, *n*-propyl, isopropyl, *n*-butyl, *tert*-butyl, isobutyl, or *sec*-butyl, such as methyl;
(15) in $R^{2-2}$, the $C_{1-4}$ alkyl is independently methyl, ethyl, *n*-propyl, isopropyl, *n*-butyl, *tert*-butyl, isobutyl, or *sec*-butyl, such as methyl or ethyl;
(16) in $R^{2-2}$, the halogen is independently F, Cl, Br, or I, such as F.

**7.** The quinazoline compound of formula I, the pharmaceutically acceptable salt thereof, the solvate thereof, or the solvate of the pharmaceutically acceptable salt thereof according to claim 6, wherein the compound satisfies one or more than one of the following conditions:

(1) in $R^{2-1}$, in the 3- to 7-membered heterocycloalkyl and the 3- to 7-membered heterocycloalkyl substituted by 1, 2, or 3 $R^{2-1-1}$, the 3- to 7-membered heterocycloalkyl is independently tetrahydropyrrolyl or oxetanyl, such as

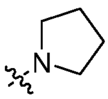 or 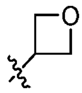 ;

(2) in $R^{2-1}$, in the 5- to 6-membered heteroaryl and the 5- to 6-membered heteroaryl substituted by 1, 2, or 3 $R^{2-1-2}$, the 5- to 6-membered heteroaryl is independently oxazolyl, triazolyl, pyridyl, or pyrazinyl, such as

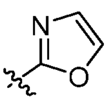 , 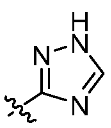 , 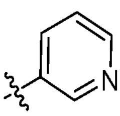 , 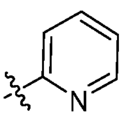 ,

or

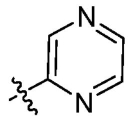
;

(3) in $R^{2-1}$, in the $C_{6-12}$ aryl and the $C_{6-12}$ aryl substituted by 1, 2, or 3 $R^{2-1-1}$, the $C_{6-12}$ aryl is independently phenyl or naphthyl, such as

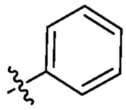 or 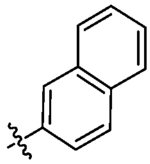.

**8.** The quinazoline compound of formula I, the pharmaceutically acceptable salt thereof, the solvate thereof, or the solvate of the pharmaceutically acceptable salt thereof according to claim 4, wherein the compound satisfies one or more than one of the following conditions:

(1) in $R^a$, $R^b$, and $R^c$, the $C_{1-4}$ alkyl is independently methyl, ethyl, *n*-propyl, isopropyl, *n*-butyl, *tert*-butyl, isobutyl, or *sec*-butyl, such as methyl;
(2) $R^b$ and $R^c$, together with the atom to which they are attached, form a $C_{3-6}$ hydrocarbon ring, and the $C_{3-6}$ hydrocarbon ring is cyclopropane, cyclobutane, cyclopentane, or cyclohexane, such as cyclopropane, cyclobutane, or cyclopentane;
(3) when $R^b$ and $R^c$, together with the atom to which they are attached, form a 3- to 6-membered heterocycle, the number of heteroatoms in the 3- to 6-membered heterocycle is 1;
(4) when $R^b$ and $R^c$, together with the atom to which they are attached, form a 3- to 6-membered heterocycle, the heteroatom in the 3- to 6-membered heterocycle is N;
(5) when $R^b$ and $R^c$, together with the atom to which they are attached, form a 3- to 6-membered heterocycle, the 3- to 6-membered heterocycle is a 6-membered heterocycle;
(6) in the definition of

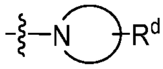,

in the 3- to 6-membered nitrogen-containing heterocycloalkyl, the number of heteroatoms is 1 or 2;
(7) in the definition of

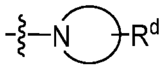,

in the 3- to 6-membered nitrogen-containing heterocycloalkyl, the heteroatom is independently N, N, or S;
(8) in the definition of

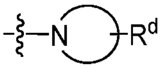,

the $C_{3-6}$ hydrocarbon ring is independently cyclopropane, cyclobutane, cyclopentane, or cyclohexane, such as cyclopentane;
(9) in the definition of

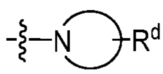

, in the 3- to 6-membered heterocycle, the number of heteroatoms is independently 1;

(10) in the definition of

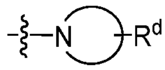

,

in the 3- to 6-membered heterocycle, the heteroatom is independently N or O;

(11) in $R^{d-1}$, the $C_{1-4}$ alkyl is methyl, ethyl, *n*-propyl, isopropyl, *n*-butyl, *tert*-butyl, isobutyl, or sec-butyl, such as methyl.

**9.** The quinazoline compound of formula I, the pharmaceutically acceptable salt thereof, the solvate thereof, or the solvate of the pharmaceutically acceptable salt thereof according to claim 8, wherein the compound satisfies one or more than one of the following conditions:

(1) when $R^b$ and $R^c$, together with the atom to which they are attached, form a 3- to 6-membered heterocycle, the 3- to 6-membered heterocycle is piperidine, such as

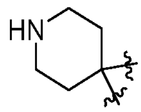

;

(2) in the definition of

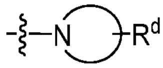

, the 3- to 6-membered nitrogen-containing heterocycloalkyl is independently thiomorpholinyl, tetrahydropyrrolyl, or azetidinyl, such as

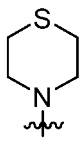

,

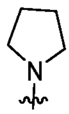

, or

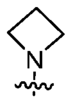

;

(3) in the definition of

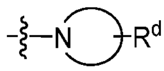

,

the 3- to 6-membered heterocycle is independently oxetane, tetrahydropyrrole, or tetrahydropyran, for example, when two $R^d$ linking the same atom, together with the atom to which they are attached, form a 3- to 6-membered heterocycle or a 3- to 6-membered heterocycle substituted by 1, 2, or 3 $R^{d-1}$, the 3- to 6-membered heterocycle is

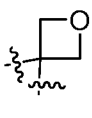

,

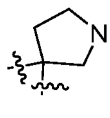

, or

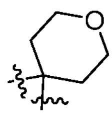

.

**10.** The quinazoline compound of formula I, the pharmaceutically acceptable salt thereof, the solvate thereof, or the solvate of the pharmaceutically acceptable salt thereof according to claim 1, wherein the compound satisfies any one of the following conditions:

(1) X is halogen; Y is CH or N; $R^1$ is

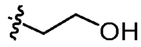

; $R^2$ is $C_{1-4}$ alkyl substituted by 1, 2, or 3 $R^{2-1}$; $R^{2-1}$ is $C_{6-12}$ aryl substituted by 1, 2, or 3 $R^{2-1-3}$; L is preferably

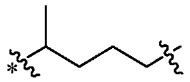;

(2) X is halogen; Y is N; $R^1$ is

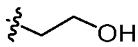, $R^2$ is 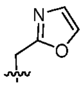,

L is $-(CH_2)_m-$, and m is 4 or 5;
(3) X is Cl; Y is CH; L is $-(CH_2)_m-$ substituted by 1 methyl group, and $R^1$ and $R^2$, together with the nitrogen atom between them, form a ring A, and the ring A is

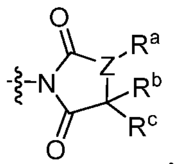,

wherein $R^a$ is $C_{1-4}$ alkyl, and $R^b$ and $R^c$ are both H;
(4) X is Cl; Y is N; L is $-(CH_2)_m-$ substituted by 1 methyl group, and $R^1$ and $R^2$, together with the nitrogen atom between them, form a ring A, and the ring A is

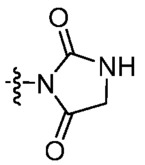 or 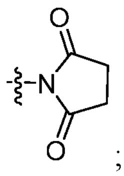;

(5) X is Cl; Y is CH; L is $-(CH_2)_m-$ substituted by 1 methyl group; $R^1$ and $R^2$, together with the nitrogen atom between them, form a ring A, wherein the ring A is

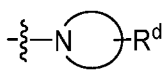,

and $R^d$ is oxo or halogen; or adjacent two $R^d$, together with the atom to which they are attached, form a $C_{3-6}$ hydrocarbon ring; or two $R^d$ linking to the same atom, together with the atom to which they are attached, form a 3- to 6-membered heterocycle or a 3- to 6-membered heterocycle substituted by 1, 2, or 3 $R^{d-1}$; the heteroatom in the heterocycle is 1, 2, or 3 heteroatoms selected from one or more than one of oxygen, sulfur, and nitrogen;
(6) X is Cl; Y is CH; L is $-(CH_2)_m-$ substituted by 1 methyl group, and m is 4 or 5; $R^1$ is H; $R^2$ is $C_{1-4}$ alkyl substituted by 1, 2, or 3 $R^{2-1}$, and $R^{2-1}$ is 3- to 7-membered heterocycloalkyl substituted by 1, 2, or 3 $R^{2-1-1}$.

**11.** The quinazoline compound of formula I, the pharmaceutically acceptable salt thereof, the solvate thereof, or the solvate of the pharmaceutically acceptable salt thereof according to claim 1, wherein the quinazoline compound of formula I is a compound of formula I-1 or I-1':

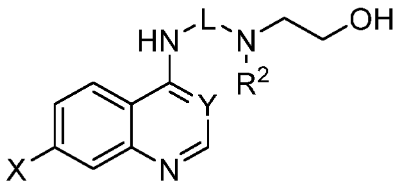

I-1

,

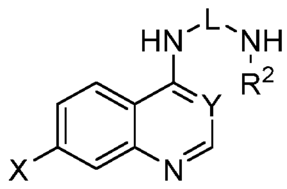

I-1'

.

12. The quinazoline compound of formula I, the pharmaceutically acceptable salt thereof, the solvate thereof, or the solvate of the pharmaceutically acceptable salt thereof according to claim 1, wherein the quinazoline compound of formula I is a compound of formula I-2:

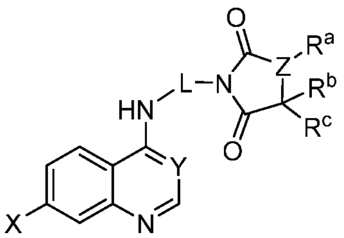

I-2

.

13. The quinazoline compound of formula I, the pharmaceutically acceptable salt thereof, the solvate thereof, or the solvate of the pharmaceutically acceptable salt thereof according to claim 1, wherein the quinazoline compound of formula I is a compound of formula I-3:

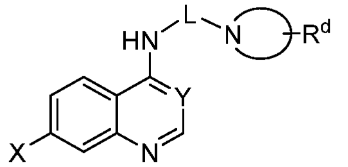

I-3

.

14. The quinazoline compound of formula I, the pharmaceutically acceptable salt thereof, the solvate thereof, or the solvate of the pharmaceutically acceptable salt thereof according to at least one of claims 1 to 13, wherein the quinazoline compound of formula I is any one of the following compounds:

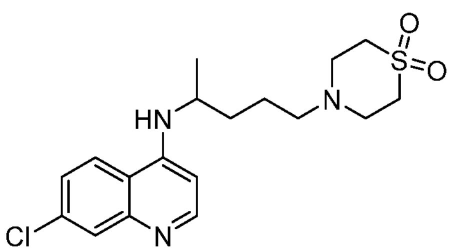

,

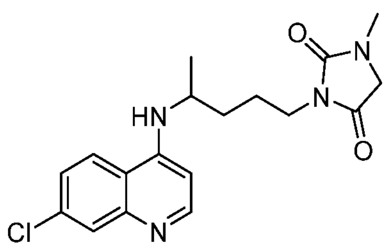

,

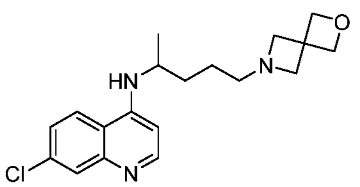

,

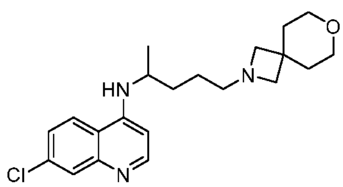

,

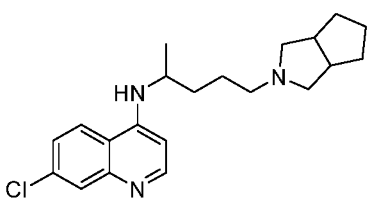

,

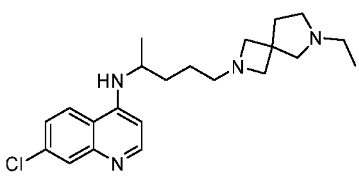 , 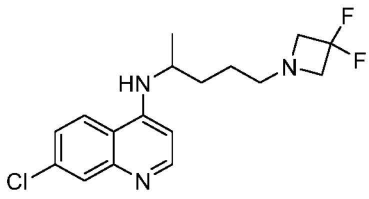 , 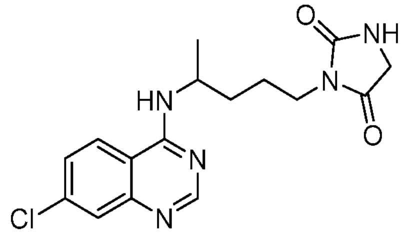 ,

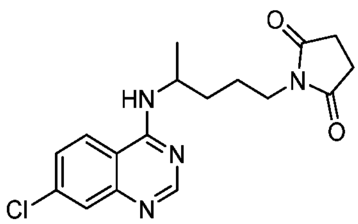 , 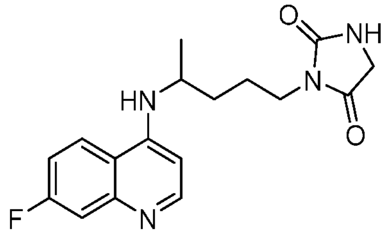 , 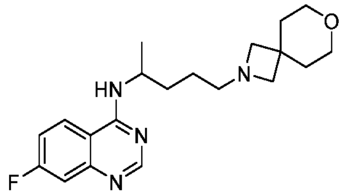 ,

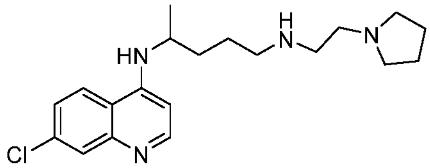 , 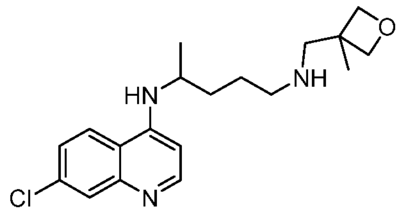 ,

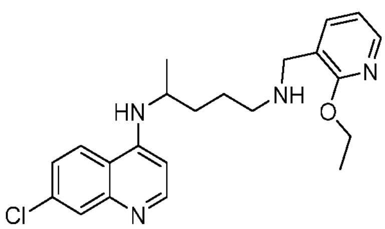 , 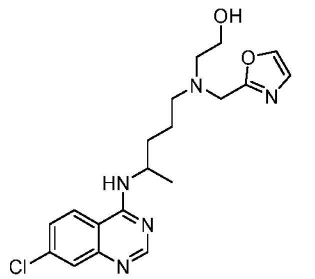 , 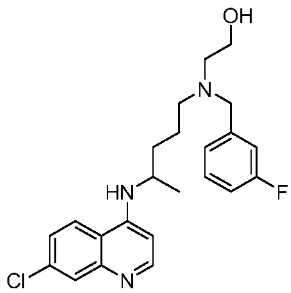 ,

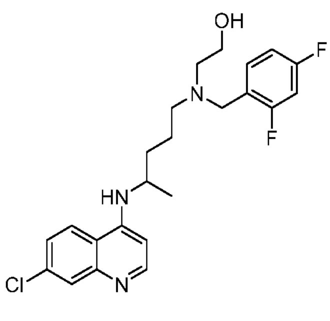 , 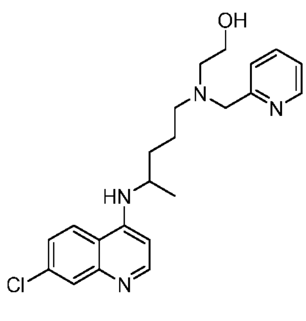 , 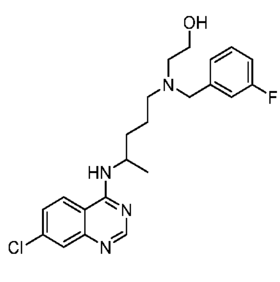 ,

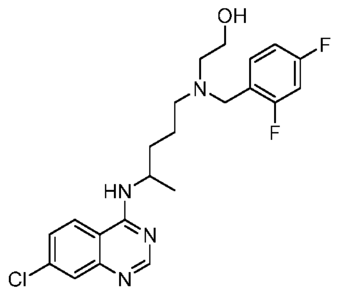 , 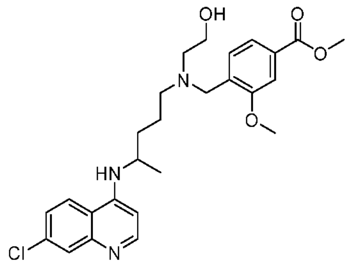 , 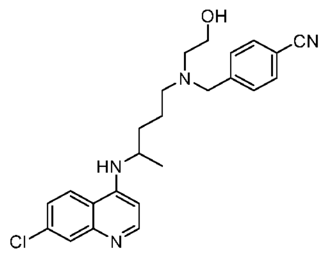 ,

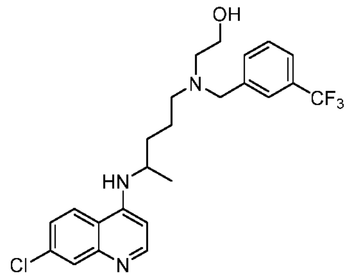 , 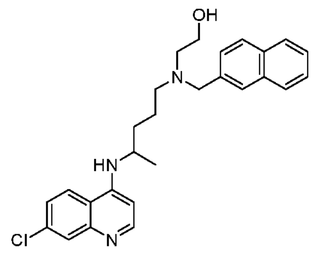 , 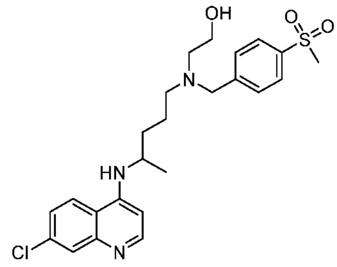 ,

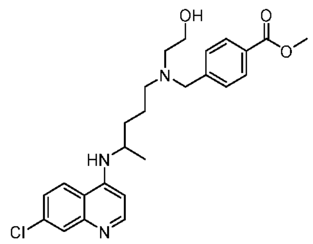 , 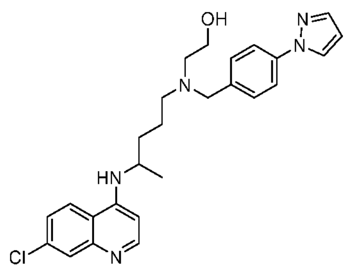 , 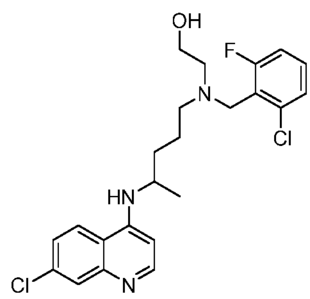 ,

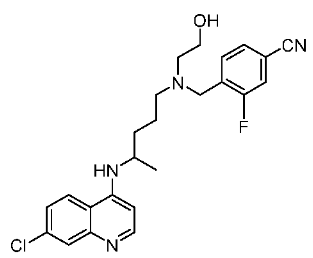 , 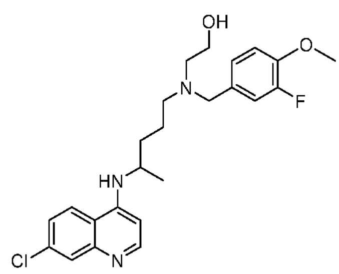 , 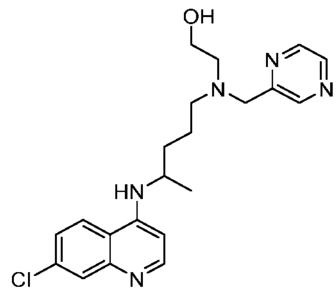 ,

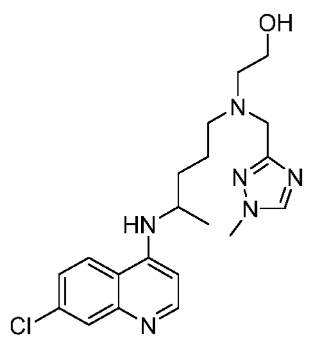 , 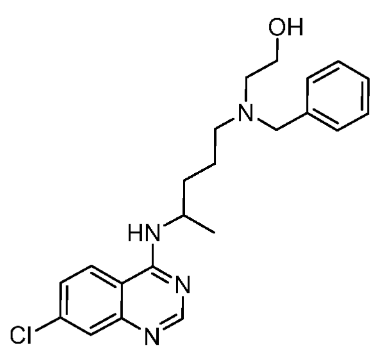 , 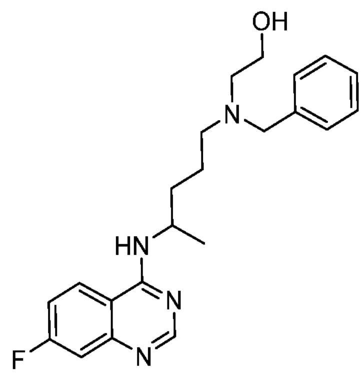 ,

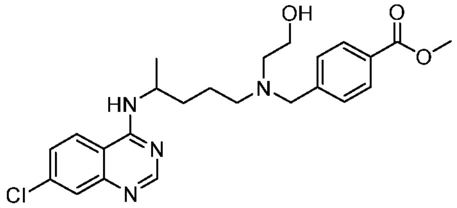

,

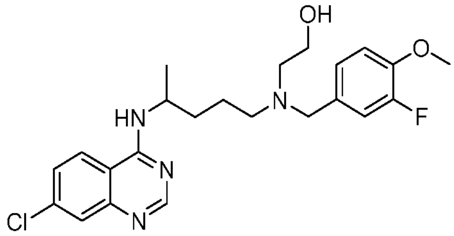

,

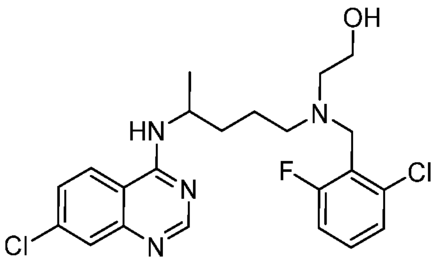

,

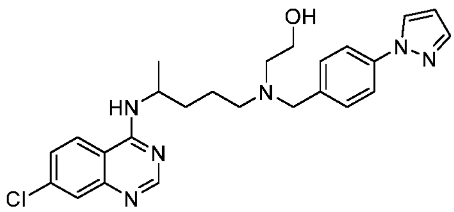

,

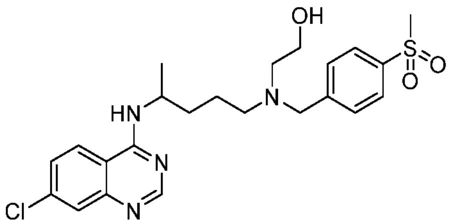

,

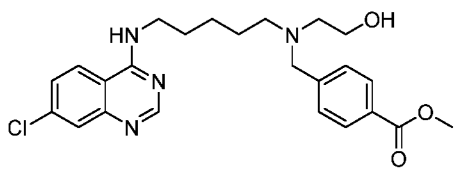

,

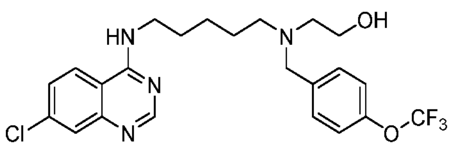

,

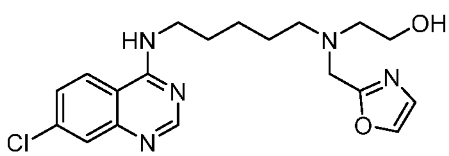

,

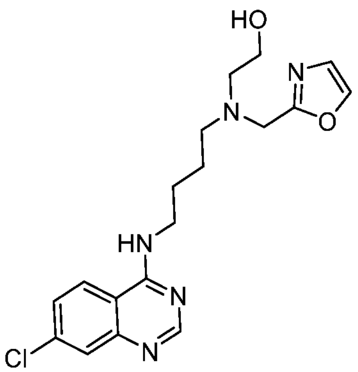

,

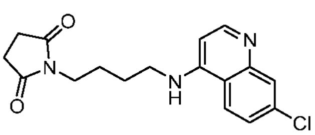

,

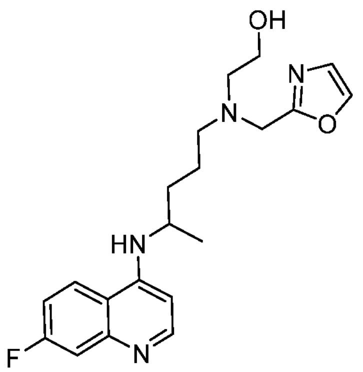

,

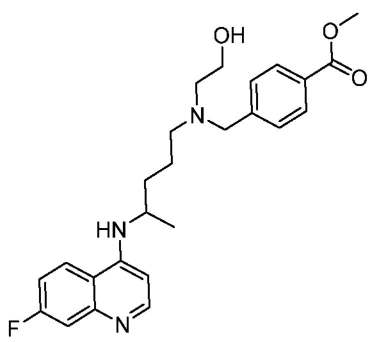 , 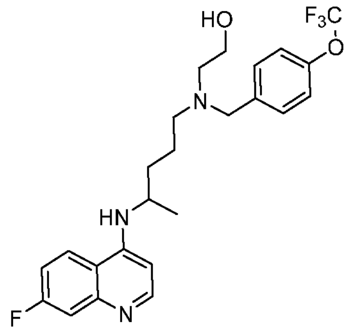 , 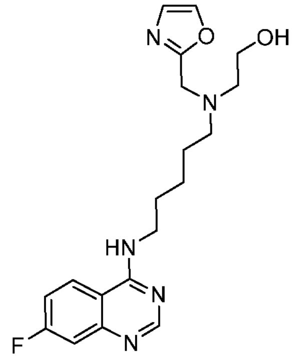 ,

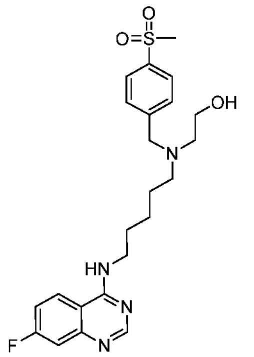 , 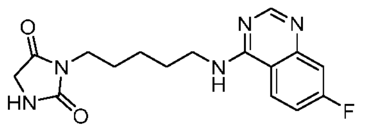 , 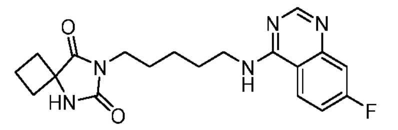 ,

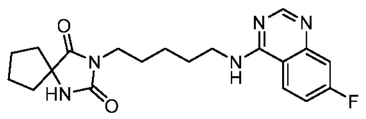 , 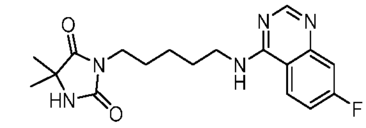 , 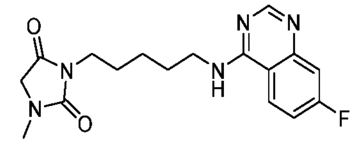 ,

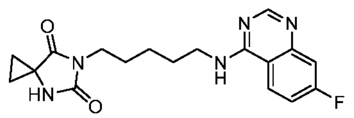 , 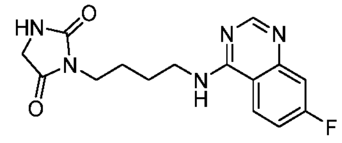 , 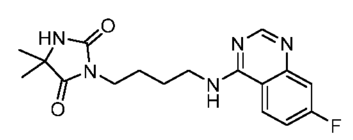 ,

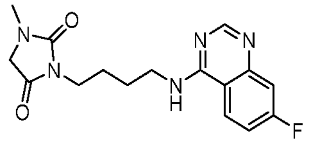 , 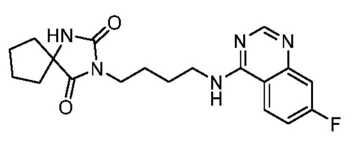 , 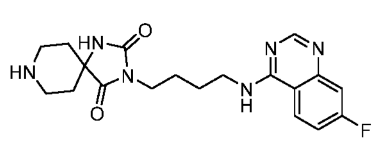 ,

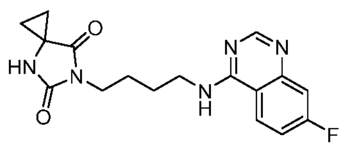 , 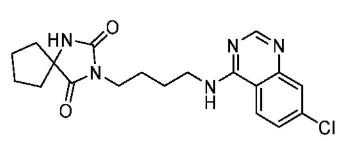 , 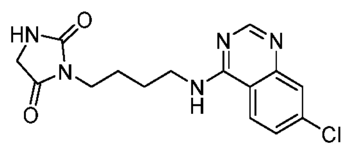 ,

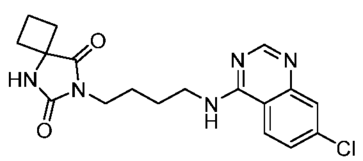 , 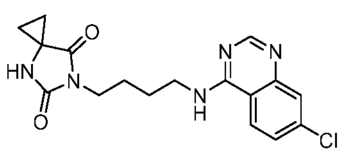 , 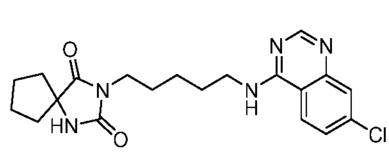 ,

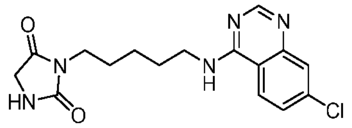,
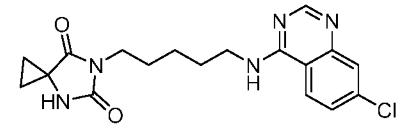,
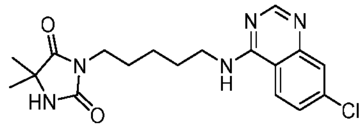,

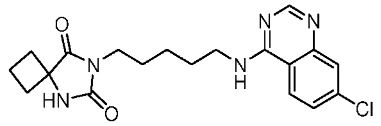,
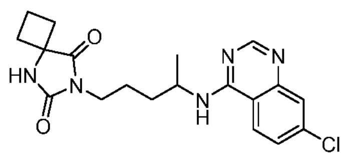,
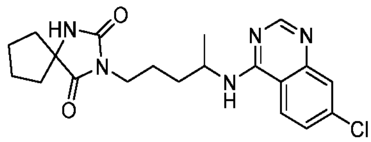,

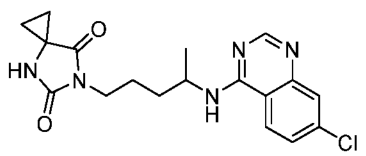,
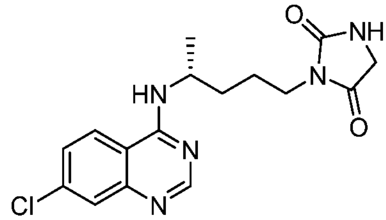,
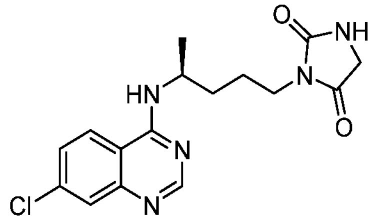,

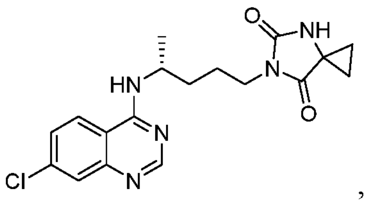, or
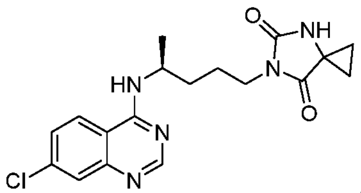.

**15.** A method for preparing the quinazoline compound of formula I according to at least one of claims 1 to 14, and the method is any one of the following methods:

method 1, comprising the following steps: in a solvent, in the presence of a base and an iodine salt, reacting a compound of formula II-1 with a compound of formula II-2;

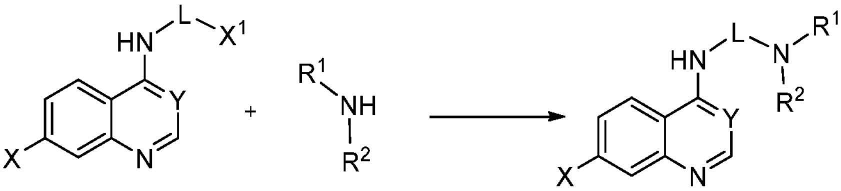

II-1 II-2 I ;

wherein $X^1$ is halogen, such as F, Cl, Br, or I, preferably Cl; Y is CH;
the solvent can be a solvent commonly used for such reactions in the art, such as a nitrile solvent, preferably acetonitrile;
the base can be a base commonly used for such reactions in the art, such as an alkali metal carbonate, preferably potassium carbonate;
the iodine salt can be an iodine salt commonly used for such reactions in the art, such as KI;

method 2: in a solvent, in the presence of a base and a quaternary ammonium salt, reacting a compound of formula II-1 with a compound of formula II-2;

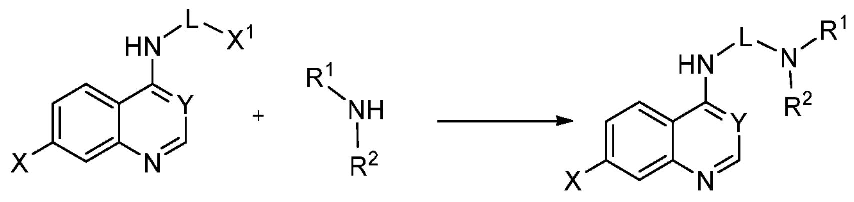

II-1 II-2 I

wherein $X^1$ is halogen, such as F, Cl, Br, or I, preferably Cl; Y is N;

the solvent can be a solvent commonly used for such reactions in the art, such as a nitrile solvent, preferably acetonitrile;

the base can be a base commonly used for such reactions in the art, such as an alkali metal carbonate, preferably potassium carbonate;

the quaternary ammonium salt can be a quaternary ammonium salt commonly used for such reactions in the art, such as $n$-$Bu_4NBr$;

method 3, comprising the following steps: in a solvent, in the presence of a base, reacting a compound of formula II-3 with a compound of formula II-4;

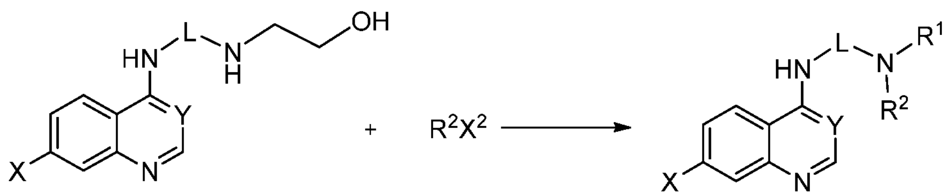

II-3 II-4 I ;

wherein in formula II-4, $X^2$ is halogen, such as F, Cl, Br, or I, preferably Cl; in formula I, $R^1$ is

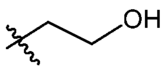

;

the solvent can be a solvent commonly used for such reactions in the art, such as a nitrile solvent, preferably acetonitrile;

the base can be a base commonly used for such reactions in the art, such as an alkali metal carbonate, preferably potassium carbonate.

**16.** A pharmaceutical composition, wherein the pharmaceutical composition comprises a substance X and a pharmaceutical excipient; the substance X is the quinazoline compound of formula I, the pharmaceutically acceptable salt thereof, the solvate thereof, or the solvate of the pharmaceutically acceptable salt thereof according to at least one of claims 1 to 14.

**17.** A use of a substance Y in the manufacture of a medicament, wherein the substance Y is the quinazoline compound of formula I, the pharmaceutically acceptable salt thereof, the solvate thereof, or the solvate of the pharmaceutically acceptable salt thereof according to at least one of claims 1 to 14, or the pharmaceutical composition according to claim 16, and the medicament is a medicament having an inhibitory effect on 1, 2 or 3 of cytokines TNF-$\alpha$, IL-6, and IL-1$\beta$.

**18.** A use of a substance Y in the manufacture of an inhibitor; the substance Y is the quinazoline compound of formula I, the pharmaceutically acceptable salt thereof, the solvate thereof, or the solvate of the pharmaceutically acceptable salt thereof according to at least one of claims 1 to 14, or the pharmaceutical composition according to claim 16, wherein the inhibitor is 1, 2, or 3 of a TNF-$\alpha$ inhibitor, an IL-6 inhibitor, and an IL-1$\beta$ inhibitor, and preferably, the inhibitor is an inhibitor having an *in vitro* inhibitory effect on 1, 2, or 3 of TNF-$\alpha$, IL-6, and IL-1$\beta$.

**19.** A use of a substance Y in the manufacture of a medicament; the substance Y is the quinazoline compound of formula I, the pharmaceutically acceptable salt thereof, the solvate thereof, or the solvate of the pharmaceutically acceptable salt thereof according to at least one of claims 1 to 14, or the pharmaceutical composition according to claim 16; the medicament is used for preventing and/or treating a disease related to 1, 2, or 3 of TNF-$\alpha$, IL-6, and IL-1$\beta$.

**20.** A use of a substance Y in the manufacture of a medicament, wherein the substance Y is the quinazoline compound of formula I, the pharmaceutically acceptable salt thereof, the solvate thereof, or the solvate of the pharmaceutically acceptable salt thereof according to at least one of claims 1 to 14, or the pharmaceutical composition according to claim 16; the medicament is used for preventing and/or treating autoimmune diseases (such as rheumatoid arthritis, systemic lupus erythematosus, cutaneous lupus erythematosus, diabetes, Sjögren's syndrome, multiple sclerosis), malaria, infectious diseases (such as AIDS, viral hepatitis, coronaviruses), allergic diseases (such as asthma), cardiovascular diseases, anti-rejection reactions after organ transplantation, chronic obstructive pulmonary disease, tumors, and other diseases related to the abnormal function and response state of the body's immune system.

**INTERNATIONAL SEARCH REPORT**

International application No. **PCT/CN2022/092860**

**A. CLASSIFICATION OF SUBJECT MATTER**

C07D 215/46(2006.01)i; C07D 233/76(2006.01)i; C07D 241/12(2006.01)i; C07D 231/12(2006.01)i; C07D 269/02(2006.01)i; C07D 295/02(2006.01)i; A61K 31/4706(2006.01)i; A61K 31/4166(2006.01)i; A61K 31/421(2006.01)i; A61K 31/4155(2006.01)i; A61K 31/5375(2006.01)i; A61K 31/496(2006.01)i; A61P 31/14(2006.01)i; A61P 31/18(2006.01)i; A61P 31/20(2006.01)i; A61P 37/02(2006.01)i

According to International Patent Classification (IPC) or to both national classification and IPC

**B. FIELDS SEARCHED**

Minimum documentation searched (classification system followed by classification symbols)

C07D; A61K; A61P

Documentation searched other than minimum documentation to the extent that such documents are included in the fields searched

Electronic data base consulted during the international search (name of data base and, where practicable, search terms used)

CNTXT, WPI, EPODOC, CNKI, ISI web of science, STN (REGISTRY, CAPLUS): 喹啉, 氨, 羟乙基, 氯喹, 羟氯喹, 咪唑啉, 二酮, 免疫, quinoline, amine, chloroquine, CQ, hydroxychloroquine, HCQ, imidazolin+, dione, TNF-α, IL-6, IL-1β, immune, structural formula search

**C. DOCUMENTS CONSIDERED TO BE RELEVANT**

| Category* | Citation of document, with indication, where appropriate, of the relevant passages | Relevant to claim No. |
|---|---|---|
| X | CAS. "RN=2489438-21-1 et al." *STN Registry,* 07 October 2020 (2020-10-07), RN=2489438-21-1 et al. | 1-11, 13, 15 |
| X | CN 112457244 A (HANHAI XINTUO HANGZHOU BIOLOGICAL MEDICINE CO., LTD.) 09 March 2021 (2021-03-09) description, paragraphs [0058] and [0107]-[0110], and embodiments 1-14 and 67 | 1-3, 5-6, 11, 15-20 |
| X | JANG, C. H. et al. "Chloroquine Inhibits Production of TNF-α, IL-1β and IL-6 from Lipopolysaccharide-Stimulated Human Monocytes/Macrophages by Different Modes" *Rheumatology,* Vol. 45, 17 January 2006 (2006-01-17), pp. 703-710 | 1-3, 5-6, 11, 15-20 |
| A | NISHA et al. "β-amino-alcohol Tethered 4-aminoquinoline-isatin Conjugates: Synthesis and Antimalarial Evaluation" *European Journal of Medicinal Chemistry,* Vol. 84, 21 July 2014 (2014-07-21), pp. 566-573 | 1-20 |

☑ Further documents are listed in the continuation of Box C. ☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **28 July 2022** | **10 August 2022** |
| Name and mailing address of the ISA/CN<br>**China National Intellectual Property Administration (ISA/CN)**<br>**No. 6, Xitucheng Road, Jimenqiao, Haidian District, Beijing 100088, China** | Authorized officer |
| Facsimile No. **(86-10)62019451** | Telephone No. |

Form PCT/ISA/210 (second sheet) (January 2015)

**INTERNATIONAL SEARCH REPORT**

International application No.
**PCT/CN2022/092860**

**C. DOCUMENTS CONSIDERED TO BE RELEVANT**

| Category* | Citation of document, with indication, where appropriate, of the relevant passages | Relevant to claim No. |
|---|---|---|
| A | DEVI, K. et al. "Synthesis and Antimicrobial Studies of Oxazolidinone Derivatives" *International Journal of Pharmaceutical Sciences Review and Research,* Vol. 14, No. 2, 31 December 2012 (2012-12-31), pp. 124-129 | 1-20 |

Form PCT/ISA/210 (second sheet) (January 2015)

**INTERNATIONAL SEARCH REPORT**
**Information on patent family members**

International application No.
**PCT/CN2022/092860**

| Patent document cited in search report | Publication date (day/month/year) | Patent family member(s) | Publication date (day/month/year) |
|---|---|---|---|
| CN 112457244 A | 09 March 2021 | CN 113527201 A | 22 October 2021 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 2021105302760


### Non-patent literature cited in the description


- **P. HEINRICH STAHL.** Handbook of Pharmaceutical Salts: Properties, Selection, and Use. 2002
- Pharmacopoeia of the People's Republic of China. 2020
- **RAYMOND C ROWE.** Handbook of Pharmaceutical Excipients. 2009